(19) Europäisches Patentamt / European Patent Office / Office européen des brevets

(11) **EP 4 167 838 B1**

(12) **EUROPEAN PATENT SPECIFICATION**

(45) Date of publication and mention
of the grant of the patent:
**20.05.2026 Bulletin 2026/21**

(21) Application number: **21828973.4**

(22) Date of filing: **23.06.2021**

(51) International Patent Classification (IPC):
**A61B 5/00** (2006.01)

(52) Cooperative Patent Classification (CPC):
**A61B 5/445; A61B 5/4815; A61B 5/681;
A61B 5/7264; A61B 5/7275; A61B 5/7405;
A61B 5/7425**

(86) International application number:
**PCT/US2021/038699**

(87) International publication number:
**WO 2021/262857 (30.12.2021 Gazette 2021/52)**

(54) **COMPUTERIZED DECISION SUPPORT TOOL AND MEDICAL DEVICE FOR SCRATCH
DETECTION AND FLARE PREDICTION**

COMPUTERISIERTES ENTSCHEIDUNGSUNTERSTÜTZUNGSWERKZEUG UND MEDIZINISCHE
VORRICHTUNG ZUR KRATZERKENNUNG UND FLARE-VORHERSAGE

OUTIL INFORMATISÉ DE SUPPORT DE DÉCISION ET DISPOSITIF MÉDICAL POUR LA
DÉTECTION DE GRATTAGE ET LA PRÉDICTION D'ÉRUPTIONS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **23.06.2020 US 202063043108 P
22.06.2021 US 202163213592 P**

(43) Date of publication of application:
**26.04.2023 Bulletin 2023/17**

(73) Proprietor: **Pfizer Inc.
New York, NY 10001-2192 (US)**

(72) Inventors:
• **MAHADEVAN, Nikhil**
**Cambridge, Massachusetts 02139 (US)**
• **DI, Junrui**
**Cambridge, Massachusetts 02139 (US)**
• **CHRISTAKIS, Yiorgos Perikles**
**Cambridge, Massachusetts 02139 (US)**
• **PATEL, Shyamal**
**Cambridge, Massachusetts 02139 (US)**

(74) Representative: **Plasseraud IP
104 Rue de Richelieu
CS92104
75080 Paris Cedex 02 (FR)**

(56) References cited:
US-A1- 2016 302 735     US-A1- 2018 228 427
US-A1- 2018 228 427     US-A1- 2019 279 480

• ARNAUD MOREAU ET AL: "Detection of
Nocturnal Scratching Movements in Patients
with Atopic Dermatitis Using Accelerometers
and Recurrent Neural Networks", IEEE
JOURNAL OF BIOMEDICAL AND HEALTH
INFORMATICS, vol. 22, no. 4, 1 July 2018
(2018-07-01), Piscataway, NJ, USA, pages 1011 -
1018, XP055663063, ISSN: 2168-2194, DOI:
10.1109/JBHI.2017.2710798
• JOHANNA PETERSEN ET AL: "Actigraphy-
Based Scratch Detection Using Logistic
Regression", IEEE JOURNAL OF BIOMEDICAL
AND HEALTH INFORMATICS, IEEE,
PISCATAWAY, NJ, USA, vol. 17, no. 2, 1 March
2013 (2013-03-01), pages 277 - 283, XP011496365,
ISSN: 2168-2194, DOI: 10.1109/
TITB.2012.2204761

- LUNDBERG ET AL.: "From local explanations to global understanding with explainable A1 for trees", NATURE MACHINE INTELLIGENCE, vol. 2, no. 1, 17 January 2020 (2020-01-17), pages 56 - 67, XP055756937, DOI: 10.1038/ s42256-019-0138-9
- VAN HEES ET AL.: "Estimating sleep parameters using an accelerometer without sleep diary", SCIENTIFIC REPORTS, vol. 8, no. 1, 28 August 2018 (2018-08-28), pages 1 - 11, XP055892993
- MOREAU ET AL.: "Detection of nocturnal scratching movements in patients with atopic dermatitis using accelerometers and recurrent neural networks", IEEE JOURNAL OF BIOMEDICAL AND HEALTH INFORMATICS, vol. 22, no. 4, 8 June 2017 (2017-06-08), pages 1011 - 1018, XP055663063, DOI: 10.1109/ JBHI.2017.2710798

**Description**

BACKGROUND OF THE INVENTION

Atopic dermatitis is a chronic relapsing and remitting skin disease that affects approximately 10% of adults and 12% of children in the United States. It is characterized by red, excoriated lesions on the skin with pruritus (itch). Individuals experiencing pruritus typically scratch the affected skin, which exacerbates the inflammation causing the pruritus and perpetuates an itch-scratch cycle. For many individuals with atopic dermatitis, pruritus peaks in the nighttime, resulting in sleep disturbance.

Assessments of a disease associated with pruritus, such as atopic dermatitis, are traditionally subjective, episodic, and provide poor measurements on the impact of atopic dermatitis. For example, one traditional tool is a clinical outcome assessment (COA) that involves a clinician assessing total body surface area of a lesion and lesion severity. COAs are subjective in that their assessments vary across different clinicians and are episodic in nature, as they can only be done when an individual is seen by a clinician. Another traditional tool is a patient reported outcome (PRO) that is a qualitative and subjective report from the patient as to the severity of the pruritus. PROs may lack accuracy due to lack of compliance, recall bias, and diary fatigue.

Document "Detection of Nocturnal Scratching Movements in Patients with Atopic Dermatitis Using Accelerometers and Recurrent Neural Networks", ARNAUD MOREAU ET AL:, IEEE JOURNAL OF BIOMEDICAL AND HEALTH INFORMATICS, vol. 22, no. 4, 1 July 2018 (2018-07-01) discloses a system for providing decision support based on scratch events.

SUMMARY OF THE INVENTION

This summary is provided to introduce a selection of concepts in a simplified form that are further described below in the detailed description. This summary is not intended to identify key features or essential features of the claimed subject matter, nor is it intended to be used in isolation as an aid in determining the scope of the claimed subject matter.

Embodiments of the present disclosure enable improved computer decision support tools for detecting scratch and, in some aspects, predicting flare events in the future. As used herein, the term "flare event" may refer to an acute or particularly severe phase of pruritus. Embodiments may include utilizing data acquired by a sensor device, which may be a wearable device, to automatically detect scratch events. In this way, scratch events may be detected based on a continuous stream of data input into one or more machine learning classifiers to provide an objective assessment of scratching. The scratch behavior detected, in accordance with some embodiments herein, is nighttime scratching or scratching during a period in which the user is intending to sleep. This detection helps track scratching during peak pruritus time or even when a user is unaware of the scratching. As such, scratch events detected, in accordance with embodiments of this disclosure, may provide more accurate measures of the user's current pruritus and atopic dermatitis. Further, embodiments may utilize patterns of detected scratching to predict a likely itch level in a future interval, which may indicate a future flare event. The detected scratch events and, in some embodiments, predicted future itch level and/or flare event, may be utilized in computerized decision support tools to more accurately and timely track atopic dermatitis symptoms and initiate intervening and/or therapeutic treatments to alleviate or prevent symptoms.

Scratch may be detected utilizing accelerometer data acquired by a sensor that is worn by a monitored individual (which may also be referred to herein as a patient or a user). Using the sensor data, a hand motion event may be detected, and it may be determined whether that hand motion event is a likely scratch event. In some aspects, prior to detecting hand motion events, context is determined to limit the potential sensor data utilized for detecting hand motion events. In some aspects, the context includes detecting whether the sensor is configured for proper data acquisition, such as detecting that the sensor is being worn by the user, which is more likely to result in accurate detection of hand motion events and, in turn, scratch events. Additionally, a user sleep opportunity may be detected to determine a period of time during which the user intends to sleep, and hand motion events and scratch events may be detected using sensor data acquired during this user sleep opportunity. In this way, scratches occurring at nighttime (when pruritus peaks) and/or while a user is sleeping and less likely to be aware of the scratching may be detected.

A detected likely scratch event may be recorded, and an action may be initiated based on one or more detected scratch events. For instance, an alert or a notification may be issued to a user to notify that user of the detected scratch event(s). Additionally, data related to the detected scratch event may be processed for computer-implemented decision making. For example, scratch event data may be aggregated to identify a total number of detected scratch events over a period of time, such as a 24-hour time period. In some embodiments, a total scratch duration may also be determined by adding the durations of all detected scratch events within the defined period of time. The total scratch events and/or total scratch duration may be utilized to initiate recommendations to seek medical treatment or consultation with a clinician or issuing a notification to a user device associated with a clinician of the monitored individual. Additionally, or alternatively, the total scratch event and/or total scratch duration may be added to a user's electronic calendar for the period of time during which the scratch data was detected. Additionally, embodiments may determine total scratch events and/or total scratch duration for multiple periods of time to identify scratching severity over time and/or changes in scratching behavior, either of which may be utilized to initiate an action.

Detection of a scratch event may be achieved by applying one or more machine learning models to feature values extracted from sensor data for a detected hand motion event. In some aspects, the machine learning model is an ensemble of models, such as gradient boosting or a random forest classifier. Aspects of the present disclosure may, therefore, include training machine learning model(s) to detect whether a hand motion is a scratch event or not.

Some embodiments of the present disclosure may further utilize detected scratch events to predict a likelihood of a user having itch in a future time interval. Scratch patterns may be determined based on the detected scratch events over a period of time. In some embodiments, the period of time may be 24 hours, but it is contemplated that other periods of time, such as 3 days or 5 days, may be utilized. Additional contextual information may be determined, such as the temperature and/or humidity levels at a location of the user for the time period during which the scratch events were detected. Additionally, the temperature and/or humidity level forecast for the future time interval may be determined. Based on the scratch pattern and contextual information, a likely itch level for the future time interval may be determined. Further, some embodiments may predict a likely flare event for the user by determining whether a predicted itch level is of sufficient severity to rise to a level of a flare event. Determining a likelihood of a future flare event may be determined by comparing the predicted itch level to one or more threshold itch levels.

Some embodiments may initiate an action based on the predicted itch level and, in some instances, a flare event, during a future time interval. Initiating an action may include generating an itch or flare notification to a patient or a clinician treating the monitored patient, adding the predicted itch level and/or flare event to an electronic calendar for the future time interval, and/or making one or more recommendations. A recommendation may be to start treatment, continue treatment, or modify treatment of the monitored patient. Additionally, a recommendation may be for the monitored patient to schedule a consultation with a clinician.

Further aspects of this disclosure include detecting whether the monitored user is asleep utilizing the sensor data. Similar to some embodiments of detecting scratch events, sensor data, acquired during times in which a configuration for proper data acquisition is detected and/or the user's sleep opportunity, may be utilized to determine whether the user is asleep or not. The sensor data may be utilized to determine activity index values for windows of time, and a combination of the activity index values, such as a weighted sum, may be compared to a sleep threshold to detect whether the user is asleep or not. Determinations of periods of time during a user's sleep opportunity when the user is awake versus asleep may be utilized to determine an overall sleep score, which provides a measure of the user's quality of sleep for a period of time, such as one night. In some aspects, the sleep score may further be determined based on detected scratch events as more scratch events during the user's sleep opportunity may indicate a lower quality of sleep.

BRIEF DESCRIPTION OF THE DRAWING

Aspects of the disclosure are described in detail below with reference to the attached drawing figures, wherein:

FIG. 1 is a block diagram of an example operating environment suitable for implementing aspects of the present disclosure;

FIG. 2 is a diagram depicting an example computing architecture suitable for implementing aspects of the present disclosure;

FIGS. 3A and 3B illustratively depict uses of embodiments of the present disclosure;

FIG. 4A illustratively depicts a flow diagram of an example method for detecting scratch, in accordance with an embodiment of the present disclosure;

FIG. 4B illustratively depicts a diagrammatic representation of detecting sensor wear, in accordance with an embodiment of the present disclosure;

FIG. 4C illustratively depicts a diagrammatic representation of determining user sleep opportunity, in accordance with an embodiment of the present disclosure;

FIG. 4D illustratively depicts a diagrammatic representation of an example process for detecting user sleep and wake periods, in accordance with an embodiment of the present disclosure;

FIG. 4E illustratively depicts a diagrammatic representation of example aspects of a scratch detection process, in accordance with an embodiment of the present disclosure;

FIG. 4F illustratively depicts a diagrammatic representation of an example process for providing decision support based on scratch events, in accordance with an embodiment of the present disclosure;

FIG. 4G illustratively depicts a diagrammatic representation of an example process treating pruritus utilizing a motion sensing device associated with a subject, in accordance with an embodiment of the present disclosure;

FIG. 4H illustratively depicts a diagrammatic representation of an example process utilizing scratch detection, in accordance with an embodiment of the present disclosure;

FIG. 5 illustratively depicts a flow diagram of an example method of predicting flare, in accordance with an embodiment of the present disclosure;

FIG. 6A illustratively depicts a diagrammatic representation of training an example scratch detector, in accordance with an embodiment of the present disclosure;

FIG. 6B illustratively depicts graphic representation of feature selection for an example scratch detector, in accordance with an embodiment of the present disclosure;

FIG. 6C illustratively depicts a representation of performance validation of an example scratch detector, in accordance with an embodiment of the present disclosure;

FIG. 6D illustratively depicts a representation statistical performances example scratch detectors, in accordance with an embodiment of the present disclosure;

FIG. 6E illustratively depicts a representation of performance of an example sleep opportunity determiner, in accordance with an embodiment of the present disclosure;

FIG. 6F depicts a representation of performance validation of an example sleep opportunity algorithm, in accordance with an embodiment of the present disclosure;

FIG. 7A illustratively depicts a diagrammatic representation of signals showing detected hand movement, in accordance with an embodiment of the present disclosure;

FIG. 7B illustratively depicts a diagrammatic representation of continuous sleep and nighttime scratch detection, in accordance with an embodiment of the present disclosure;

FIGS. 8A-F illustratively depict exemplary screenshots from a computing device showing aspects of example graphical user interfaces (GUIs), in accordance with embodiments of the present disclosure;

FIGS. 9A-I depict an example embodiment of a computer program routine for detecting scratch and sleep, in accordance with embodiments of the present disclosure;

FIGS. 10A-I depict an example embodiment of a computer program routine for detecting scratch, in accordance with embodiments of the present disclosure;

FIGS. 11A-M depict an example embodiment of a computer program routine for detecting sleep, in accordance with embodiments of the present disclosure; and

FIG. 12 is a block diagram of an exemplary computing environment suitable for use in implementing an embodiment of the present disclosure.

## DETAILED DESCRIPTION OF THE INVENTION

The subject matter of the present disclosure is described herein with specificity with the help of different aspects to meet statutory requirements. However, the description itself is not intended to limit the scope of this patent. The claimed subject matter might be embodied in other ways, to include different steps or combinations of steps similar to the ones described in this present disclosure, in conjunction with other present or future technologies. Moreover, although the terms "step" and/or "block" may be used herein to connote different elements of methods employed, the terms should not be interpreted as implying any particular order among or between various steps disclosed herein, unless and except when the order of individual steps is explicitly stated. Each method described herein may comprise a computing process that may be performed using any combination of a hardware, firmware, and/or software. For instance, various functions may be carried out by a processor executing instructions stored in memory. The methods may also be embodied as computer-useable instructions stored on computer storage media. The methods may be provided by a stand-alone application, a service or a hosted service (stand-alone or in combination with another hosted service), or a plug-in to another product, to name a few.

Aspects of the present disclosure relate to computerized decision support tools for predicting scratch and flare events. Affecting approximately 10% of adults and 12% of children in the United States, atopic dermatitis is a chronic relapsing and remitting skin disease that is characterized by red, excoriated lesions on the skin with pruritus (itch). Individuals experiencing pruritus typically scratch the affected skin, which exacerbates the inflammation causing the pruritus and perpetuates an itch-scratch cycle. For many individuals with atopic dermatitis, pruritus peaks in the nighttime, resulting in sleep disturbance. Not only does the physical act of scratching disrupt sleep, but scratching has also found to trigger cognitive and behavioral changes that lead to and reinforce insomnia and sleep-disruptions. Additionally, scratch-medicated epidermal damage may result in inflammatory responses that disrupts circadian rhythm.

Conventional assessments of a disease associated with pruritus, such as atopic dermatitis, are traditionally subjective, episodic, and provide poor measurements on the impact of atopic dermatitis. For example, one traditional tool is a clinical outcome assessment (COA) that involves a clinician assessing total body surface area of a lesion and lesion severity. COAs are subjective in that their assessments vary among different clinicians and are episodic in nature, as they can only be assessed when the individual is seen by a clinician. Another traditional tool is a patient reported outcome (PRO) that is a qualitative and subjective report from a patient about the severity of pruritus. Such PROs may include Patient Global Impression Severity (PGIS), Peak Pruritus Numerical Rating Scale (ppNRS), Severity of Pruritus Scale (SPS), Dermatology Life Quality Index (DLQI), Family or Children DLQI (FDLQI/CDLQI), Medical Outcome Study (MOS) Sleep Scale, Patient Oriented Eczema Measure (POEM), PROMIS Pain Interference, and PROMIS-Anxiety. PROs may lack accuracy due to lack of compliance, recall bias, and diary fatigue.

Attempts to provide an objective assessment have been made by utilizing recurrent neural networks to detect scratching from sensor data. However, these current tools require two sensors (one on each wrist of a user or patient), thus increasing the burden on the patient, the likelihood of lack of user compliance, and inaccurate results due to challenges associated with aligning time among the two sensors and possibility of one of the sensors not being properly configured. Further, current machine learning attempts to detect scratch do not focus on detecting scratching during sleep opportunities. As explained above, pruritus peaks at nighttime and can disrupt sleep, and, therefore, conventional solutions that do not detect scratch events within the context of sleep opportunity fail to provide an accurate assessment of the current state of pruritus. Further, conventional tools do not predict future itch or flare events and, therefore, have a limited ability to enable preventative therapeutic measures.

To improve accuracy and reliability, embodiments of the present disclosure result in improved computer decision support tools by detecting scratch and, in some aspects, predicting flare events that are likely to occur in the future from continuous sensor data, unobtrusively acquired by a sensor device worn by a user. As such, the information utilized to detect scratching is not episodic in nature. Additionally, some embodiments of the sensor device, such as a wrist worn device, are less invasive than conventional techniques requiring the user to sleep in a controlled, monitored environment, which results in a greater likelihood of user compliance and are particularly well adapted for use by populations that are traditionally not very compliant, such as children. In some aspects, only one sensor device is worn by a monitored user (interchangeably referred herein as patient) to further reduce potential user burden. Additionally, feature values extracted from the sensor data may be utilized to detect scratching using one or more machine learning classifiers, thereby removing subjectivity. Embodiments may detect scratching from sensor data obtained during nighttime or during a user sleep opportunity, facilitating tracking of scratch during peak pruritus time or when a user is unaware of the scratching. Further, a likelihood of the user experiencing an itch level or a flare event in the future may be predicted from patterns of detected scratch events. The detected scratch events and/or predicted future itch level and/or flare event may be fed into computerized decision support tools to accurately and timely track atopic dermatitis symptoms and initiate intervening and/or therapeutic treatments to alleviate or prevent worsening symptoms.

At a high level, a sensor device worn by a user may acquire sensor data to detect scratch. In exemplary aspects, the sensor data is accelerometer data captured by a wearable sensor located on or around the user's wrist. From the sensor data, a two-tier approach may be utilized to detect scratch. In some embodiments, a hand movement event may be detected, and sensor data detected within the hand movement event may then be classified as a scratch event.

In some aspects, prior to detecting hand movement, context is determined to narrow the scope of the sensor data for hand movement analysis. In some aspects, the context includes detecting whether the sensor device is configured for proper data acquisition, which is more likely to result in accurate detection of hand movement and scratch events. For instance, detecting whether the sensor device is configured for proper data acquisition may include determining that a wearable sensor device, such as a wrist-worn device, is being worn by a user or not. In some implementations, this step includes determining not only whether the sensor device is worn but whether the manner in which the device is worn facilitates capturing the intended data. As described herein, the determination that the sensor device is properly configured for data acquisition may include utilizing sensed temperature information (e.g., a user's near-body temperature) and comparing the sensed temperature information to a predetermined threshold to determine whether the device is being worn or not. In other implementations, this determination is made by applying a set of heuristic rules to statistical features of motion data, such as standard deviations and/or ranges of x, y, and z variables in accelerometer data. In some embodiments, a combination of variables, such as temperature and motion data, may be utilized to detect that the device is not worn.

Additionally, in some aspects, the scope of the data utilized for hand movement detection may further be narrowed to data captured within a sleep opportunity or an interval in which the user intends to sleep. As such, embodiments of this disclosure may determine a sleep opportunity. The sleep opportunity may be identified by comparing changes in arm angles, as derived from motion sensed data, to a sleep opportunity threshold to detect candidate sleep opportunity periods. In some embodiments, a longest group of candidate sleep opportunity periods (which may exclude periods of non-wear) within a relevant time frame, such as a 24-hour period, may be selected as the sleep opportunity.

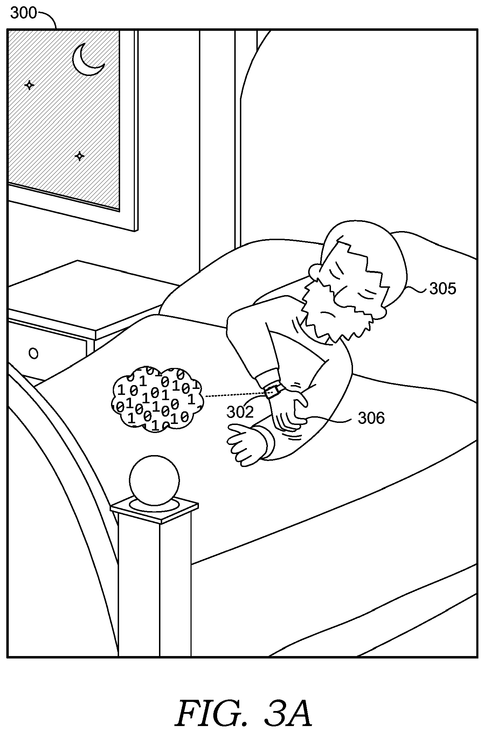

After determining a sleep opportunity, motion data captured during the determined sleep opportunity may be utilized for detecting hand movement and scratch events. In this way, embodiments may determine scratching at nighttime (when pruritus peaks) and/or when a user is sleeping and less likely to be aware of the scratching. FIG. 3A depicts an example scratch detection implementation in operation in which a scratch event is being detected while a user is sleeping. As illustrated in FIG. 3A, a smart watch being worn by a sleeping user may sense motion data, detect a scratch event, and connect to a network, such as a cloud, to log the data.

In some embodiments, detecting hand motion includes segmenting the sensor data within the user sleep opportunity into windows of time and applying a heuristic algorithm to each window to determine the presence of hand movement within each window. In some embodiments, the heuristic algorithm for hand motion detection includes computing a rolling coefficient of variation and determining whether that value satisfies a motion threshold.

Various embodiments of the disclosure may determine whether the hand movement corresponds to a scratch event. To detect a scratch event, feature values may be extracted from sensor data within the windows determined to represent hand movement. In exemplary aspects, the features are time domain features or frequency domain features. The extracted feature values may run through a scratch detector that determines whether the detected hand motion was a scratch event or not. In exemplary aspects, the scratch detector comprises an ensemble of machine learning models, such as a random forest classifier. Aspects of the disclosure may include building the scratch detector, which may include feature selection and engineering and training one or more machine learning models. In some aspects, the machine learning models are trained by utilizing a leave-one-subject-out (LOSO) validation process.

In some aspects, a detected scratch event may be recorded, and an action may be initiated based on one or more detected scratch events. For instance, an alert or a notification may be issued to a user, via a user interface on a user device, to notify the user of the scratch event(s). Additionally, the detected scratch event data may be processed for computer-implemented decision making. In one embodiment, scratch endpoint data may be determined from detected scratch events. For example, a total number of detected scratch events over a period of time, such as a 24-hour period of time, and/or a total scratch duration within that period may be determined. The total scratch events and/or total scratch duration may be utilized to initiate recommendations to a monitored individual to seek medical treatment or consultation with a clinician. Additionally, or alternatively, total scratch events and/or total scratch duration may be utilized to issue a notification to a user device associated with a clinician of the monitored individual. The total scratch event and/or total scratch duration may be added to a tracking application or a service to present the scratch endpoints as associated with the period of time for which it was detected. A scratch score may further be computed based on the detected scratch events and/or scratch endpoints and may be presented to the monitored user or clinician. Additionally, embodiments may determine total scratch events and/or total scratch duration for multiple periods of time to identify scratching severity over time and/or changes in patterns, either of which may be utilized to initiate an action. Scratch endpoints disclosed herein represent novel digital endpoints that are useful in quantitatively and objectively measuring pruritus or, more specifically, atopic dermatitis. This new type of data may be created utilizing the disclosed technology for monitoring scratch, which may be done using one or more wearable devices for continuous monitoring. In this way, the disclosed method of gathering data for measuring scratch results in new scratch endpoint data that is more accurate and useable than the conventional technologies for monitoring and treating a user because it provides a quantitative, accurate, and objective measure. As stated above, this method of obtaining the data used in creating the scratch endpoints is particularly useful in populations with typically lower compliance rates, such as children.

Some embodiments of the disclosure may include detecting whether the monitored user is asleep and/or awake during the sleep opportunity. As such, similar to some embodiments of detecting scratch, sleep may be detected by utilizing sensor data acquired during times in which a sensor configuration for proper data acquisition is detected (e.g., when the sensor is worn) and within the determined sleep opportunity. Detecting sleep may include determining activity index values for windows of time based on motion sensed data (e.g., accelerometer data), and a combination of multiple activity index values, such as a weighted sum, may be compared to a sleep threshold to detect whether the user is asleep or highly likely to be asleep. Determination of periods in which the user is awake or asleep within the user's sleep opportunity may be utilized to determine an overall sleep score that provides one or more measures of the user's sleep for a period of time, such as one night. In some aspects, the sleep score may further be determined based on a number of detected scratch events, as more scratch events during the user's sleep opportunity may indicate a lower quality of sleep.

Further embodiments of the present disclosure utilize detected scratch events to predict a likelihood of the user having itch in a future time interval. Scratch patterns may be assembled based on historical scratch events over a period of time. Additional contextual information may be determined and utilized for this prediction, such as atmospheric temperature and/or humidity levels at a location of the user. This contextual information may be historical contextual information such that it may provide insight so that an itch or flare predictor may learn and current or forecasted contextual information may be input into that predictor. Based on the scratch pattern and contextual information, a likely itch level for the future time interval may be determined. Further, some embodiments may predict a likely flare event for the user by determining whether the predicted itch level is of sufficient severity to rise to the level of a flare event. Determining a likelihood of a future flare event may include comparing the predicted itch level with one or more threshold itch levels, which may be based on reference population or user-specific threshold(s) defined based on historical user information and/or user or clinician settings or preferences.

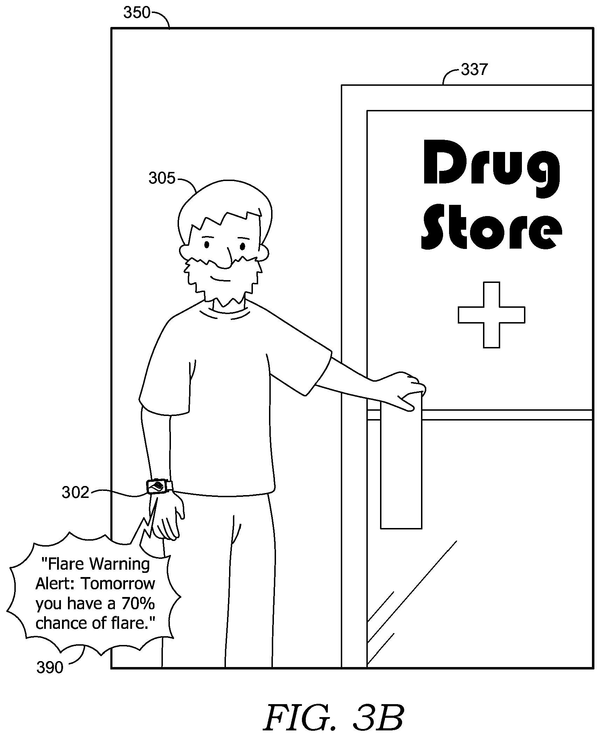

Embodiments may initiate an action based on the predicted user itch level and, in some instances, a flare event, within a future time interval. Initiating an action may include generating an itch or flare notification to a user or a clinician who is treating or expected to treat the user. FIG. 3B shows an example flare notification provided in an implementation in operation. As depicted in FIG. 3B, a user may receive a flare alert notification indicating likelihood of experiencing a particular itch level in the future and/or a risk of a flare occurring in the future. Receiving the flare notification may prompt the user to go to the pharmacy to purchase a treatment to treat or mitigate the potential flare.

In addition, or alternatively, initiating an action may include adding the detected itch level and/or flare event to an electronic calendar for a future time interval, thereby allowing a user to track predicted itch levels and future flare events. Further, an action may include making one or more recommendations. A recommendation may be to start treatment, continue treatment, and/or modify existing treatment. For instance, in operation, a user may receive a recommendation to purchase or refill a treatment to reduce or mitigate a predicted flare risk. Additionally, a recommendation may be for the user to schedule a consultation with a clinician.

Among others, a benefit of embodiments of the disclosure includes providing an assessment of pruritus (based on the resulting scratch) with greater accuracy and reliability (as compared to conventional solutions) based on continuous (or semi-continuous, periodic, as needed, or as-it-becomes-available) data acquired in a way to reduce burden on the user and increase user compliance. For instance, studies have shown that itch, as measured subjectively, does not have a high correlation with nighttime scratching, and itch has a lower correlation with severity of atopic dermatitis than objective scratch measures determined in accordance with embodiments herein. As such, embodiments may be used to more effectively treat and manage pruritus or atopic dermatitis compared to conventional subjective measures. Further, applying machine learning classifiers to the sensor data to detect scratch events removes bias and subjectivity, further improving accuracy and reliability. These classifiers help to provide reliable computer decision support tools that are based on detected scratch data, thereby improving recommendations for treatment and/or responses to scratching. Compared to other scratch detection approaches utilizing a recurrent neural network, some embodiments of this disclosure utilize gradient boosting or a random forest classifier and yield results that are more interpretable, when compared to the recurrent neural network approaches, and, therefore, better capable of being modified or refined for particular contexts. These embodiments further may be performed faster and are less computationally burdensome on computing systems. Additionally, embodiments enable prediction of itch and, to some extent, flare events within the future to better help a monitored user make informed decisions about treatment and/or to help the user's clinician manage care of the condition by proactively treating the skin to reduce the risk of itch or a flare. Further advantageous may result from embodiments determining a user's sleep opportunity and measuring scratching within the determined sleep opportunity. As previously stated, scratching may be particularly disruptive on a user's sleep and, as such, monitoring scratching during a sleep opportunity may more reliably lead to effective measures to improve a user's sleep.

As can be appreciated, embodiments of this disclosure may comprise a tracking application or service that tracks scratch events per night in an accurate manner with limited burden on the user. Such tracking, including alerts, notifications, and recommendations, may promote better treatment compliance on the user's part. Accurate and non-sporadic tracking over time may also enable a clinician to make informed decisions with respect to the monitored individual's treatment. In this way, embodiments of this disclosure may be desirable for both the monitored individual and treating clinician in the form of a tracking service. Also, utilizing the tracking service may be part of a clinician's prescription and/or treatment plan for an individual suffering from pruritus or who was prescribed a medication that lists pruritus as a known potential side effect. For example, a clinician may prescribe a cream to a patient suffering from pruritus with directions to apply the cream every other day and to utilizing an embodiment of the disclosed tracking application or service. Based on the scratch event data acquired for the patient over the next few weeks, it may be determined that the scratching is not improving and the clinician may determine to alter the prescribed course of treatment.

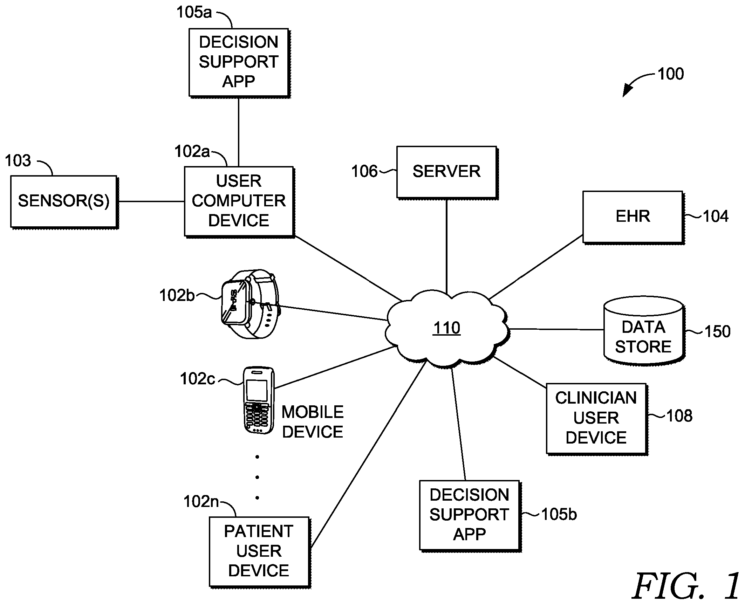

Turning now to FIG. 1, a block diagram is provided showing an example operating environment 100 in which some embodiments of the present disclosure may be employed. It should be understood that this and other arrangements described herein are set forth only as examples. Other arrangements and elements (e.g., machines, interfaces, functions, orders, and groupings of functions) can be used in addition to, or instead of, those shown in FIG. 1 as well as other figures, and some elements may be omitted altogether for the sake of clarity. Further, many of the elements described herein are functional entities that may be implemented as discrete or distributed components or in conjunction with other components, and in any suitable combination and location. Various functions or operations described herein are being performed by one or more entities including a hardware, firmware, software, and a combination thereof. For instance, some functions may be carried out by a processor executing instructions stored in memory.

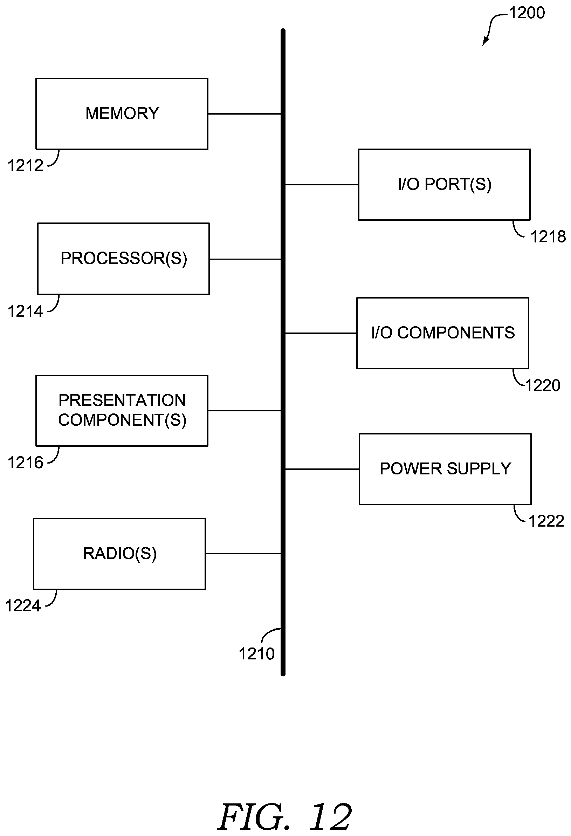

Among other components not shown, example operating environment 100 includes a number of user devices, such as user computer devices 102a, 102b, 102c through 102n and a clinician user device 108; one or more decision support applications, such as decision support applications 105a and 105b; an electronic health record (EHR) 104; one or more data sources, such as a data store 150; a server 106; one or more sensors, such as a sensor(s) 103; and a network 110. It should be understood that operating environment 100 shown in FIG. 1 is an example of one suitable operating environment. Each of the components shown in FIG. 1 may be implemented via any type of computing device, such as a computing device 1200 described in connection with FIG. 12, for example. These components may communicate with each other via network 110, which may include, without limitation, one or more local area networks (LANs) and/or wide area networks (WANs). In exemplary implementations, network 110 comprises Internet and/or a cellular network, amongst any of a variety of possible public and/or private networks.

It should be understood that any number of user devices, servers, decision support applications, data sources, and EHRs may be employed within operating environment 100 within the scope of the present disclosure. Each element may comprise a single device or component, or multiple devices or components cooperating in a distributed environment.

For instance, server 106 may be provided via multiple devices arranged in a distributed environment that collectively provide the functionality described herein. Additionally, other components not shown herein may also be included within the distributed environment.

User devices 102a, 102b, 102c through 102n and clinician user device 108 can be client user devices on a client-side of operating environment 100, while server 106 can be on a server-side of operating environment 100. Server 106 can comprise server-side software designed to work in conjunction with client-side software on user devices 102a, 102b, 102c through 102n and 108 so as to implement any combination of the features and functionalities discussed in the present disclosure. This division of operating environment 100 is provided to illustrate one example of a suitable environment, and there is no requirement that any combination of server 106 and user devices 102a, 102b, 102c through 102n and 108 remain as separate entities.

User devices 102a, 102b, 102c through 102n and 108 may comprise any type of computing device capable of use by a user. For example, in one embodiment, user devices 102a, 102b, 102c through 102n and 108 may be the type of computing devices described in relation to FIG. 12 herein. By way of example and not limitation, a user device may be embodied as a personal computer (PC), a laptop computer, a mobile or a mobile device, a smartphone, a smart speaker, a tablet computer, a smart watch, a wearable computer, a personal digital assistant (PDA) device, a music player or an MP3 player, a global positioning system (GPS) or device, a video player, a handheld communications device, a gaming device, an entertainment system, a vehicle computer system, an embedded system controller, a camera, a remote control, an appliance, a consumer electronic device, a workstation, or any combination of these delineated devices, or any other suitable computer device.

Some user devices, such as user devices 102a, 102b, 102c through 102n may be intended to be used by a user who is being monitored via one or more sensors, such as sensor(s) 103. In some embodiments, a user device may include an integrated sensor (similar to sensor 103) or operate in conjunction with external sensor 103. In other exemplary aspects, sensor 103 may be positioned on or near the monitored user's wrist. It is contemplated that sensor 103 may alternatively be positioned on or near an appendage (e.g., on or near the user's head, attached to the subject's clothing, worn around the subject's head, neck, leg, arm, ankle, finger, etc.). In other aspects, sensor 103 may be a skin-patch sensor adhered to the subject's skin; ingestible or sub-dermal sensor; sensor components integrated into the subject's living environment (including the bed, pillow, or bathroom); and sensors operable with or through a smartphone carried by the subject, for example. In one embodiment, user device comprises a wearable wrist computing device with an integrated sensor, such as a smart watch or a tablet that is communicatively coupled to a source of sensor data.

In exemplary embodiments, sensor 103, such as a gyroscopic or an accelerometer sensor, senses motion information. For example, sensor 103 may comprise a wearable accelerometer sensor, which may be implemented on a fitness tracker wristband device, a smartwatch, and/or a smart mobile device. Other types of sensors may also be integrated into or work in conjunction with user devices, such as sensors configured to detect ambient light (e.g., a photodetector); sensors configured to detect user location (e.g., an indoor positioning system (IPS) or a global positioning system (GPS)); sensors configured to detect atmospheric information (e.g., a thermometer, a hygrometer or a barometer); and physiological sensors (e.g., sensors detecting heart rate, blood pressure, core body temperature, near body temperature, or galvanic skin response (GSR)). Some embodiments include multiple sensors 103, such as three sensors, to obtain accelerometer data, ambient light data, and temperature (e.g., near-body temperature) data. Some embodiments of sensors 103 may include sensors measuring information to be used to monitor fine finger movement, such as electromyography (EMG) for measuring activation of muscles, acoustic surveillance, and/or vibration transducers. It is contemplated, however, that physiological information about the monitored individual, according to embodiments of the disclosure, may also be received from the monitored individual's historical data in EHR 104, or from human measurements or human observations.

Data may be acquired by sensor 103 continuously, periodically, as needed, or as it becomes available. Further, data acquired by sensor 103 may be associated with time and date information and may be represented as one or more time series of measured variables. In an embodiment, sensor 103 collects raw sensor information and performs signal processing, forming variable decision statistics, cumulative summing, trending, wavelet processing, thresholding, computational processing of decision statistics, logical processing of decision statistics, pre-processing and/or signal condition. Alternatively, one or more of these functions may be performed by a user device, such as user device 102c or clinician user device 108, server 106, and/or decision support applications (apps) 105a or 105b.

Some user devices, such as clinician user device 108, may be intended to be used by a clinician who is treating or otherwise monitoring a user associated with sensor 103. Clinician user device 108 is communicatively coupled through network 110 to EHR 104. Operating environment 100 depicts an indirect communicative coupling between clinician user device 108 and EHR 104 through network 110. However, it is contemplated that an embodiment of clinician user device 108 may be communicatively coupled to EHR 104 directly. An embodiment of clinician user device 108 includes a user interface operated by a software application or a set of applications on clinician user device 108. In an embodiment, the application is a Web-based application or applet. In accordance with embodiments presented herein, a healthcare provider (clinician) application may facilitate accessing and receiving information from a clinician about a specific patient or a set of patients for which the scratch events, future itch levels, and/or sleep detection are determined. Embodiments of clinician user device 108 also facilitate accessing and receiving information from a clinician about a specific patient or population of patients including patient history; healthcare resource data; physiological variables (e.g., vital signs), measurements, time series, predictions (including plotting or displaying the determined outcome and/or issuing an alert) described herein; or other health-related information. The clinician user device 108 further facilitates display of results, recommendations, or orders, for example. In an embodiment, clinician user device 108 facilitates receiving orders for the patient based on the results of monitoring and predictions. Clinician user device 108 may also be used for providing diagnostic services or evaluation of the performance of the technology described herein in conjunction with various embodiments.

Embodiments of decision support applications 105a and 105b comprise a software application or a set of applications (which may include programs, routines, functions, or computer-performed services) residing on a client computing device, one or more servers in the cloud, distributed in the cloud environment, or on a client computing device such as a personal computer, a laptop, a smartphone, a tablet, a mobile computing device, or front-end terminals in communication with back-end computing systems. In an embodiment, decision support applications 105a and 105b include Web-based applications or a set of applications usable to manage user services provided by an embodiment of the invention. For example, in an embodiment, each of the decision support applications 105a and 105b facilitates processing, interpreting, accessing, storing, retrieving, and communicating information acquired from user devices 102a-n and 108, sensor 103, EHR 104, or data store 150, including predictions and evaluations determined by embodiments of the invention.

Accessing and/or utilizing information through decision support applications 105a and 105b or utilizing associated functionality may require a user, such as a patient or a clinician, to login with credentials. Further, decision support applications 105a and 105b may store and transmit data in accordance with privacy settings defined by clinician, patient, an associated healthcare facility or system, and/or applicable local and federal rules and regulations regarding protecting health information, such as Health Insurance Portability and Accountability Act (HIPAA) rules and regulations.

In an embodiment, decision support applications 105a and 105b can send a notification (such as an alarm or other indication) directly to clinician user device 108 or user devices 102a-n through network 110. Decision support applications 105a and 105b may also send maintenance indications to clinician user device 108 or user devices 102a-n. Further, an interface component may be used in decision support applications 105a and 105b to facilitate access by a user (including a clinician/caregiver or patient) to functions or information on sensor 103, such as operational settings or parameters, user identification, user data stored on sensor 103, and diagnostic services or firmware updates for sensor 103, for example.

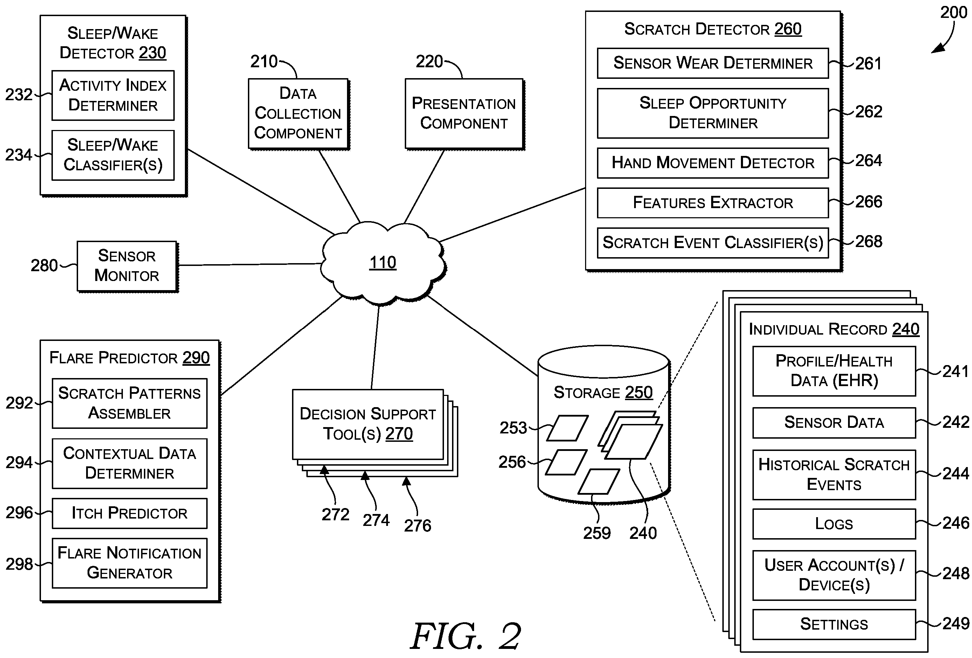

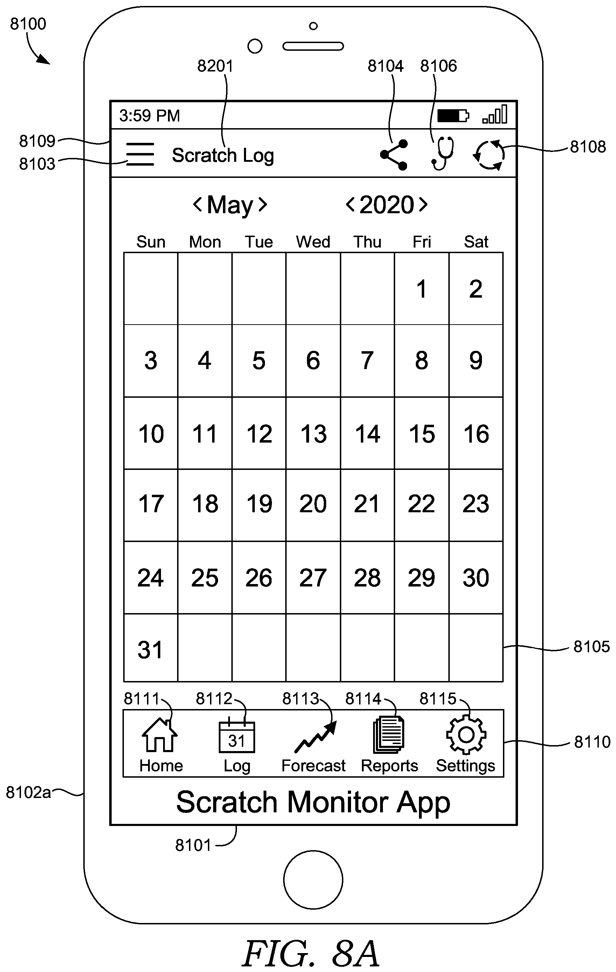

Further, embodiments of decision support applications 105a and 105b may collect sensor data directly or indirectly from sensor 103 and utilize the sensor data to detect scratch events, predict future itch levels and flare events, and/or detect sleep, as described further with respect to FIG. 2. As used herein, a flare event may refer to an acute phase of pruritus in which the level of itch and/or one or more additional symptoms (e.g., red skin, flaking skin, lesions) may exceed a threshold level. In one aspect, decision support applications 105a and 105b may display results of such processes to a user via a user device, such as user devices 102a-n and 108, including through example graphic user interfaces (GUIs) depicted in FIGS. 8A-F. In this way, the functionality of one or more components discussed below with respect to FIG. 2 may be performed by computer programs, routines, or services that are part of or otherwise controlled by decision support applications 105a and 105b. In addition, or alternatively, decision support applications 105a and 105b may include decision support tools, such as a decision support tool(s) 270 of FIG. 2.

As mentioned above, operating environment 100 includes one or more EHRs 104, which may be associated with a monitored individual. EHR 104 may be directly or indirectly communicatively coupled to user devices 102a-n and 108, via network 110. In some embodiments, EHR 104 represents health information from different sources and may be embodied as distinct records systems, such as separate EHR systems for different clinician user devices (such as 108). As a result, the clinician user devices may be for clinicians of different provider networks or care facilities.

Embodiments of EHR 104 include one or more data stores of health records, which may be stored on data store 150, and may further include one or more computers or servers that facilitate storing and retrieving health records. In some embodiments, EHR 104 may be implemented as a cloud-based platform or may be distributed across multiple physical locations. EHR 104 may further include record systems that store real-time or near real-time patient (or user) information, such as wearable, bedside, or in-home patient monitors, for example.

Data store 150 represents one or more data sources and/or data systems, which are configured to make data available to any of the various components of operating environment 100, or system 200 described in connection with FIG. 2. For instance, in one embodiment, data store 150 provides (or make available for accessing) sensor data, which may be available to a data collection component 210 of FIG. 2. Data store 150 may be discrete from user devices 102a-n and 108 and server 106, or may be incorporated and/or integrated with at least one of those components.

Operating environment 100 can be utilized to implement one or more of the components of system 200 (described in FIG. 2) including components for collecting sensor data or user-related data; detecting scratch events;

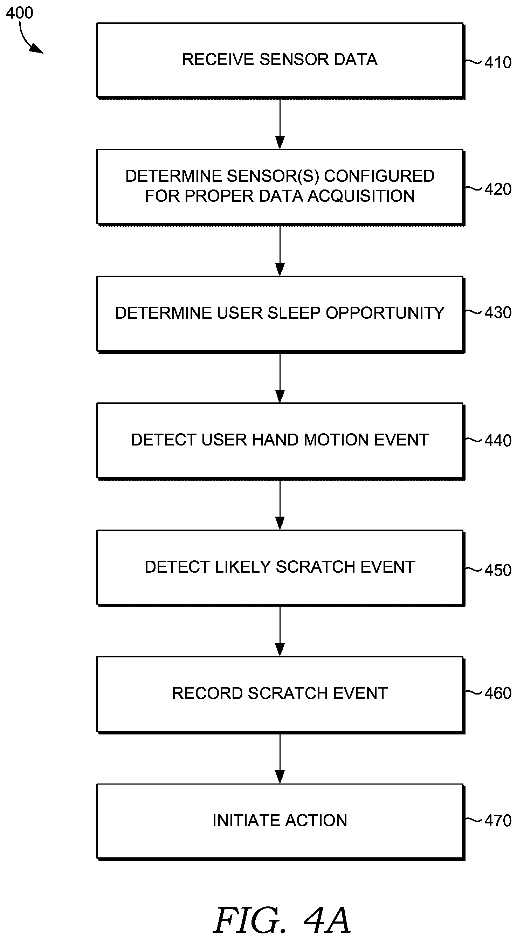

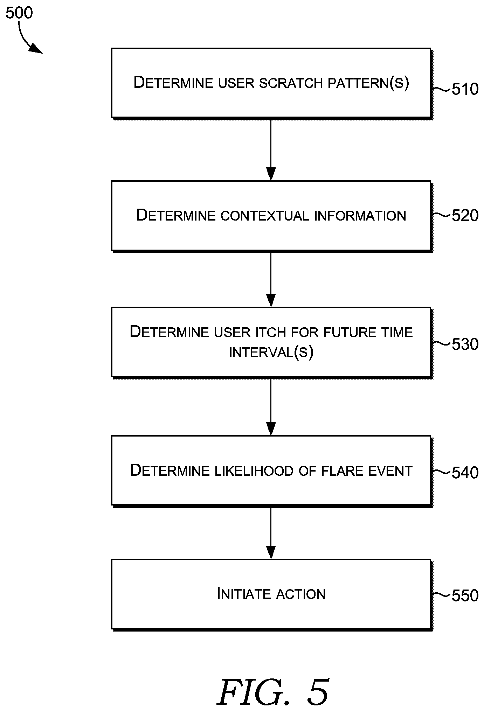

predicting future itch and flare events; detecting sleep; and implementing one or more decision support tools. Operating environment 100 can also be utilized for implementing aspects of methods 400 and 500, as described in conjunction with FIGS. 4A and 5, respectively.

Referring now to FIG. 2 and with continuing reference to FIG. 1, a block diagram is provided showing aspects of an example computing system architecture suitable for implementing an embodiment of the present disclosure and designated generally as system 200. System 200 represents only one example of a suitable computing system architecture. Other arrangements and elements can be used in addition to, or instead of, those shown, and some elements may be omitted altogether for the sake of clarity. Further, similar to operating environment 100, many elements described herein are functional entities that may be implemented as discrete or distributed components or in conjunction with other components, and in any suitable combination and location.

Example system 200 includes network 110, which is described in connection with FIG. 1, and which communicatively couples components of system 200 including a data collection component 210, a presentation component 220, a scratch detector 260, a sleep/wake detector 230, a flare predictor 290, a decision support tool(s) 270, a sensor monitor 280, and a storage 250. One or more of these components may be embodied as a set of compiled computer instructions or functions, program modules, computer software services, or an arrangement of processes carried out on one or more computer systems, such as computing device 1200 described in connection with FIG. 12, for example.

In one embodiment, the functions performed by components of system 200 are associated with one or more decision support applications, services, or routines (such as decision support applications 105a-b of FIG. 1). In particular, such applications, services, or routines may operate on one or more user devices (such as user computer device 102a and/or clinician user device 108), servers (such as server 106), distributed across one or more user devices and servers, or implemented in the cloud environment (not shown). Moreover, in some embodiments, these components of system 200 may be distributed across a network, connecting one or more servers (such as server 106) and client devices (such as user computer devices 102a-n or clinician user device 108), in the cloud, or may reside on a user device, such as any of user computer devices 102a-n or clinician user device 108. Moreover, functions performed by these components, or services carried out by these components may be implemented at appropriate abstraction layer(s) such as an operating system layer, an application layer, a hardware layer, or so on of the computing system(s). Alternatively, or in addition, the functionality of these components and/or the embodiments described herein can be performed, at least in part, by one or more hardware logic components. For example, and without limitation, illustrative types of hardware logic components that can be used include Field-Programmable Gate Arrays (FPGAs), Application-Specific Integrated Circuits (ASICs), Application-Specific Standard Products (ASSPs), System-on-a-Chip systems (SoCs), Complex Programmable Logic Devices (CPLDs), etc. Additionally, although functionality is described herein with regards to specific components shown in example system 200, it is contemplated that in some embodiments functionality of these components can be shared or distributed across other components.

Continuing with FIG. 2, data collection component 210 may be generally responsible for accessing or receiving (and in some cases identifying) data from one or more data sources, such as data from sensor 103 and/or data store 150 of FIG. 1, to utilize in embodiments of the present disclosure. In some embodiments, data collection component 210 may be employed to facilitate accumulation of sensor data acquired for a particular user (or in some cases, a plurality of users including crowdsourced data) for other components of system 200, such as scratch detector 260, sleep/wake detector 230 and/or flare predictor 290. The data may be received (or accessed), and accumulated, reformatted, and/or combined, by data collection component 210 and stored in one or more data stores such as storage 250, where it may be available to other components of system 200. For example, the user data may be stored in or associated with an individual record 240, as described herein. Additionally, or alternatively, in some embodiments, any personally identifiable data (i.e., user data that specifically identifies particular users) is not uploaded, otherwise provided from the one or more data sources with user data, not permanently stored, and/or not made available to other components of system 200. In some embodiments, a user may opt into or out of services provided by the technologies described herein and/or select which user data and/or which sources of user data are to be utilized by these technologies.

Data utilized in embodiments of the present disclosure may be received from a variety of sources and may be available in a variety of formats. For example, in some embodiments, user data received via data collection component 210 may be determined via one or more sensors (such as sensor 103 of FIG. 1), which may be stored on or associated with one or more user devices (such as user computer device 102a), servers (such as server 106), and/or other computing devices. As used herein, a sensor may include a function, a routine, a component, or a combination thereof for sensing, detecting, or otherwise obtaining information, such as user data from data store 150, and may be embodied as hardware, software, or both. As mentioned earlier, by way of example and not limitation, data that is sensed or determined from one or more sensors may include motion information, such as accelerometer or gyroscope data; ambient light information, such as photodetector information; location information, such as an Indoor Positioning System (IPS) or Global Positioning System (GPS) data from a mobile device; atmospheric information, such as temperature, humidity, and/or air pressure; and physiological information, such as heart rate, blood pressure, core body temperature, skin temperature, or galvanic skin response. In some aspects, sensor information collected by data collection component 210 may include further properties or characteristics of the user device(s) (such as a device state, charging data, date/time, or other information derived from a user device such as a mobile device); user-activity information (for example, app usage, online activity, online search, voice data such as automatic speech recognition, or activity log) including, in some embodiments, user activity that occurs on more than one user device; user history; session logs; application data; contacts; calendar and schedule data; notification data; social-network data; news (including popular or trending items on search engines or social networks); ecommerce activity (including data from online accounts such as Microsoft®, Amazon.com®, Google®, eBay®, PayPal®, etc.); user-account(s) data (which may include data from user preferences or settings associated with a personal assistant application or service); home-sensor data; appliance data; vehicle signal data; traffic data; other wearable device data; other user device data (for example, device settings, profiles, network-related information (e.g., a network name or ID, domain information, workgroup information, connection data, Wi-Fi network data, or configuration data, data regarding a model number, firmware, equipment, device pairings, such as where a user has a mobile phone paired with a Bluetooth headset, for example, or other network-related information)); payment or credit card usage data (which may include information from a user's PayPal® account); purchase history data (such as information from a user's Amazon.com® or online drugstore account); other sensor data that may be sensed or otherwise detected by a sensor (or other detector) component(s) including data derived from a sensor component associated with the user (including location, motion, orientation, position, user-access, user-activity, network-access, user-device-charging, or other data that is capable of being provided by one or more sensor components); data derived based on other data (for example, location data that can be derived from Wi-Fi, Cellular network, or Internet Protocol (IP) address data); and nearly any other source of data that may be sensed or determined, as described herein.

In some aspects, data collection component 210 may provide data collected in form of data streams or signals. A "signal" can be a feed or stream of data from a corresponding data source. For example, a user signal could be user data from a wearable device, a smartphone, a home-sensor device, a GPS device (e.g., for location coordinates), a vehicle-sensor device, a user device, a gyroscope sensor, an accelerometer sensor, a calendar service, an email account, a credit card account, or other data sources. In some embodiments, data collection component 210 receives or accesses data continuously, periodically, or on as needed basis. Data collection component 210 may obtain data at a predetermined sampling rate. In one example, data collection component 210 utilizes a sampling rate of 100 Hz for one or more data signals, such as accelerometer signal, ambient light signal, and a body temperature signal.

Sensor monitor 280 may be generally responsible for monitoring collected data for information that may be used for detecting scratch, predicting flare (including predicting itch), and/or detecting sleep, which may include identifying and/or tracking features (sometimes referred to herein as "variables"), such as motion or accelerometer data or other related contextual information. In an embodiment, sensor monitor 280 comprises one or more applications or services that analyze information detected via one or more sensors integrated into or communicatively coupled to user devices used by the user and/or cloud-based services associated with the user, to determine motion information and related contextual information. For instance, sensor monitor 280 may comprise a service of a decision support application, such as any of decision support applications 105a-b of FIG. 1, or may be integrated as part of another application or program on a device working in conjunction with the decision support application. Information about user devices associated with a user may be determined from the user data made available via data collection component 210, and provided to sensor monitor 280 or other components of system 200. In some embodiments, sensor monitor 280 runs on or in association with each user device associated with a monitored individual (or user).

Additionally, sensor monitor 280 may determine current or near real-time information, such as motion information and, in some embodiments, may also determine historical motion information, which may be determined based on individual record 240. Further, in some embodiments, sensor monitor 280 may determine motion information, detected scratch data, predicted itch/flare events, and detected sleep/wake periods (which may include historical activity) from other similar users (i.e., crowdsourcing), as described previously.

In some embodiments, information determined by sensor monitor 280 may be provided to scratch detector 260, flare predictor 290, and sleep/wake detector 230, including motion information acquired from a sensor (such as sensor 103 in FIG. 1), context (such as current or future weather forecasts) and historical context (historical observations) for the monitored individual.

Some embodiments of sensor monitor 280, or its subcomponents, may determine a device name or identification (device ID) for each device associated with a user. This information about the identified user device(s) associated with a user may be stored in a user profile associated with the user, such as in user account(s)/device(s) 248 of individual record 240. In an embodiment, the user devices may be polled, interrogated, or otherwise analyzed to determine information about the devices. This information may be used for determining a label or an identification of the device (e.g., a device ID) so that user interaction with the device may be recognized from user data by sensor monitor 280. In some embodiments, users may declare or register a device, such as by logging into an account via the device, installing an application on the device, connecting to an online service that interrogates the device, or otherwise providing information about the device to an application or a service. In some embodiments, devices that sign into an account associated with the user, such as an email account, social network, or the like, are identified and determined to be associated with the user.

Continuing with system 200 of FIG. 2, scratch detector 260 is generally responsible for utilizing sensor data, such as data accumulated by data collection component 210 from sensor 103, to detect scratch by a monitored individual. As described herein, the scratch event detected by scratch detector 260 may be stored in a record of the monitored individual, such as historical scratch events 244 of individual record 240. Historical scratch events 244 may be utilized to make predictions about individual's future behavior, such as future itch level or flare event, by flare predictor 290, and/or may be provided to one or more decision support tool(s) 270. In some embodiments, scratch detector 260 may run on a client computing device, a server, a distributed application across multiple devices, or in the cloud environment.

At a high level, an embodiment of scratch detector 260 may utilize sensor data of a monitored individual to detect individual's hand movement and classify that hand movement as a scratch event or not. In some implementations, the sensor data considered for detecting hand movement is data acquired during a period in which the sensor 103 is properly worn. Further, an embodiment of scratch detector 260 detects nighttime scratch by detecting scratch events within a user's sleep opportunity, which is a period of time when the user intends to sleep.

As shown in FIG. 2, embodiments of scratch detector 260 may comprise a sensor wear determiner 261, a sleep opportunity determiner 262, a hand movement detector 264, a features extractor 266, and a scratch event classifier(s) 268. Sensor wear determiner 261 may be generally responsible for determining when a sensor (such as 103), acquiring motion data for embodiments of scratch detector 260 (or other components of system 200), is being worn by the monitored user. In exemplary embodiments, sensor wear determiner 261 specifically determines when the sensor 103 is worn in a configuration for providing reliable data. A configuration for providing reliable data may include being in a proper placement on or within proximity to the monitored individual. For instance, in an embodiment in which a sensor on a wristband is acquiring motion data of the wearer, sensor wear determiner 261 may determine that the wristband is secured around the wearer's wrist. Determining when a sensor is being worn properly helps to ensure embodiments of scratch detector 260 utilizing motion data within the intended context (e.g., around the user's wrist) to detect scratch events.

In example embodiments, sensor wear determiner 261 may automatically determine when the sensor 103 is being worn, utilizing data received from the sensor 103 or another sensor (not shown). For instance, sensor wear determiner 261 may automatically determine when sensor 103 capturing motion data is being worn utilizing motion data, physiological data, such as human body temperature, heart rate, blood pressure, pulse, galvanic skin response, etc., received from a sensor on a device acquiring motion data. Alternatively, sensor wear determiner 261 may determine when a device is being worn based on manual indication by the wearer. For instance, the wearer may enter an indication when the device is being worn and when it is taken off. In another instance, the wearer may enter times corresponding to these events.

As such, in one embodiment, sensor wear determiner 261 determines a period of non-wear configuration by comparing statistical measurements of motion data over windows of time to a non-wear threshold. For example, accelerometer data, which may comprise x, y, and z measurements, may be divided into windows of time, and statistical measurements may be computed and utilized with one or more heuristic rules to determine a wear configuration or a non-wear configuration. In an exemplary embodiment, the accelerometer data may be divided into multiple one-hour windows with 15 minutes overlap. A non-wear determination may be a vector of binary values representing wear/non-wear configuration for each window of the motion data. A window where a period of non-wear is not detected may be considered a period of wear.

In an example embodiment, sensor wear determiner 261 may determine whether sensor 103 is in a worn configuration or not during a window of time by comparing a statistical features of motion data in the window to a predefined threshold value. For example, in an embodiment, sensor wear determiner 261 determines whether the standard deviation of any of the three axes (x-axis, y-axis, or z axis) signals of accelerometer data within a window satisfies a non-wear motion threshold value, and if so, that window is determined to be non-wear. In an exemplary embodiment, the non-wear motion threshold is 0.001 g, and sensor wear determiner 261 determines that a window is non-wear if the standard deviation of values of any axes is less than the non-wear motion threshold.

In another example embodiment, sensor wear determiner 261 may determine whether the sensor 103 is in the worn configuration or not by comparing a temperature during a window (or interval) of time to a non-wear temperature threshold. In one exemplary embodiment, the non-wear temperature threshold is 25 degrees Celsius (i.e., 77 degrees Fahrenheit), and sensor wear determiner 261 determines that a window is non-wear if the temperature during that window is less than the non-wear temperature threshold.

Further, in exemplary embodiments, sensor wear determiner 261 considers both motion data and temperature data to determine whether to classify a window of time as wear or non-wear. In one exemplary embodiment, sensor wear determiner 261 determines that a window is non-wear if the temperature is less than the non-wear temperature threshold (e.g., 25 degrees Celsius) or if the standard deviation of values of any axes of motion data within the window is less than the non-wear motion threshold (e.g., 0.001 g).

In some embodiments, multiple statistical features may be computed for motion data and compared to thresholds to determine whether the window is a period of non-wear or not. In one exemplary embodiment, if any two axes have a standard deviation that satisfies (i.e., is less than) a non-wear standard deviation motion threshold, the period is determined as a non-wear window, or if any two axes have a range that satisfies (i.e., is less than) a non-wear range motion threshold, the period is detected as a non-wear window. In an example, a non-wear standard deviation motion threshold is approximately 0.013 g, and an example non-wear range motion threshold is 0.15 g.

In further aspects, the above processes may provide an initial wear/non-wear determination, and sensor wear determiner 261 may apply heuristic rule(s) to rescore one or more windows. Rescoring may help identify times where interruptions in the data indicate that the device is not worn, but contextual information, such as the length of time of this interruption and the accelerometer data occurring before or after, may indicate otherwise (i.e., may indicate that the device is being worn).

In an example embodiment, the heuristic rules consider the lengths of time of the wear and non-wear blocks to determine whether to switch a wear/non-wear determination for any of the blocks of time. As used herein with respect to rescoring by sensor wear determiner 261, blocks of time may be successive windows with the same wear or non-wear classification. For instance, three successive one-hour windows, initially determined to be "non-wear", form a three-hour block of non-wear. In an example embodiment, sensor wear determiner 261 applies the following for rescoring one or more windows:

- Rules 1 and 2 may be performed a number of times in succession, such as three times, and rule 3 may be performed only if sensor wear determiner 261 is running on a predetermined time of data, such as the last 24 hours of data.

- In accordance with rules 1, 2, and 3, "current" refers to a current block of wear being examined; "prev" refers to a preceding non-wear block; and "post" refers to a next non-wear block.

- Rule 1: If current < 3 hours and (current/(prev + post)) < 80%, block is rescored from wear to non-wear.

- Rule 2: If current < 6 hours and (current/(prev + post)) < 30%, block is rescored from wear to non-wear.

- Rule 3: If current < 3 hours and prev ≥ 1 hour, block is rescored from wear to non-wear.

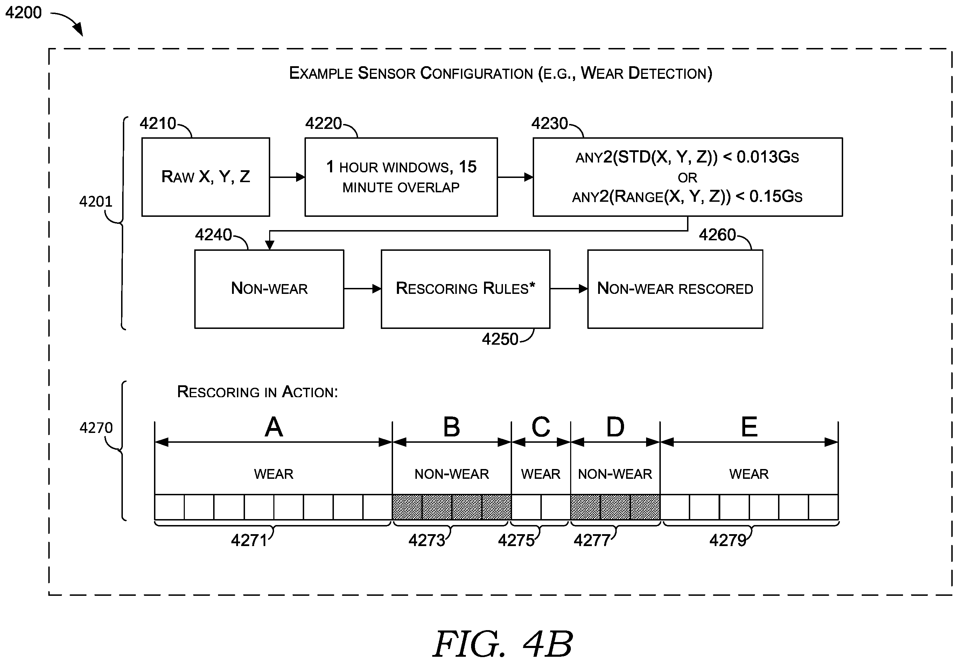

Further details of an embodiment of sensor wear determiner 261 may be implemented as described below in conjunction with FIG. 4B.

Additionally, prior to sensor wear determiner 261 determining a wear configuration, motion data may be preprocessed and filtered. For example, motion data may be first down-sampled, such as from 100 Hertz (Hz) to 20 Hz. Additionally, data may be segmented into relevant periods of time for which a scratch analysis is detected. For example, data may be separated into 24-hour segments (12:00pm today to 12:00pm the following day). Further, in some embodiments, any 24-hour period that does not have a minimum amount of recording time, such as 6 hours, may be discarded and not analyzed further by scratch detector 260.

As part of scratch detector 260, sleep opportunity determiner 262 may be generally responsible for determining a user's sleep opportunity. As used herein, sleep opportunity refers to an interval of time in which an individual intends to sleep, which may or may not be consistent with when the individual actually sleeps. As such, in some embodiments, the sleep opportunity is the time between when an individual lays down to rest and gets up from rest. A user's sleep opportunity within a predefined period may also be referred to as a total sleep opportunity (TSO). For instance, for a 24-hour period, individuals typically intend to go to sleep only once (e.g., at nighttime), and sleep opportunity determiner 262 may determine the total sleep opportunity to be the longest interval during that 24-hour period in which a user intends to rest.

The determination of a user's sleep opportunity may be utilized to focus sensor data within the context of nighttime or sleep scratching for further processing by scratch detector 260. For instance, scratch detector 260 may detect nighttime scratching by specifically detecting scratch events based on motion data captured during the period of time determined to represent the user's sleep opportunity by sleep opportunity determiner 262. The term "nighttime" is used herein to represent a typical period in which an individual takes the longest rest; however, it is contemplated that embodiments of this disclosure are not limited to detecting scratch at night. For instance, some individuals, such as individuals who work evenings or overnight, may take their longest rest or sleep during the day, and the sleep opportunity for such individuals may be a daytime interval.

Sleep opportunity determiner 262 may determine user's sleep opportunity for motion data captured over a predefined period, such as a 24-hour period. Example implementations of sleep opportunity determiner 262 may apply a heuristic approach based on a change in arm angle determined from motion data to determine candidate sleep opportunity periods. A largest consecutive group of candidate rest periods within the predefined period (e.g., 24-hour) may be selected as the user's sleep opportunity. In exemplary aspects, sleep opportunity determiner 262 may determine the sleep opportunity utilizing only motion data within the predefined period in which sensor wear is detected by sensor wear determiner 261, while non-wear periods are excluded by sleep opportunity determiner 262 when identifying the largest group of candidate rest periods.

In some aspects, an arm angle is computed from accelerometer signals (x-axis, y-axis, and z-axis measurements), and an absolute difference between successive arm angle values (i.e., a change in arm angle over time) may be compared to a rest threshold. In an example embodiment, a rolling median of raw signal values (x-axis, y-axis, and z-axis measurements) is computed over an interval (e.g., 5 seconds), and the rolling median of raw signal values are utilized to calculate arm angle in accordance with the following formula, where $a_x$, $a_y$, and $a_z$ refer to accelerometer values along the x-axis, y-axis, and z-axis respectively and :

$$arm\_angle_z = \left(tan^{-1} \frac{a_z}{\sqrt{a_x^2 + a_y^2}}\right) \times \frac{180}{\pi}$$

An average arm angle may be computed for an interval (e.g., consecutive 5 seconds), and the absolute difference between successive average arm angle values may be computed. A rolling median of the difference between successive average arm angle values may be computed for an interval (e.g., 5 minutes), and the rolling median of the difference between successive average arm angle values may be compared to a rest threshold. The rest threshold may be defined by arm angle values measured for the monitored individual. For example, in one embodiment, a candidate rest period is determined when the median difference between successive average arm angle values is less than or equal to the rest threshold, which may be defined as 0.15 multiplied by the 10th percentile value of all differences in arm angle values within the 24-hour period.

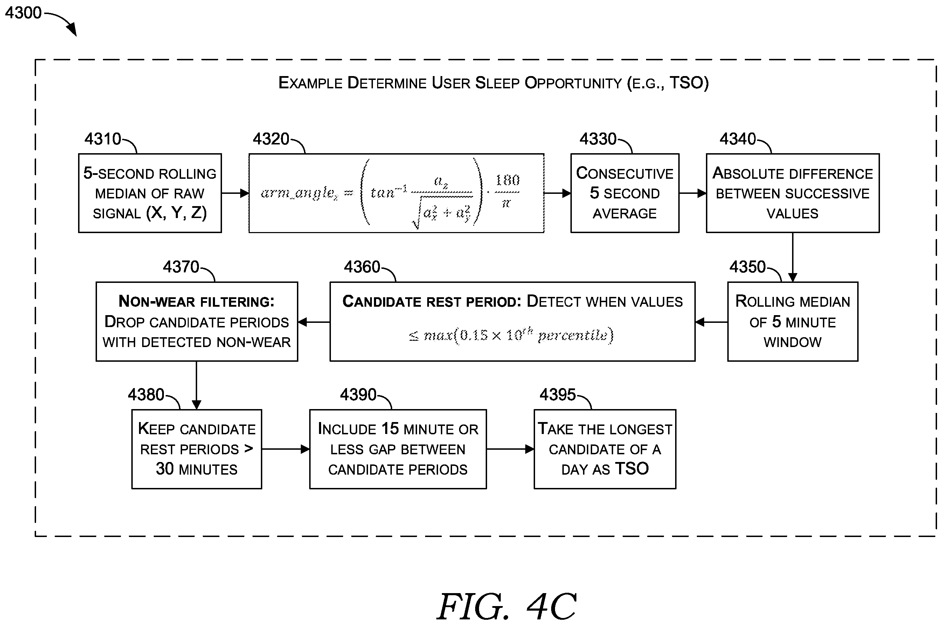

Sleep opportunity determiner 262 may determine the sleep opportunity based on the intervals identified as candidate rest periods. In an example, candidate periods with periods of detected non-wear are removed. The remaining candidate rest periods may be compared to a threshold length. In one implementation, the threshold length is 30 minutes, and candidate rest periods are kept if they are greater than 30 minutes. Additionally, candidate periods may be grouped together if the gaps between the periods satisfy a maximum length of time. For instance, candidate periods with a gap less than 15 minutes may be grouped together. In one example, sleep opportunity determiner 262 may determine the user's sleep opportunity to be the longest group of candidate periods within the 24-hour period. Further details of an embodiment of sleep opportunity determiner 262 are discussed further below in conjunction with FIG. 4C.

Reliably detecting sleep opportunity within which to measure scratch helps effectively determine how an individual's sleep and nighttime scratch vary on a day-to-day basis. Embodiments of this disclosure may utilizing a sleep opportunity that captures difficulties falling asleep by not limiting the sleep opportunity to times when the user is actually asleep.

Other implementations of sleep opportunity determiner 262 may determine the sleep opportunity from other sensor data. For example, in one embodiment, sleep opportunity determiner 262 may determine the sleep opportunity utilizing light information from a photodetector, and the sleep opportunity may be determined as a period of time in which the amount of light remains below a threshold level for a minimum time period. Alternatively, physiological data, such as heart rate, core body temperature, near body temperature, blood pressure, and/or respiration rate, captured from the monitored individual may be utilized to determine the sleep opportunity. Further, in some aspects, sleep opportunity determiner 262 may determine the sleep opportunity from user-entered data. For example, a user may input times corresponding to when the user intends to go to sleep and wake up or times corresponding to when the user did go to sleep and wake up.

As previously stated, embodiments of scratch detector 260 utilize a two-tier approach to detect scratch events. In some embodiments, hand movements may be detected, and each detected hand movement may be classified as a scratch event or a non-scratch event. Hand movement detector 264 is generally responsible for detecting hand movement using motion sensor information. Example embodiments of hand movement detector 264 may receive (from sensor 103) motion sensor information, such as accelerometer data and/or gyroscopic data. In one embodiment, hand movement detector 264 may output an indication of hand motion for the received data.

In exemplary aspects, hand movement detector 264 may apply a heuristic algorithm to motion sensor data captured during a sleep opportunity, which may be determined by sleep opportunity determiner 262. The motion sensor data, such as accelerometer data, may be segmented into windows of pre-determined length, and motion sensor data for each window may be passed through a heuristic hand movement detection algorithm to determine the presence of hand movement. An example embodiment utilizes three-second non-overlapping windows within the sleep opportunity for a given 24-hour period. It is contemplated that other windows may be utilized, such as a one-second window or a two-second window for instance.

In exemplary aspects, the hand movement detection algorithm includes computing the vector magnitude of the motion sensor signal (e.g., $\sqrt{x^2 + y^2 + z^2}$). A low pass filter may be applied to the vector magnitude signal, in accordance with some embodiments. In an example embodiment, the low-pass filter has a 6 Hz cutoff. The hand movement detection algorithm may further include calculating a rolling coefficient of variation (CoV) and applying a threshold to the calculated CoV values. As used herein, CoV refers to a relative standard deviation or a ratio of standard deviation to the mean. Any values that satisfy the threshold (e.g., are above or equal to) may be determined to be a hand movement. **In** some embodiments, this threshold utilized is 25th percentile of all calculated CoV values from testing data. In an example embodiment, the CoV threshold is 0.023.

The rolling CoV may be computed for each second within a non-overlapping 3-second window. For instance, for accelerometer data of 20 Hz, or 20 samples per second, hand movement detector 264 may make 60 classifications of hand movement for each non-overlapping 3-second window.

In an embodiment, hand movement may be detected for a given window if it is present for each second within that window utilizing the CoV threshold. For instance, hand movement detector 264 may detect hand movement for a three-second window if movement is detected for each of the three seconds within that window.

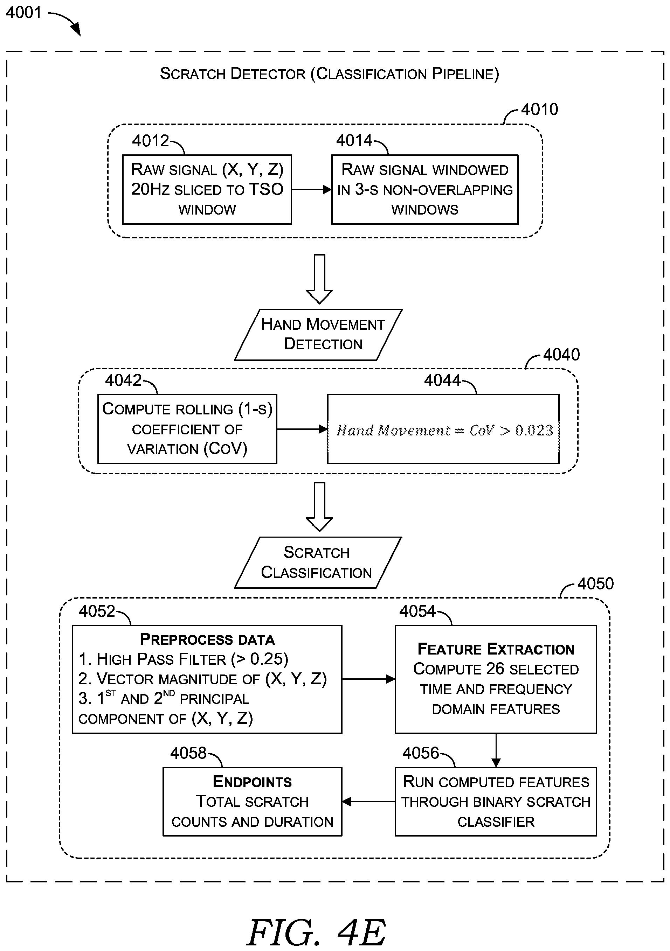

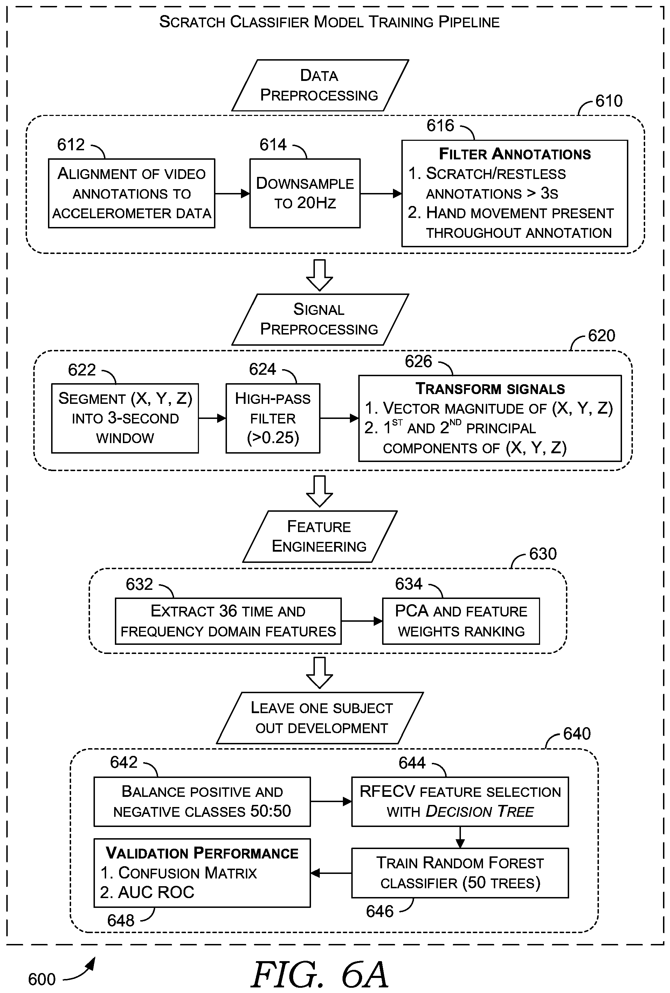

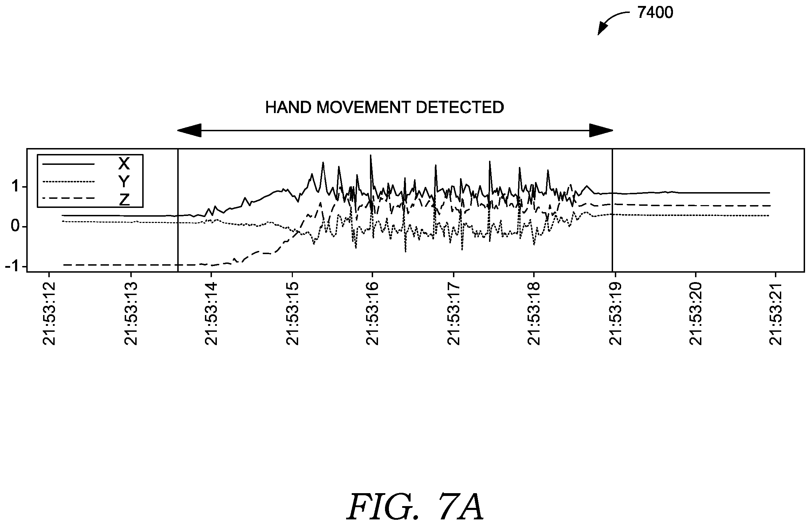

Further details of an embodiment of hand movement detector are described in conjunction with FIG. 4E. Additionally, example outputs of an embodiment of hand movement detector 264 are depicted in conjunction with FIGS. 6A and 7A.

Once hand movement detector 264 identifies a hand movement event, the motion sensor information corresponding to the detected hand movement event may be considered as a potential scratch event. In some embodiments, determining whether the hand movement event is a scratch event may include analyzing features within motion sensor data. In one such embodiment, features extractor 266 may generally be responsible for extracting feature information that may be indicative of a scratch motion. Features may be extracted from motion sensor data corresponding to the hand movement detected by hand movement detector 264. In extracting features, feature values may be computed for each window (e.g., 3-second window) for which hand motion is detected.

Features may be extracted from one or more components of motion sensor data in the form of a motion signal. For example, in some embodiments, a vector magnitude, a first principal component, and a second principal component of accelerometer signal are each utilized for feature extraction. Additionally, in some embodiments, a filter is applied to the motion sensor data prior to feature extraction. In one instance, a high-pass filter with a 0.25 Hz cutoff may be applied prior to feature extraction, which may help to remove drift and the contribution of gravity. Alternatively, in another instance, a band filter may be applied.

In exemplary embodiments, the features fall within the time domain or frequency domain. Example embodiments of features extractor 266 may extract, or compute, one or more of the following features:

- Root mean square (RMS) value of vector magnitude - RMS is a measure of signal energy and may be correlated with amount and intensity of motion;

- Signal range of vector magnitude - Signal range is a measure of the extremes of motion observed in a given time window of sensor data, where a higher range may indicate occurrence of a large excursion in sensor values;

- Signal entropy of vector magnitude, first principal component, and second principal component - Signal entropy may be calculated by estimating Shannon entropy of the probability mass function of a signal. Signal entropy values close to zero may indicate that the signal is periodic and smooth, whereas large negative values may indicate that the signal is irregular and non-periodic;

- Interquartile range (IQR) of auto-covariance of vector magnitude, first principal component, and second principal component - IQR of auto-covariance is a measure of long-range dependency or periodicity of a signal and may capture if the signal is periodic or irregular;

- Skewness of vector magnitude, first principal component, and second principal component - Skewness is a measure of asymmetry in a signal;

- Dominant frequency value of first principal component and second principal component - Dominant frequency value is the value of the frequency with the highest magnitude in the normalized power spectrum of the accelerometer signal and captures the fundamental frequency of the underlying movement producing the acceleration signal;

- Dominant frequency magnitude of first principal component and second principal component - Dominant frequency magnitude captures the percentage of total signal energy in the dominant frequency;

- Ratio of dominant frequency band to total energy in spectrum of first principal component and second principal component - This feature captures periodicity of a signal by calculating the ratio of the energy in the dominant frequency component to the sum of energy in the entire frequency spectrum of a signal;

- Mean cross rate of vector magnitude, first principal component and second principal component - Mean cross rate calculates the number of times the signal changes from positive to negative and may be normalized by total signal length;

- Jerk ratio of vector magnitude, first principal component and second principal component - Jerk ratio may be calculation of smoothness of motion;

- Log dimensionless jerk of vector magnitude, first principal component, and second principal component - This feature may also be a calculation of smoothness of motion;

- Spectral arc length measure (SPARC) of vector magnitude, first principal component, and second principal component - This feature may also be a calculation of smoothness of motion;

- Permutation entropy of vector magnitude, first principal component, and second principal component - Permutation entropy is a measure of complexity of a signal;

- Spectral flatness of first principal component and second principal component - Spectral flatness captures the amount of modulation or the level of consistency and may range from 0 to 1; and

- Spectral entropy of first principal component and second principal component - Spectral entropy may be calculated by estimating Shannon entropy of the probability mass function of the power spectrum of a signal, where values closer to 1 indicate presence of white noise and values closer to 0 indicate presence of periodicity in the signal.

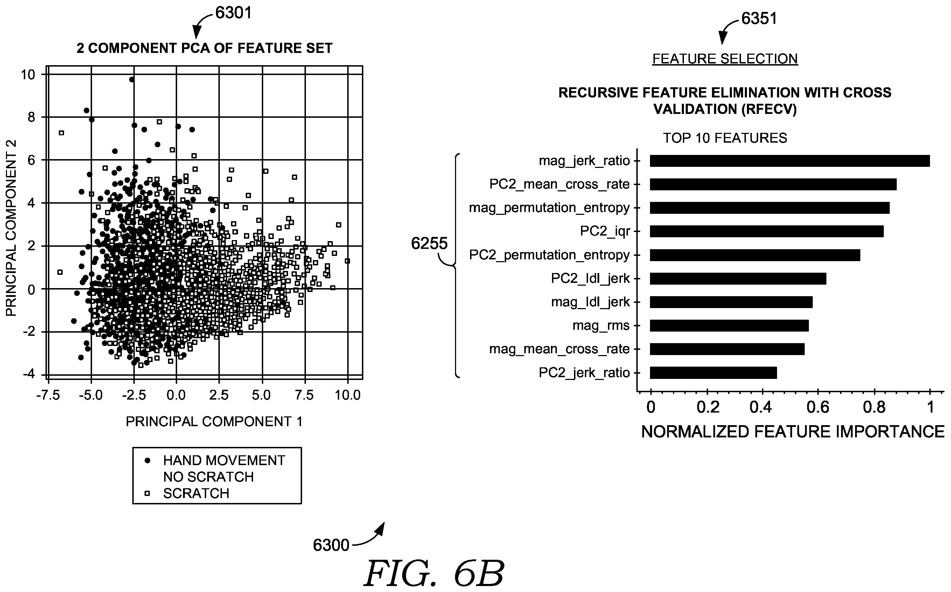

In example embodiments, each of the above 36 time and frequency domain features (where features of vectors magnitude, first principal component and second principal component are separate features) may be extracted during training of scratch event classifier(s) 268, and a subset of the features are selected to be extracted by features extractor 266 during runtime. For instance, one embodiment of features extractor 266 extracts the following 26 time and frequency domain features: RMS (vector magnitude); signal entropy (vector magnitude, first principal component, and second principal component); IQR of auto-covariance (vector magnitude, first principal component, and second principal component); skewness (first principal component and second principal component); dominant frequency value (first principal component); dominant frequency magnitude (first principal component and second principal component); mean cross rate (second principal component); jerk ratio (vector magnitude and second principal component); log dimensionless jerk (first principal component); SPARC (vector magnitude, first principal component, and second principal component); permutation entropy (vector magnitude, first principal component, and second principal component); spectral flatness (first principal component and second principal component); spectral entropy (second principal component); and signal range (vector magnitude). Alternative embodiments of features extractor 266 may extract values for different combinations of the above and/or other features. The particular features for extraction by features extractor 266 may be determined from feature selection and feature engineering. An example process for feature selection is described in connection with FIGS. 6A and 6B.

Continuing with scratch detector 260, scratch event classifier(s) 268 is generally responsible for determining whether to classify a motion signal as a scratch event. Embodiments of scratch event classifier 268 may utilize at least the extracted features of the motion signal (as determined by features extractor 266) to output a classification of the motion signal as a scratch event or not a scratch event (i.e., non-scratch event). As discussed earlier, the extracted features may be extracted from windows (e.g., 3-second windows) of motion signal corresponding to a detected hand movement such that the classification may determine whether the hand motion represents a scratch event or not.

In some embodiments, scratch event classifier 268 may utilize scratch-event detection logic 256 in storage 250 to determine whether motion signal is a scratch event or not. Scratch-event detection logic 256 may include rules, conditions, associations, machine learning models, or other criteria for inferring or detecting a likelihood of a scratch event based on motion sensor data. For example, scratch-event detection logic 256 may determine, from the accelerometer data, a probability that the detected movement was caused by a user scratching his or her body. Scratch-event detection logic 256 may take different forms depending on the mechanism(s) used to detect scratching. In some embodiments, scratch-event detection logic 256 may comprise fuzzy logic, a neural network(s), a finite state machine, a support vector machine, a logistic regression, clustering, other machine-learning techniques, similar statistical classification processes, or combinations of these to identify likely scratch events. Specifically, some exemplary embodiments of scratch-event detection logic 256 may include one or more binary machine learning classifiers. Scratch-event detection logic 256 may comprise an ensemble of machine learning models. In one embodiment, scratch-event detection logic 256 may be a random forest classifier. In another embodiment, gradient boosting may be utilized.

Model(s) forming the scratch-event detection logic 256 may be trained in accordance with embodiments of this disclosure. In one embodiment, scratch event classifier 268 is trained on an annotated training data and validated using leave-one-subject-out (LOSO) process. Further details of training are disclosed with reference to embodiments described in connection with FIGS. 6A-F.

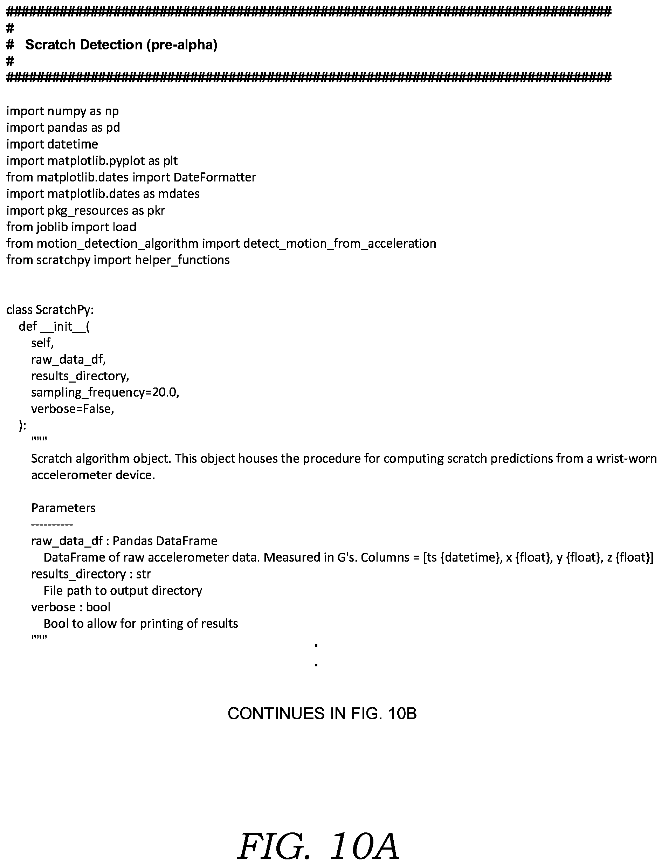

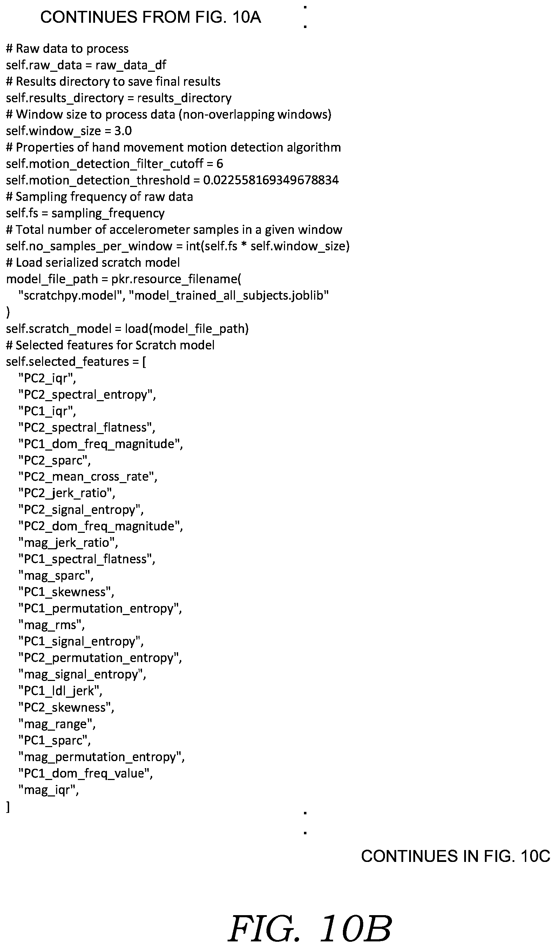

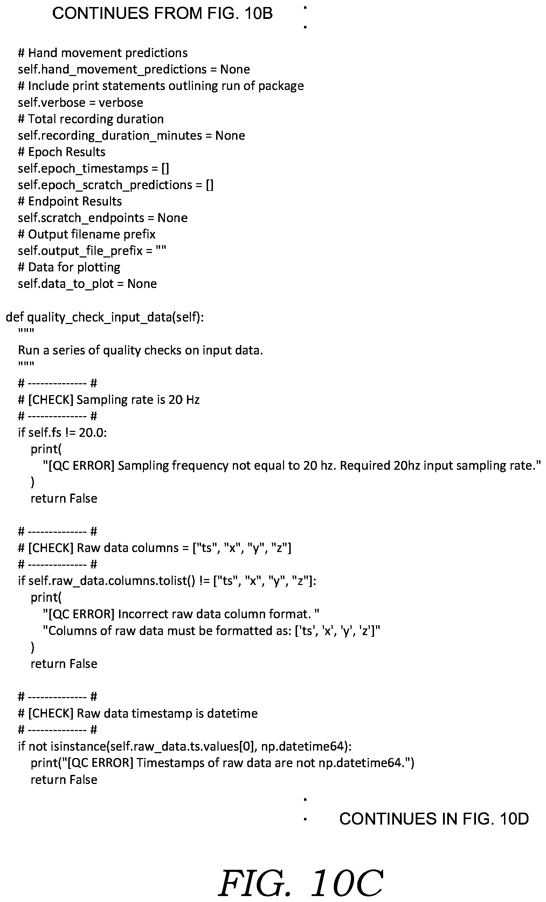

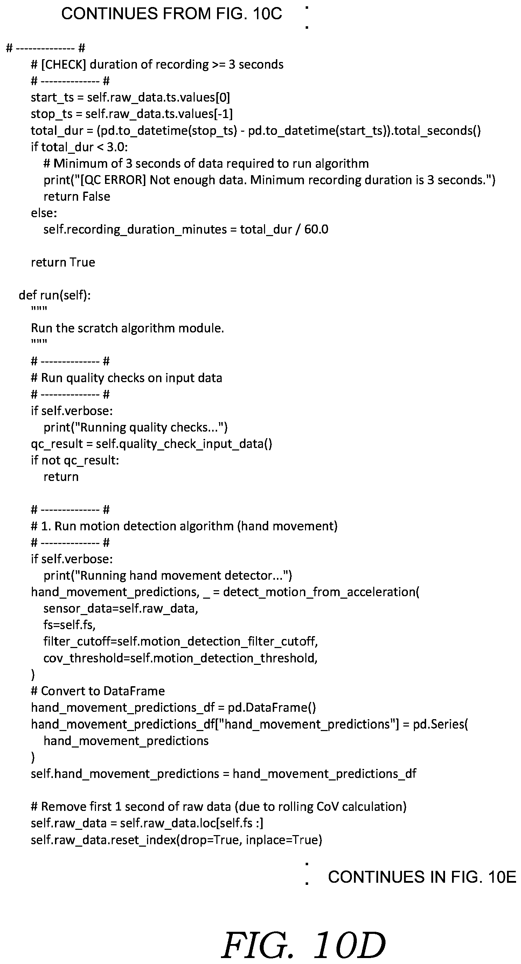

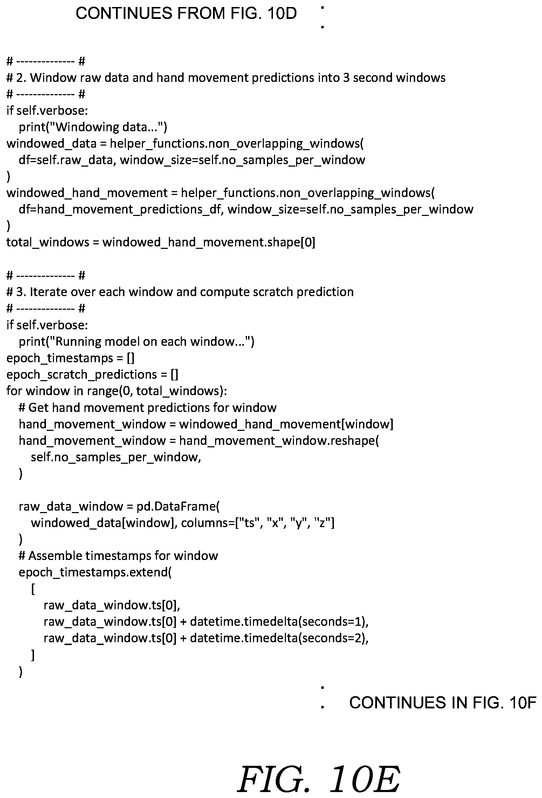

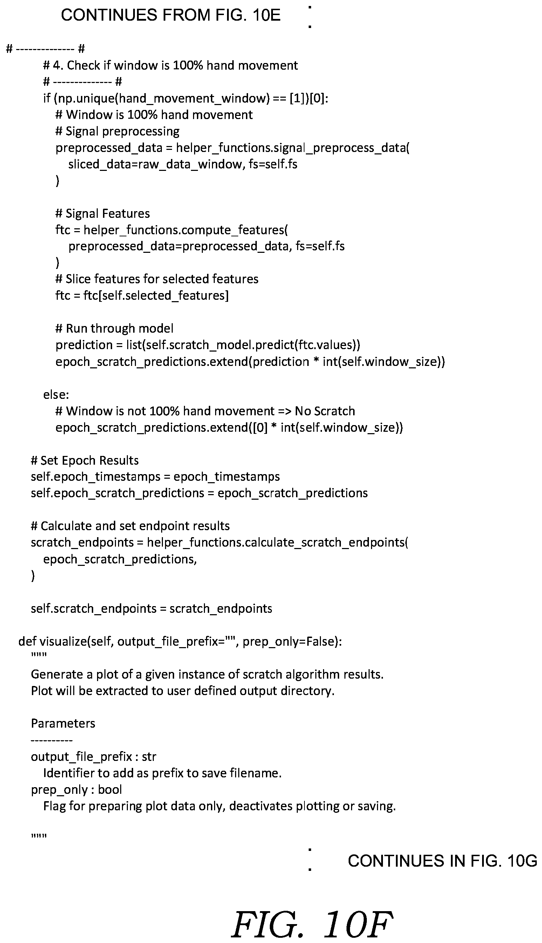

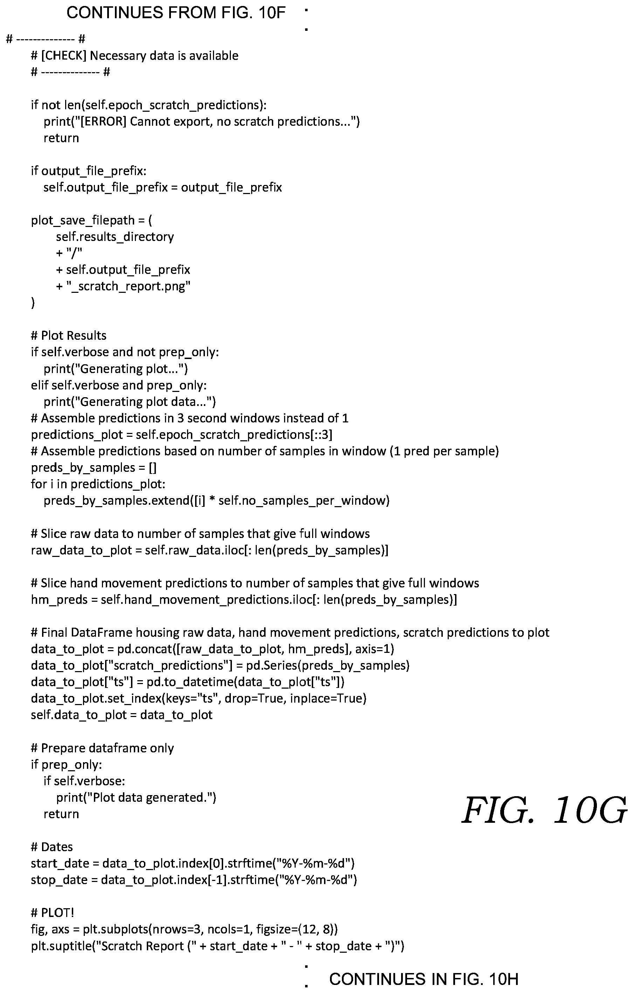

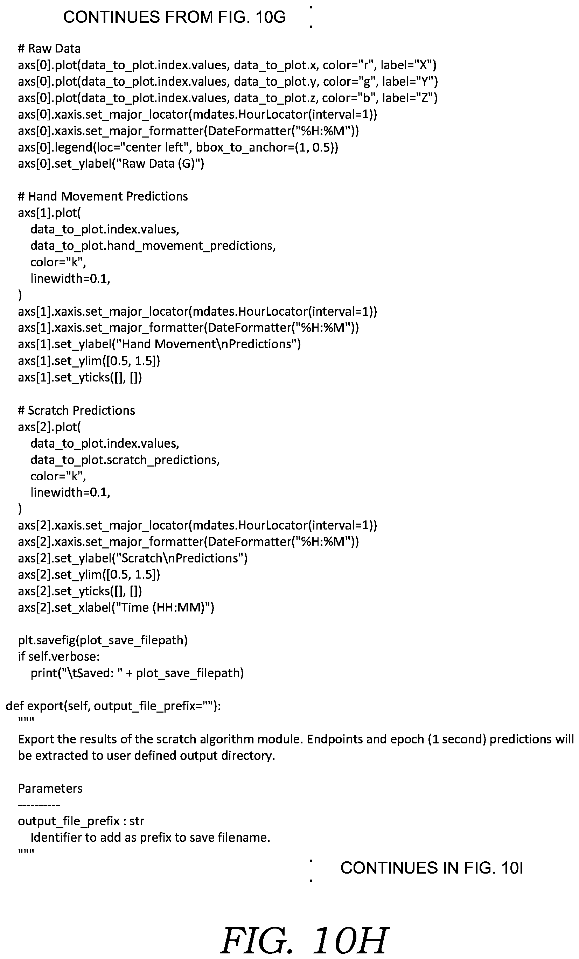

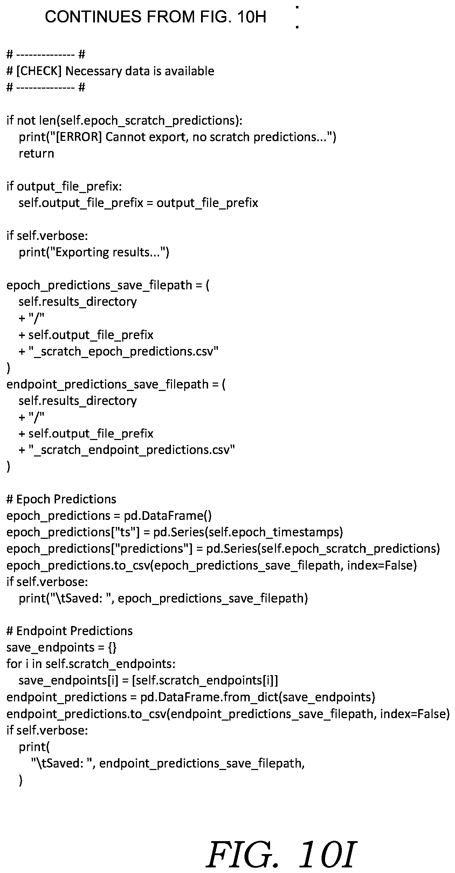

Scratch event classifier 268 outputs an indication of whether a scratch event has occurred utilizing the scratch-event detection logic 256. In some embodiments, the output of scratch event classifier 268 is binary, i.e., either scratch event or not a scratch event. Additionally, or alternatively, the output may have a corresponding quantitative or qualitative measure, such as a degree, a magnitude, or a level, associated with the detected scratch event. Output of scratch event classifier 268 may also be a "scratch event number", where if the number is above a scratch-event threshold, then it is considered as a scratch event, but if not, then it is not considered as a scratch event. In some embodiments, output of scratch event classifier 268 is stored in individual record 240 of the monitored individual. Specifically, this information may be stored as historical scratch events 244 (in individual record 240), as shown in FIG. 2. An example embodiment of scratch event classifier 268 in the form of a computer program routine is depicted in FIGS. 10A-I.

Based on detected scratch events, a number of scratch endpoints may be determined for each period of time (e.g., 24-hour period) for use by other components of system 200, such as by flare predictor 290 and/or decision support tool(s) 270, as described further herein. As used herein, the term "scratch endpoint" refers to a quantifiable measure of scratching behavior, which may be derived from raw sensor data. In one exemplary embodiment, total scratch event count may be determined by summing the number of detected scratch events within the sleep opportunity determined for the period of time. Additionally, in some embodiments, a total scratch duration may be determined by summing the lengths of time of the detected scratch events, which may be provided in minutes. Further, a duration between different scratch events may be determined by summing the time between scratch events within the sleep opportunity. A ratio of the duration between scratch events and number of scratch events may also be computed. Transformations, such as a log transformation, may be applied to one or more of the scratch endpoints. For example, a total scratch count and a total scratch duration may be each be log transformed. In one example, the log transformation is that is applied is $\log(x + 1)$ so to include possible zero values. In some aspects, scratch end points for each period are stored and provided to other components in the form of, for example, comma separated values (CSV) spreadsheets.

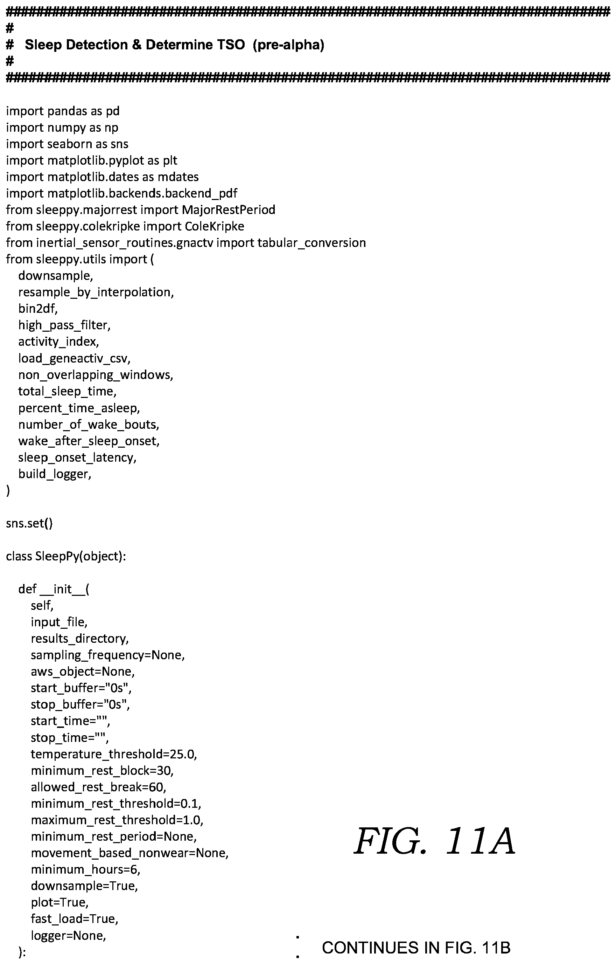

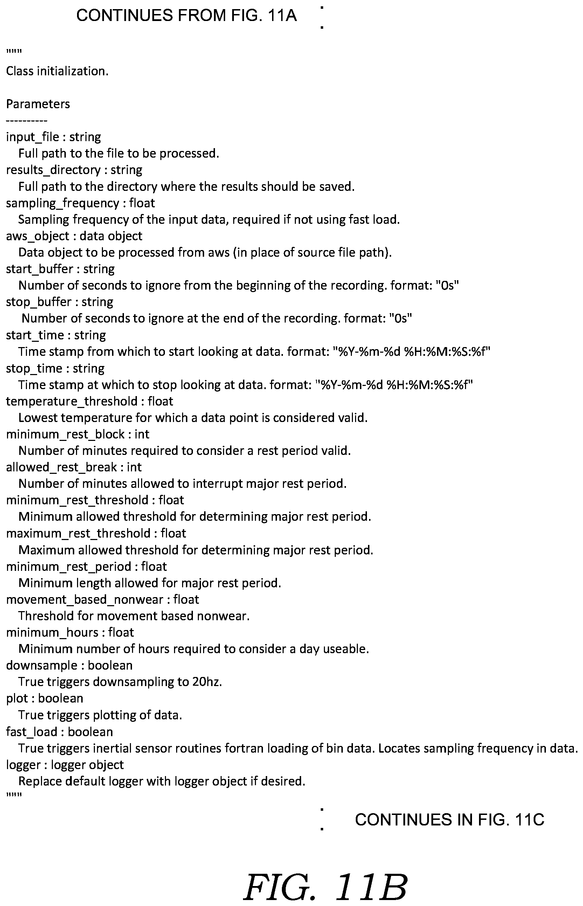

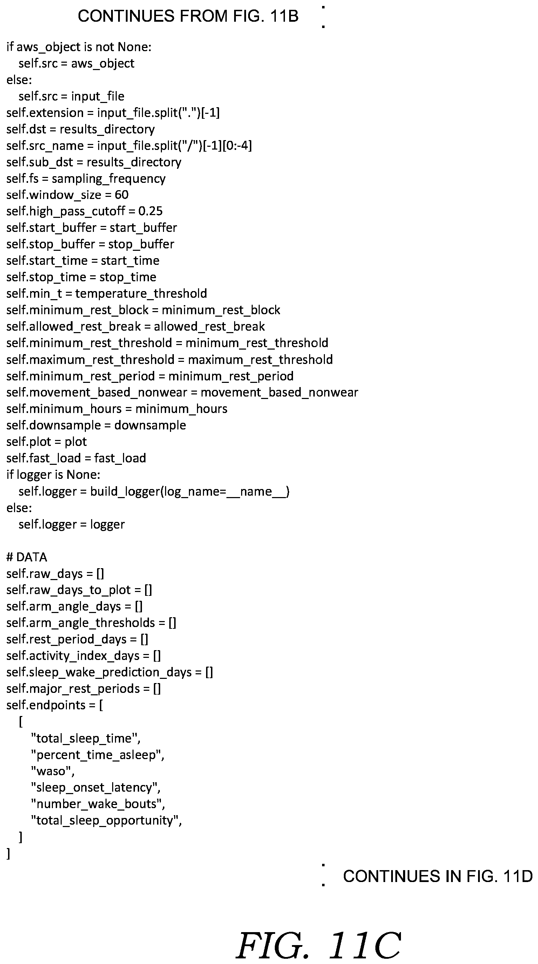

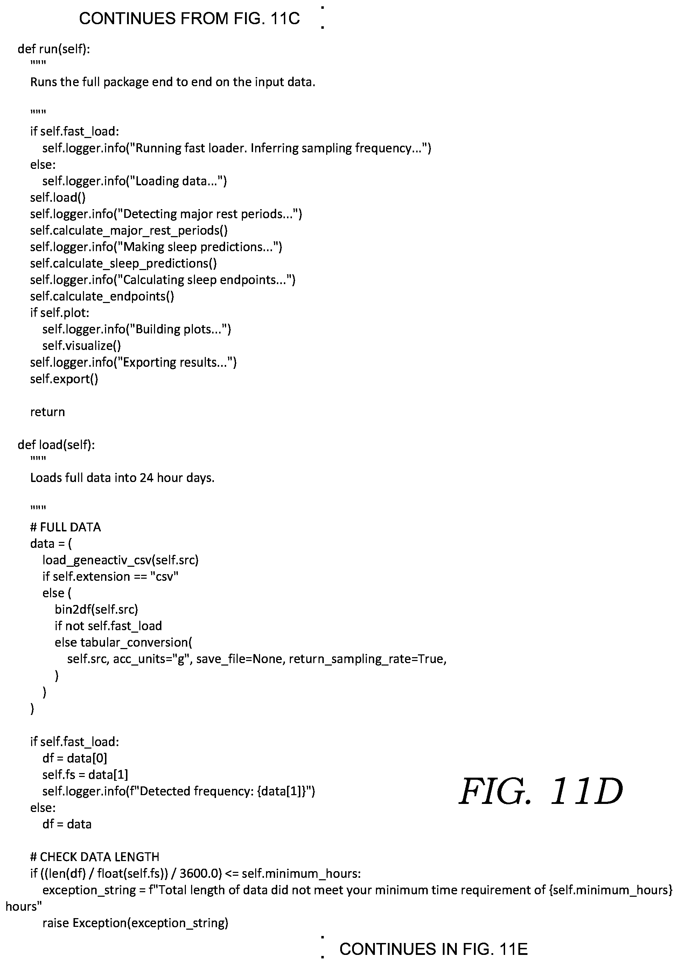

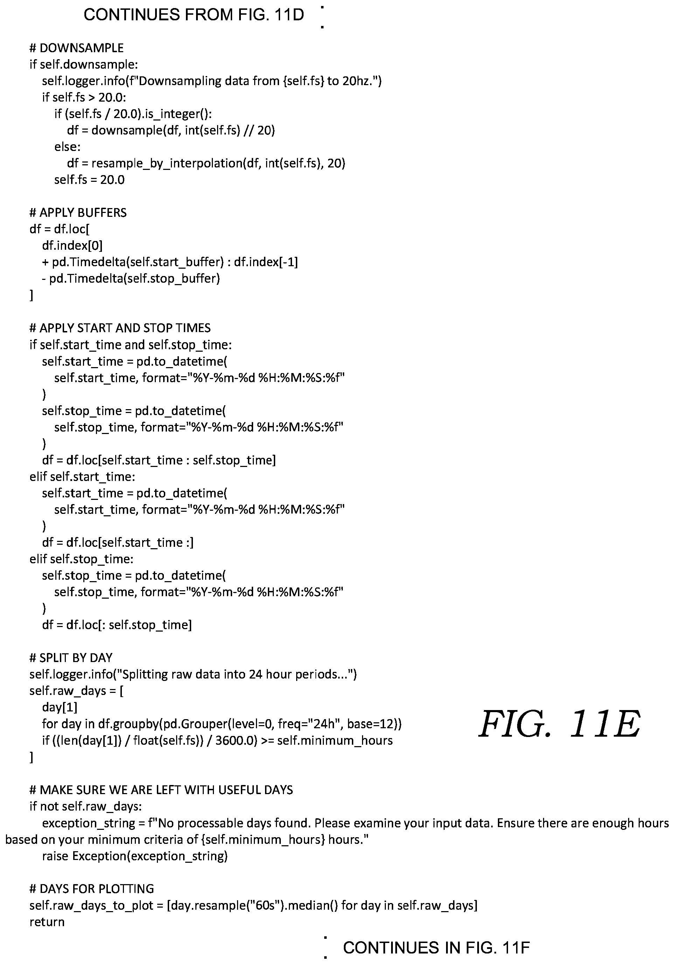

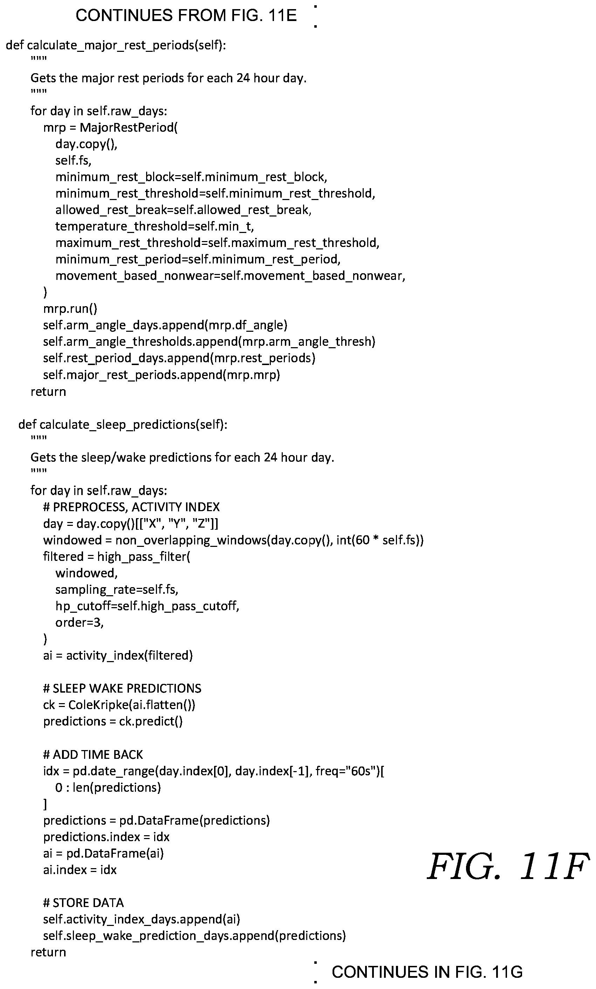

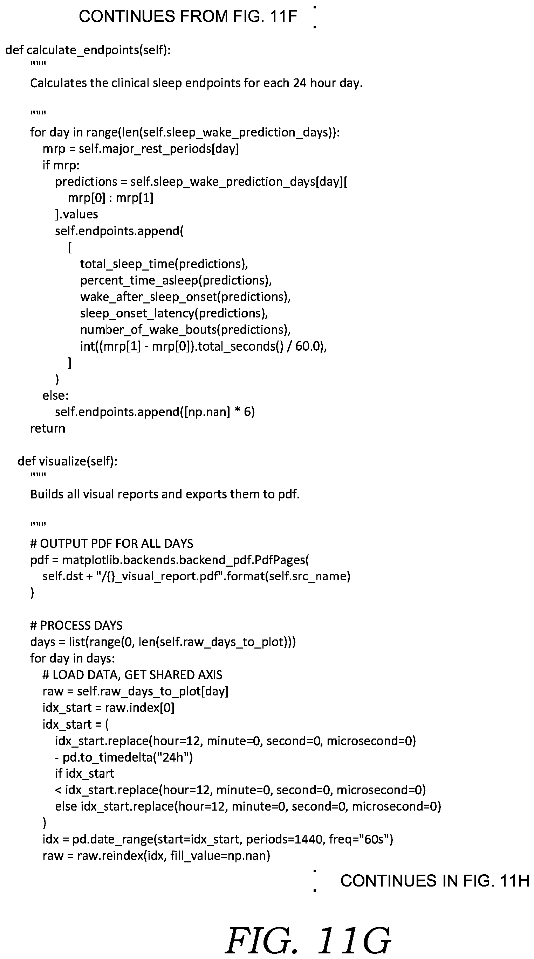

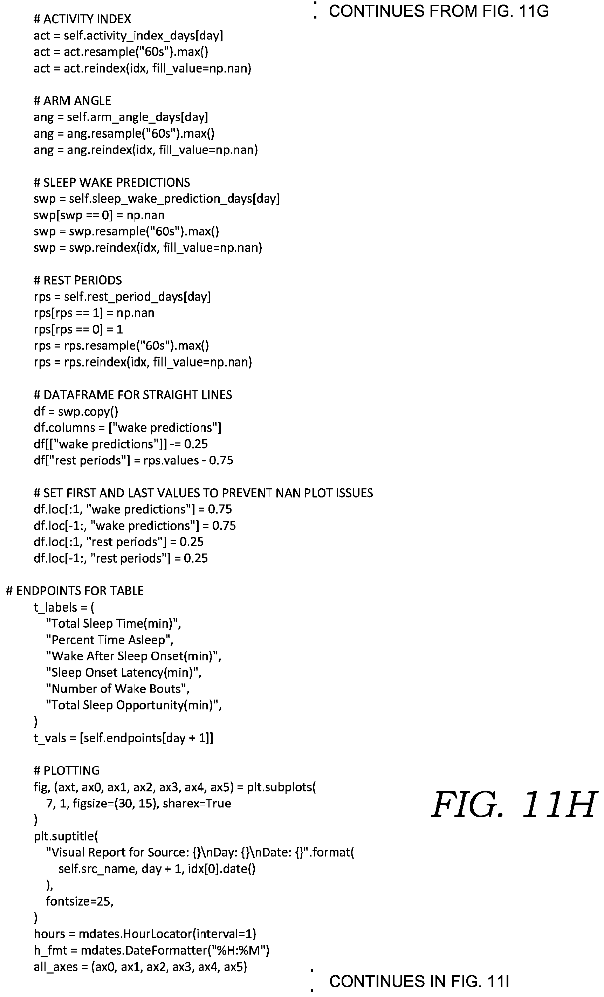

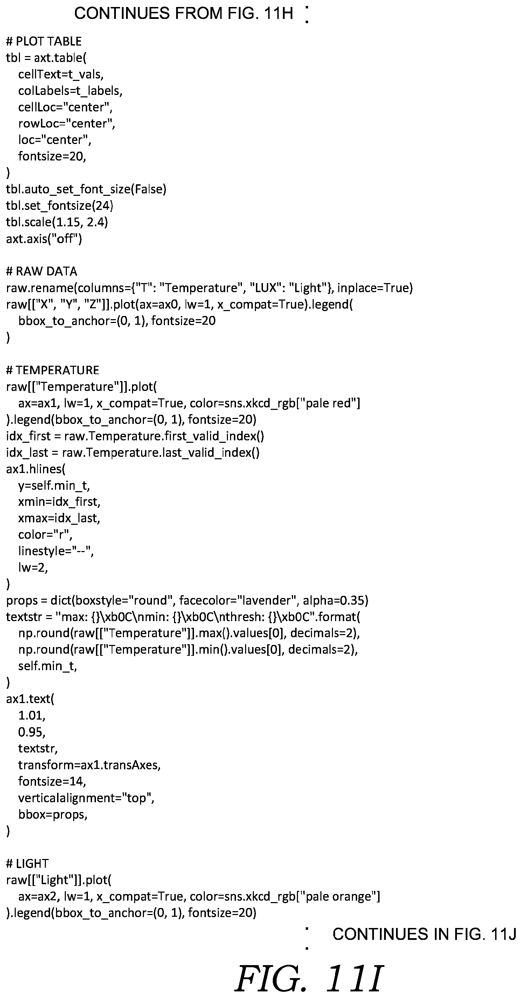

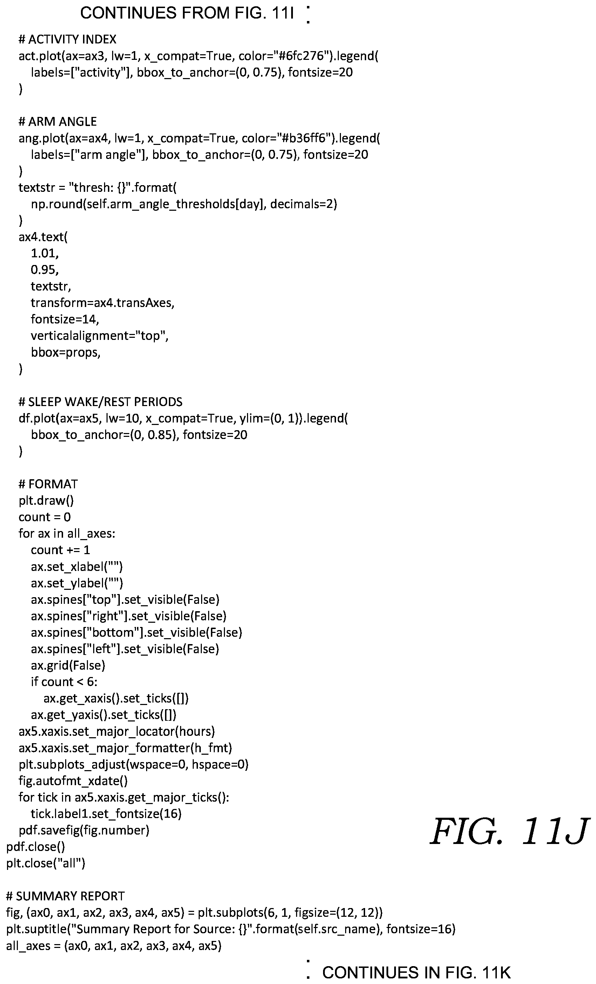

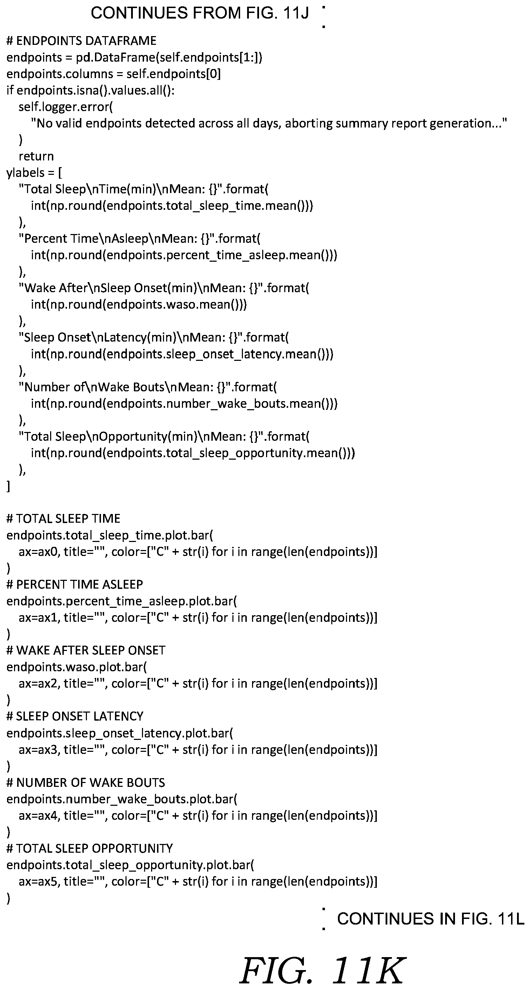

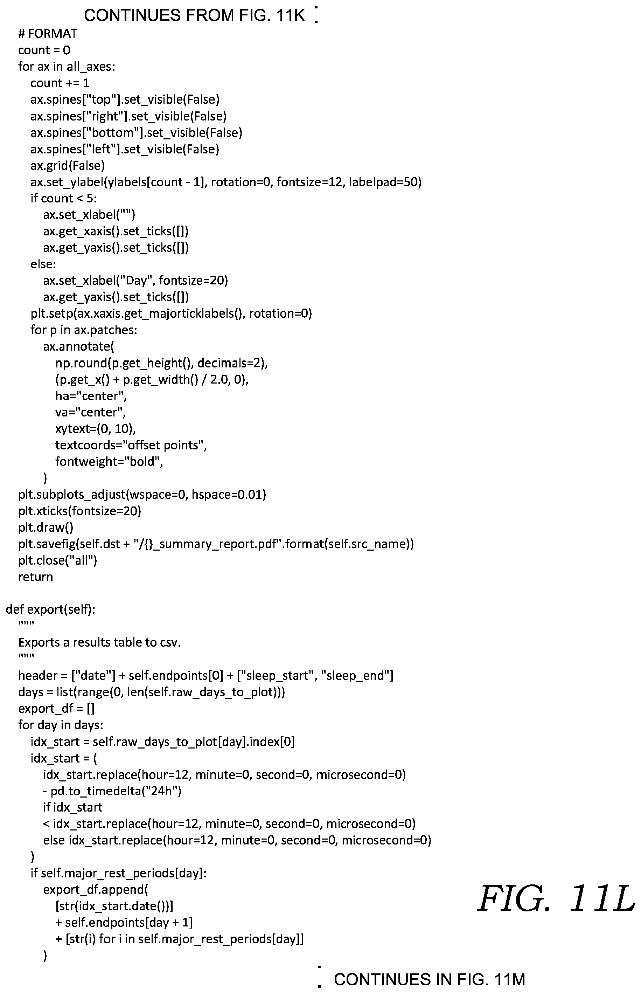

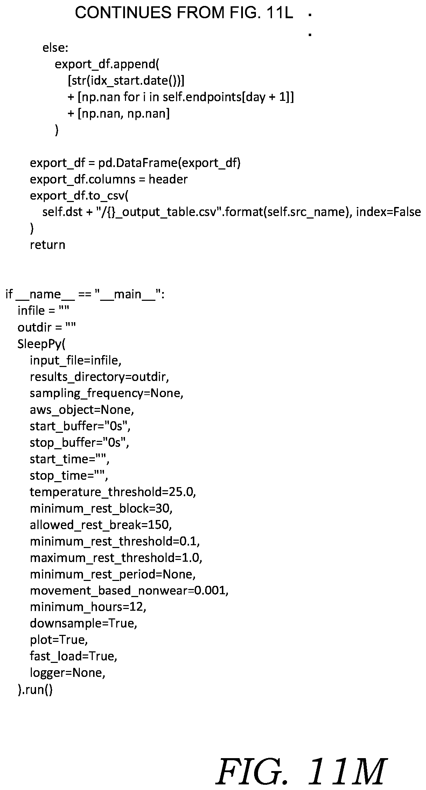

Continuing with FIG. 2, some embodiments of the technologies described herein include functionality for determining when the user is asleep or awake. As such, system 200 of FIG. 2 may comprise sleep/wake detector 230, which may generally be responsible for detecting when the user is asleep or awake. In some embodiments, sleep/wake detector 230 may utilize sleep classification logic 253 (as shown in storage 250 in FIG. 2) to determine intervals in which a user is asleep versus awake. Sleep classification logic 253 may include rules, conditions, associations, machine learning models, or other criteria for inferring or detecting a likelihood of the user being asleep based on received data, such as motion sensor data. For example, sleep classification logic 253 may determine whether the user is likely asleep based on the accelerometer data. Sleep classification logic 253 may take different forms depending on the mechanism(s) used to detect sleep. In some embodiments, sleep classification logic 253 may comprise fuzzy logic, neural network(s), finite state machine, support vector machine, logistic regression, clustering, other machine-learning techniques, similar statistical classification processes, or combinations of these to identify likely sleep periods. An example embodiment of a computer program routine for performing aspects of sleep/wake classifier 234 utilizing sleep classification logic 253 is described in conjunction with FIGS. 11A-11M.

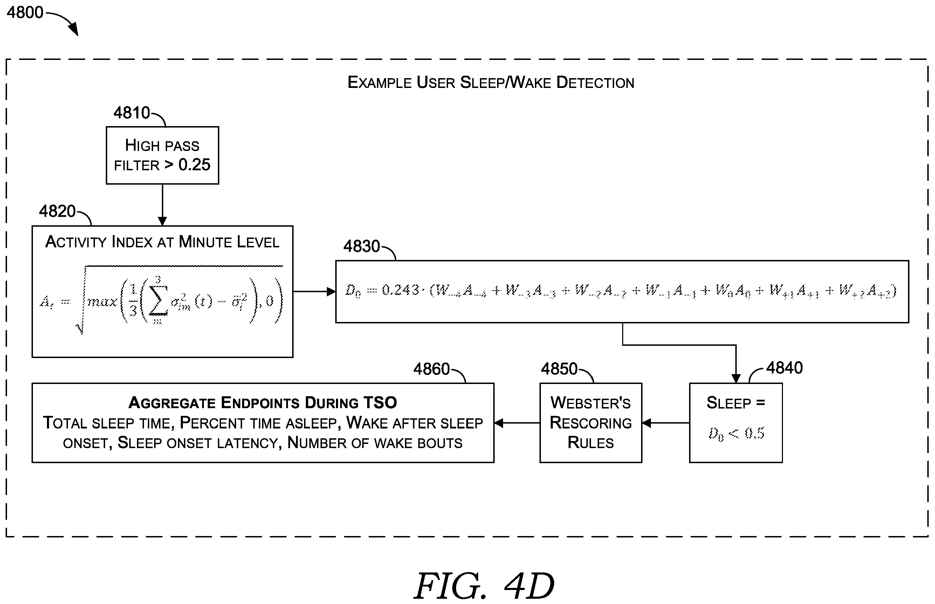

In some embodiments, sleep classification logic 253 may determine periods of sleep or wake based on motion sensor data. In one exemplary embodiment, activity values may be determined from motion sensor data within a sleep opportunity segmented into windows of time, and the activity values for those windows of time may be utilized to classify periods within the sleep opportunity as asleep or awake. As depicted in FIG. 2, embodiments of sleep/wake detector 230 may comprise activity index determiner 232 and sleep/wake classifier 234. An example embodiment of processes performed by sleep/wake detector 230 is depicted in conjunction with FIG. 4D.

Activity index determiner 232 may generally be responsible for determining activity index levels, which be a metric for summarizing tri-axial motion data. In an exemplary embodiment, motion sensor data captured during a user's sleep opportunity may be utilized to determine activity index levels. Sleep opportunity determiner 262 may determine the sleep opportunity, which may include determining sensor wear as described earlier with respect to sensor wear determiner 261. Additionally, any preprocessing steps discussed with respect to sensor wear determiner 261 and/or sleep opportunity determiner 262 may be applied to motion sensor data for determining activity index levels (by activity index determiner 232). For instance, a high-pass filter may be applied to the motion sensor data, which may be accelerometer data, and the cutoff may be 0.25 Hz.

Sleep opportunity may be segmented into windows of a predetermined length, and activity index determiner 232 may compute an activity index level for each window. In exemplary aspects, the predetermined length may be one minute, such that an activity index level is determined for each minute within the sleep opportunity. In an example embodiment, activity index determiner 232 determines activity index level, in accordance with the follow algorithm in which At is the activity level at time $t$ for patient $i$ and $m$ is axis $m$:

$$A_t = \sqrt{max\left(\frac{1}{3}\left(\sum_m^3 \sigma_{im}^2(t) - \bar{\sigma}_i^2\right), 0\right)}$$

Embodiments of sleep/wake classifier 234 may apply heuristic rules to the activity index levels (or values) to classify the windows as asleep or awake. Some embodiments of sleep/wake classifier 234 may compute a statistical feature of activity index values and apply a sleep threshold. An embodiment may determine a weighted sum of activity index values within a particular time period. For instance, the weighted sum for a one-minute window may be computed using activity index values over a span of 7 minutes, such as from time instances t-4 to t+4. An example algorithm for determining the weighted sum of activity index values is provided below:

$$D_0 = 0.243 \times (W_{-4}A_{-4} + W_{-3}A_{-3} + W_{-2}A_{-2} + W_{-1}A_{-1} + W_0A_0 + W_{+1}A_{+1} + W_{+2}A_{+2})$$

In some embodiments, sleep/wake classifier 234 may determine whether the weighted sum satisfies a sleep threshold. For example, the sleep threshold may be 0.5 and a window may be classified as a sleep period if the weighted sum for that period is less than 0.5.

Further embodiments of sleep/wake classifier 234 may apply one or more rescoring rules for improved specificity. For example, in one embodiment, Webster's rescoring rules may be similar to that described in Roger J. Cole, Daniel F. Kripke, William Gruen, Daniel J. Mullaney, J. Christian Gillin, Automatic Sleep|Wake Identification From Wrist Activity, Sleep, Volume 15, Issue 5, September 1992, Pages 461-469 (source: https://doi.org/10.1093/sleep/15.5.461).

Sleep/wake detector 230 may utilize other algorithms for detecting whether the user is sleeping, such as algorithms processing physiological variables. For instance, sleep/wake detector 230 may determine when a user is awake or asleep based on heart rate, blood pressure, core body temperature, near body temperature, and/or galvanic skin response data.

Based on detected sleep intervals, a number of sleep endpoints may be determined for each period of time (e.g., 24-hour period) for use by other components of system 200, such as by flare predictor 290 and/or decision support tool(s) 270, as described further herein. As used herein, the term "sleep endpoint" refers to a quantifiable measure of sleep behavior, which may be derived from raw sensor data. For example, total sleep time (TST) and, in some embodiments, percentage time asleep within the sleep opportunity may be computed. The number of arousals, which may also be referred to as wake bouts or periods of awake between periods of sleep, may be determined. Additionally, wake after sleep onset (WASO) and sleep onset latency (SOL) may be determined. As used herein, WASO refers to amount of time (e.g., in minutes) that a user is awake after initially falling asleep, while SOL refers to an amount of time (e.g., in minutes) at the beginning of the sleep opportunity before the first period of sleep. In some aspects, sleep end points for each period are stored and provided to other components of system 200 in the form of CSV spreadsheets. In some aspects, a user's sleep opportunity or, more specifically, TSO, as previously determined may also be saved as a sleep end point.

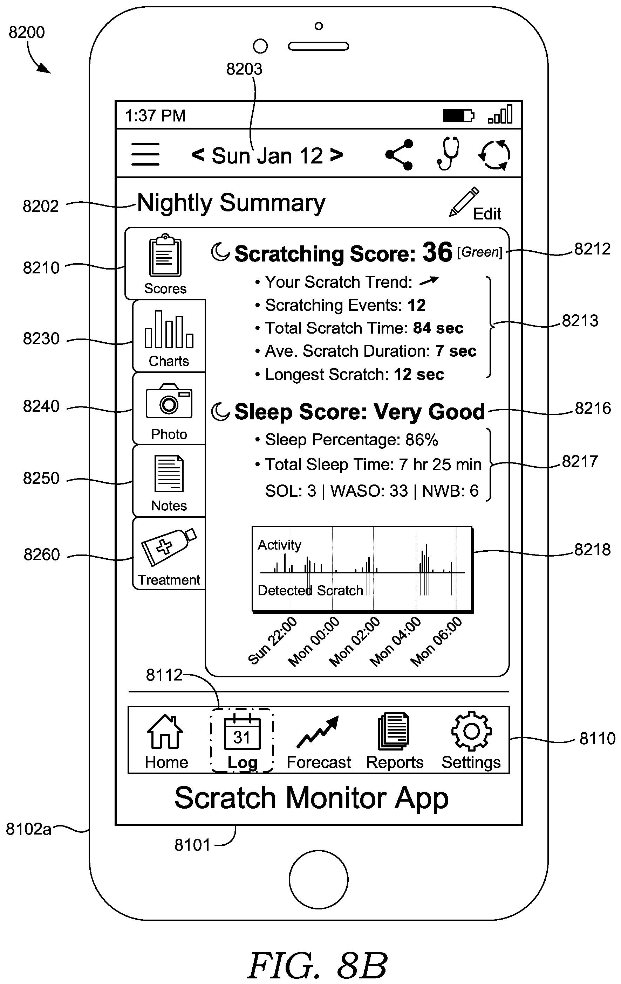

These end points may be utilized to generate a sleep score, in accordance with some embodiments. The sleep score may indicate one or more characteristics or qualities of a user's sleep for a particular evening or over a period of time. In some embodiments, scratch end points, as described with respect to scratch detector 260, may further be utilized with sleep end points to generate a sleep score. In this way, the impact of scratching during an individual's sleep may be measured. An example embodiment of output of sleep/wake detector 230, including a sleep score, is discussed below with respect to FIG. 8B.

Continuing with system 200 of FIG. 2, future flare predictor 290 may generally be responsible for determining a user's risk of having a flare over a future time interval. Embodiments of flare predictor 290 may utilize scratch patterns for a user to predict a future itch level and determine whether the future itch level rises to the severity of a flare. Example embodiments of flare predictor 290 comprise a scratch patterns assembler 292, a contextual data determiner 294, an itch predictor 296, and a flare notification generator 298.

Scratch patterns assembler 292 may assemble historic scratch information for a user, in accordance with some embodiments. The historic scratch information may include historical scratch events determined by scratch detector 260 and stored in individual record 240 of the monitored user, as shown by historical scratch events 244. In some embodiments, the historic scratch information includes scratch endpoints determined from detected scratch events such as count of total scratch episodes (or events), total scratch duration, duration between scratch events, and/or a ratio of duration between scratch events and number of scratch events. Further, some embodiments of scratch patterns assembler 292 may also consider historic sleep-related data, including sleep endpoints discussed above with respect to sleep/wake detector 230.

Contextual data determiner 294 may be generally responsible for determining context information for historic scratch events and assembled scratch patterns as well as contextual information for a future time interval, in accordance with some embodiments. This contextual data may provide insight into potential causes, signs, or symptoms of future itch or flare. For instance, some embodiments of contextual data determiner 294 may determine weather information, such as atmospheric temperature and/or humidity, which may have an impact on a user's itch level. In some embodiments, weather information is determined by a location, which may be entered by a user or may be determined based on location information, such as GPS data, obtained from a user device associated with the user. Weather information may also come from one or more smart devices associated with the user, such as a smart thermostat. Other contextual data may include user's health data, which may be determined from profile/health record (e.g., EHR) 241 in the individual record 240. This health data may include, but is not limited to, user's age, weight, diagnosed conditions, past prescriptions, and/or current prescriptions.

In addition, contextual data determiner 294 may determine context from user-input data. For example, a user may input a user-defined itch rating, notes, and/or photographs of the user's skin, including skin lesions. In some aspects, contextual information may include user input regarding past treatment details including date, etc. For instance, a user may input whether the user applied prescribed ointment on a particular day. This information may have been input by the user into a tracking or monitoring application. Additional sources of contextual information may come from workout tracking applications, food logs, and/or water consumption logs.

In some embodiments, contextual data determiner 294 may append or associate the contextual information with pattern information determined from scratch patterns assembler 292. In one exemplary embodiment, the association may be based on common date and/or time. For example, an increase in scratch events over a particular week, detected by scratch patterns assembler 292, may be correlated to a high humidity level detected by contextual data determiner 294 for that same week. In this way, pattern data from scratch patterns assembler 292 may be enriched through contextual information.

Contextual data determiner 294 may also determine current and/or future context data. For instance, contextual data determiner 294 may determine a weather forecast, such as predicted temperature and/or humidity, for the future time interval. Additionally, current health information, such as whether a user has a current prescription for atopic dermatitis and the user's current weight, may be determined.

Itch predictor 296 may generally be responsible for predicting the user's itch level within a future time interval. As used herein, a predicted itch level may be represented as a scratch level, indicating an amount of scratching a user may do at a future time interval, which may be due to itch. Itch predictor 296 may use the scratch patterns of the user, as described with reference to scratch patterns assembler 292 and contextual data determiner 294, to predict the user's itch level at a future time interval. A future time interval may be the next one day, next few days, next week, same or next month, and the like.

Itch predictor 296 may apply itch prediction logic 259 to determine a future (or predicted) itch level. Itch prediction logic 259 include rules, conditions, thresholds, associations, machine learning models, or other criteria for inferring or detecting a likelihood of a particular itch occurring in the future. Itch prediction logic 259 may take different forms depending on the mechanism(s) used to predict itch. In some embodiments, itch prediction logic 259 may comprise fuzzy logic, neural network(s), finite state machine, support vector machine, logistic regression, clustering, other machine-learning techniques, similar statistical classification processes, or combinations of these to determine a likelihood of itch at a future time interval. Itch prediction logic 259 may be applied to scratch patterns, historical context, current context (including user-specific data such as age, demographics, prior conditions, etc.) and, in some embodiments, sleep-related data, to determine the likelihood of itch.

In some embodiments, itch prediction logic 259 may be generalized logic based on reference data. In one exemplary embodiment, historical scratch patterns for a reference population may be assembled, contextual information for the reference population may be determined, and this reference information may be utilized to determine itch prediction logic 259, such as one or more heuristic rules or thresholds. In some embodiments, this logic may be based on crowdsourced data, or historic data of similar users (e.g. users with the same diagnosed condition, in the same or near the same geographic location, or same or similar demographics). Any such crowdsourced data may be pre-identified prior to use by embodiments of flare predictor 290.

Further, in some aspects, itch prediction logic 259 is based on the specific user's historical scratch patterns and, in some embodiments, sleep-related data, as well as historical contextual information. For example, one or more rules or thresholds or machine learning models may be built utilizing the monitored user's information. In this way, prior conditions, such as the level and rate increase of scratch events, weather, whether the user was taking treatment, or the like, may be considered in determining logic to apply to determine a particular itch level and/or flare event. Further, this information may also be used to then predict likely future itch levels or flare events when similar patterns are observed again.

Although itch predictor 296 has been described as predicting a level or a degree of itch, a severe and/or persistent itch may accompany a flare. In this way, a predicted itch level may, by itself, be a predicted flare risk in accordance with some embodiments. Further, in some aspects, a predicted itch level may be utilized to determine a likelihood of a future flare. In some embodiments, predicting a flare risk utilizes itch predictions for multiple future time periods.

A predicted itch level may be compared to one or more flare detection thresholds to determine whether the predicted itch level is of sufficient severity to be a flare risk. A flare detection threshold may be predetermined based on a reference population, such that this threshold may be utilized for the larger population. In other embodiments, a flare detection threshold may be determined for a particular monitored individual. For instance, the flare detection threshold may be set based on the user's historical information, including health data such as condition or age. The flare detection threshold may be set by a doctor/caregiver of the user and/or adjusted by the user, which may be stored in settings 249 in individual record 240. In this way, the determination of a flare prediction by applying the flare detection threshold may be customized for a specific user.

In some aspects, output of itch predictor 296 may be an itch level or a risk score for a future time interval. The itch level or risk score may be a numerical level or score, or a categorical level or score, such as indicating low, medium, high, and/or severe risk levels. Additionally, in some embodiments, two predictions may be made for each future time interval including one prediction based on an assumption of treatment of pruritus or an underlying condition causing pruritus, and another prediction based on an assumption of no treatment for pruritus or an underlying condition. A prediction based on an assumption of treatment may be based on a determination of a current use of a correct treatment determined by contextual data determiner 294. Additionally, or alternatively, this predication may be based on the determination of information indicating a potential treatment, which may be identified from reference information in storage 250. A prediction based on an assumption of no treatment may be based on contextual data determiner 294 determining that the user is not taking treatment or failing to determine current treatment information. Additionally, even where contextual data determiner 294 determines that a user is currently taking treatment, a prediction based on no treatment may be based on a presumption that the user may stop taking the treatment.

In some embodiments, flare notification generator 298 of flare predictor 290 may generally be responsible for generating a notification or an alert, indicating user's itch and/or flare risk. For example, where an itch level satisfies a flare detection threshold, flare notification generator 298 may issue a notification presenting that risk to a user device (such as any of 102a-n) of the monitored user and/or to a clinician user device 108 for a clinician treating the monitored user or recommended to treat the monitored user. Unless otherwise indicated, the term "flare notification" is used herein to include a notification about an itch level even if the itch level does not indicate that a flare event is likely.

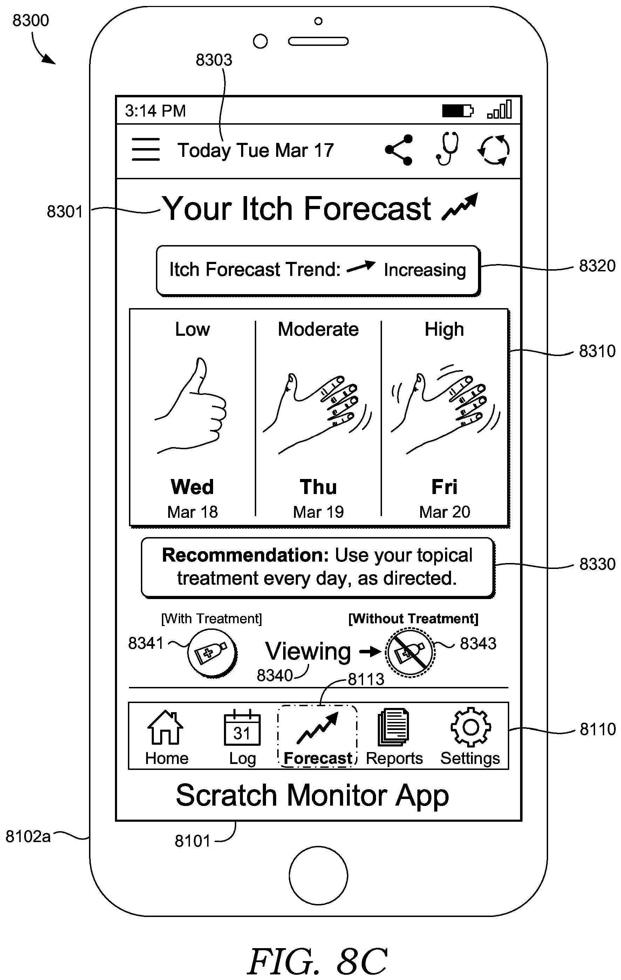

Example embodiments of a flare notification generated in accordance with embodiments of flare notification generator 298 are described below with respect to FIGS. 8C-8D and above with respect to FIG. 3B. In some aspects, an alert or fire icon may be presented on a monitored user device (e.g., any of 102a-n), such as a smart watch. The flare notification may be enriched with supporting details, enabling the user to know why a flare is predicted. For instance, a flare notification may indicate that the user's scratch event trend is increasing, weather is expected to change, or other contextual information or historical patterns (as described above) that may affect scratching. Further, in some aspects, flare notification may include recommendations to initiate actions along with the notification based on the itch level or flare risk. As an example, recommendations to schedule an appointment with a caregiver, a refill prescription, and/or add over-the-counter (OTC) therapy to user's shopping list may be included within, or along with, the notification.

Some embodiments of flare notification generator 298 may determine a time instance or a time interval, which can be used to decide when to provide the flare notification. This determination may be based on user preferences, such as those stored in settings 249. Alternatively, or additionally, this determination may be based on location information and/or time of day in a way to increase the likelihood of the user taking necessary action to mitigate the flare risk. For instance, in one embodiment, flare notification is issued either in the morning or at night, which may be correspond to times when an individual is more likely to apply an at-home treatment and/or plan a trip to a store for treatment. For one such instance, flare notification generator 298 may determine whether a location of the user is at or near a store, such as a drug store, and may issue a notification with a recommendation for an over-the-counter treatment or to refill a prescription.

Further, some embodiments of flare notification generator 298 may securely transmit a flare risk and associated data, such as recent scratch data, to the user's caregiver. This flare notification may be sent directly to a user device associated with the user's caregiver, such as clinician user device 108. In addition, or alternatively, a flare notification may be logged at regular intervals in a data source accessible by the user's caregiver, such as the user's EHR 241.

Decision support tool(s) 270 (as shown in FIG. 2) represents various computing applications, services or functionality for consuming output of one or more other components of system 200, such as detected scratch events or scratch endpoints, sleep score and/or sleep endpoints, or itch and/or flare prediction. Decision support tool(s) 270 may utilize this information to enable therapeutic and/or preventative actions, in accordance with some embodiments. In this way, decision support tool(s) 270 may be utilized by a monitored user and/or a caregiver of the monitored user. This decision support tool(s) 270 may take the form of a standalone application on a client device, a web application, and/or a service on an existing application. In some embodiments, one or more decision support tools (such as 270) may be distributed across multiple devices of system 200.

Some embodiments of the decision support tool(s) 270 may determine a daily/nightly scratch score and/or a sleep score for the monitored user and/or, in some aspects, other related metrics. An example user interface of decision support tool(s) 270 providing nightly scratch score, sleep score, and related information is shown in FIG. 8B. A scratch score may be based on scratch endpoints as previously discussed, including the number of detected scratch events, average duration, longest scratch event, for example. In some embodiments, the sleep score may be determined based on sleep-related data sensed or determined in connection to the monitored user, such as sleep metrics previously discussed, including TSO, TST, WASO, SOL, etc. In some implementations, the sleep score may also be based on the scratch score or scratch-related data, such as scratch events, for the user. For instance, a higher number of scratch events or a higher scratch score may decrease the sleep score. In this way, the sleep score for these embodiments is more meaningful than the sleep-like scores provided by conventional technologies because the sleep score determined, in accordance with embodiments of this disclosure, reflects the user's scratching while sleeping.

One example decision support tool 272 may comprise a scratch tracker application or service . In some embodiments, decision support tool 272 may associate scratch event data with periods of time, such as days, and present the scratch event data in association with the relevant period. Decision support tool 272 may include a calendar in which each day of the calendar provides scratch event data for the monitored user. This data may include historical scratch event data, which may include determined scratch endpoints, such as total scratch event count and total scratch duration, as described with respect to scratch detector 260. Decision support tool 272 may further allow a user to log additional information for each date, such as user-defined scratching or itch levels, notes/narratives, other symptoms, and/or photographs. An example scratch tracker application or service is further described in connection with FIG. 8A.

Another decision support tool 274 may comprise a flare risk predictor service and/or itch forecaster, which may be determined as described earlier with respect to flare predictor 290. Decision support tool 274 may provide the flare risk predictor or itch level prediction as a notification. Additionally, or alternatively, the flare risk or itch level prediction may be associated with future time intervals (e.g., future dates and times) and presented in association with those dates, such as, on a calendar. An example flare risk predictor service and/or itch forecaster is further described in connection with FIGS. 8C-8E.

Another exemplary decision support tool 276 shown in FIG. 2 may initiate and/or schedule a treatment recommendation, in accordance with an embodiment. A treatment recommendation may comprise a therapeutic agent (including a prescription or an over-the-counter medicine), consultation with a clinician, and/or additional testing that is recommended to alleviate itch, treat scratching, and/or reduce the risk of future itch or flare. For example, decision support tool 276 may determine a recommendation for treatment, such as continued use of an existing prescription, a new medication, or scheduling an appointment with a clinician based on current scratch event endpoints determined by scratch detector 260. Additionally, or alternatively, these recommendations may be based on a forecasted itch or a flare event as determined by flare predictor 290. An example embodiment of decision support tool 276 is described further with connection to FIG. 8C.

Some embodiments of decision support tool 276 include aspects for treating a user's pruritus, which may be presented as atopic dermatitis, based on scratching detected from a wearable device with a sensor, such as sensor 103. Treatment may be targeted to reduce the severity of a user's pruritus. Treatment determined based on the detected scratching may be intended to prevent the user's pruritus from worsening. Treating a user's pruritus based on the detected scratching may include determining a new treatment protocol, which may include a new therapeutic agent(s), a dosage of a new agent or a new dosage of an existing agent being taken by the user or a dosage of a new agent, and/or a manner of administering a new agent or a new manner of administration of an existing agent taken by the user. A recommendation for the new treatment protocol may be provided to the user or caregiver for the user. In some embodiments, a prescription may be sent to the user, the user's caregiver, or a user's pharmacy. In some instances, treatment may include refilling an existing prescription without making changes. Further embodiments may include administering the recommended therapeutic agent(s) to the user in accordance with the recommendation treatment protocol and/or tracking the application or use of the recommended therapeutic agent(s). In this way, embodiments of the disclosure may better enable controlling, monitoring, and/or managing the use or application of therapeutic agents for treating pruritus, which would not only be beneficial on a user's condition but could help healthcare providers and drug manufacturers, as well as others within the supply chain, better comply with regulations and recommendations set by the Food and Drug Administration and other governing bodies. In example aspects, treatment includes one or more therapeutic agents from the following:

- an agent for treating autoimmune and/or inflammatory disorders, such as sulfasalazine, mesalazine, azathioprine, an antibody (e.g., infliximab, adalimumab, belimumab, tanezumab, ranibizumab, bevacizumab, mepolizumab certolizumab, natalizumab, ustekinumab, and/or vedolizumab), 6-mercaptopurine, hydroxychloroquine, obeticholic acid, mofetil, sodium mycophenolate, leflunomide, rituxan, solumedrol, depomedrol, a non-steroidal anti-inflammatory drug (NSAID) (e.g., aspirin, ibuprofen, celecoxib, valdecoxib, WBI-1001 and/or MRX-6), and/or a corticosteroid (e.g., fluticasone, mometasone, budesonide, ciclesonide, beclamethasone depomedrol, betamethasone, dexamethasone, and/or prednisone);

- an agent for treating dermatological conditions, such as an immunosuppressant (e.g., cyclosporin, tacrolimus, and/or pimecrolimus), an antibody (e.g., infliximab, adalimumab, dupilumab, omalizumab, tralokinumab, etokimab, nemolizumab, Tezepelumab, lebrikizumab, fezakinumab, anti-OX40 and/or efalizumab), a TNF inhibitor (e.g., etanercept), a PDE4 inhibitor (e.g., crisaborole), and/or a topical corticosteroid (e.g., fluocinonide, mapracorat, hydrocortisone, desonide, alclometasone, triamcinolone, and/or desoximetasone);
- a histamine receptor antagonist, such as a histamine type 1 receptor antagonist and/or a histamine type 2 receptor antagonist (e.g., loratidine, fexofenadine, desloratidine, levocetirizine, methapyrilene and/or cetirizine);
- a corticosteroid (e.g., budesonide, fluticasone, mometasone, dexamethasone, prednisolone, ciclesonide, and/or beclomethasone); and/or
- an agent for treating joint disorders, such as methotrexate, azathioprine, and/or an NSAID (e.g., aspirin, ibuprofen, celecoxib, valdecoxib, WBI-1001 and/or MRX-6).

Some embodiments include treatment being one or more therapeutic agents from the following, which may be in addition to or alternative to the agents listed above:

- a JAK inhibitor, such as abrocitinib, baricitinib, brepocitinib, cerdulatinib, decernotinib, delgocitinib, fedratinib, filgotinib, gandotinib, ilginatinib, itacitinib, lestaurtinib, momelotinib, oclacitinib pacritinib, peficitinib, ritlecitinib, ruxolitinib, tofacitinib, upadacitinib, THRX-212401, PF-07055087, PF-06471658, PF-07055090, ATI-502, BMS-986165, JTE052, PF-06826647, SNA 152, and/or SHR-0302;
- an aryl hydrocarbon receptor agonist, such as tapinarof;
- an interleukin-2-inducible T cell kinase inhibitor;
- a retinoic acid derivative, such as alitretinoin;
- an antiviral agent; and/or
- a vaccine.

In a preferred embodiment, a treatment includes the PDE4 inhibitor crisaborole, and in addition or alternatively, the JAK inhibitor abrocitinib.

These example decision support tools 272, 274, and 276 may be utilized independently or in conjunction with each other. For example, one application may employ all three decision support tools. Additional details of decision support tools are discussed in conjunction with FIGS. 8A-8E.

Presentation component 220 of system 200 may generally be responsible for presenting detected scratch event information, detected sleep/wake information, itch/flare predictions, and/or related information. Presentation component 220 may comprise one or more applications or services on a user device, across multiple user devices, or in the cloud environment. For example, in one embodiment, presentation component 220 may manage the presentation of information, such as notifications and alerts, to a user across multiple user devices associated with that user. Based on presentation logic, context, and/or other user data, presentation component 220 may determine on which user device(s) content is presented, as well as the context of the presentation, such as how (e.g., in what format and how much content, which can be dependent on a user device or context) it is presented, when it is presented, or other such aspects of presentation.

In some embodiments, presentation component 220 may generate user interface features associated with or used to facilitate presenting aspects of other components of system 200, such as scratch detector 260, sleep/wake detector 230, flare predictor 290, and decision support tool(s) 270, to the user. Such features can include interface elements (such as icons or indicators, graphics buttons, sliders, menus, audio prompts, alerts, alarms, vibrations, pop-up windows, notification bar or status bar items, in-app notifications, or other similar features for interfacing with a user), queries, and prompts. Examples of graphic user interfaces (GUIs) that may be generated and provided to a user by presentation component 220 are described in connection with FIGS. 8A-E.

Storage 250 of example system 200 may generally store information including data, computer instructions (e.g., software program instructions, routines, or services), logic, profiles, and/or models used in embodiments described herein. In an embodiment, storage 250 may comprise a data store (or computer data memory), such as data store 150. Further, although depicted as a single data store component, storage 250 may be embodied as one or more data stores or in the cloud environment.

As shown in example system 200, storage 250 includes sleep classification logic 253, scratch-event detection logic 256, and itch prediction logic 259, all of which are previously described. Further, storage 250 may include one or more individual records 240, as shown in FIG. 2. Individual record 240 may include information associated with a particular monitored individual/user, such as profile/health data (EHR) 241, sensor data 242, historical scratch events 244, logs 246, user account(s)/device(s) 248, and settings 249. The information stored in individual record 240 may be available to data collection component 210, sensor monitor 280, scratch detector 260, sleep/wake detector 230, flare predictor 290, or other components of example system 200, as described herein.

Profile/health data (EHR) 241 may provide information relating to a monitored individual's health. Embodiments of profile/health data (EHR) 241 may include a portion or all of an individual's EHR or only some health data that is related to scratch or sleep. For instance, profile/health data (EHR) 241 may indicate past or currently diagnosed conditions, such as atopic dermatitis, eczema, psoriasis, or similar conditions; medications associated with treating pruritus-related conditions or with potential side effects of scratching/itching; weight; or age.

Sensor data 242 may include raw and/or processed sensor data, such as from sensor 103 (shown in FIG. 1). This sensor data may include data used for scratch event detection, such as motion sensor data and extract features. Sensor data may further include other types of information that may be stored on, or in conjunction with, a sensor device, such as atmospheric information (e.g., atmospheric temperature or humidity) or physiological data (e.g., near body temperature or heart rate). Other sensor data disclosed herein may be stored as sensor data 242.

Further, historical scratch events 244 may comprise scratch events determined by scratch event classifier 268. In some embodiments, historical scratch events 244 also include scratch endpoints, such as count of total scratch episodes, total scratch duration, duration between scratch events, and/or a ratio of duration between scratch events and scratch episodes. Embodiments of historical scratch events 244 may also include itch or flare predictions determined by flare predictor 290. Further, in some embodiments, historical scratch events 244 may also include information about the detected scratch events and/or previously predicted itch or flares, such as the date-time of a scratch event or prediction. In some aspects, other contextual data, such as weather, location, or the like, may be stored as historical scratch events 244. Additionally, or alternatively, other contextual information extracted from user-provided observational data, such as user-defined itch ratings, notes and photographs may be stored as historical scratch events 244.

In some embodiments, logs 246 may include observation logs and/or response logs. An observation log may include user notes, photographs, or other observations that the user may provide, via a scratch monitor app, in accordance with one exemplary embodiment. These observations may relate to itching, scratching, flares, sleeping and other contextual information described herein, such as weather, temperature, or the like. As previously disclosed, observation logs may be examined by contextual data determiner 294 to gain additional insights for future predictions.

Further, in some embodiments, logs 246 may also include response logs indicating how a user reacted to a detected scratch event, detected sleep/wake period, itch or flare prediction, and/or resulting notification. For instance, a response log may indicate that a monitored user scheduled a tele-appointment with a clinician in response to a predicted future flare. In another instance, a user may add a recommended ointment to an electronic shopping list in response to detected scratch events. Additionally, response log may indicate if a monitored user did not take affirmative action or selected an "ignore" feature in response to a notification or an alert generated based on detected scratch events or an itch or flare prediction. Some embodiments of this disclosure may utilize response logs for calibration, improving scratch detection, sleep/wake detection, flare or itch prediction, and/or improving decision support recommendations or actions initiated.

Also, in some embodiments, user account(s)/device(s) 248 may generally include information about user devices accessed, used, or otherwise associated with a user. Examples of such user devices may include user devices 102a-n of FIG. 1 and, as such, may include mobile phones, tablets, smart watches, or other wearable devices. Other smart devices and associated accounts, such as a home smart thermostat and/or a hygrometer may be included in user account(s)/device(s) 248.

In one embodiment, user account(s)/device(s) 248 may include information related to accounts associated with a user, for example, online or cloud-based accounts (e.g., online health record portals, network/health provider, network websites, decision support applications, social media, email, phone, e-commerce websites, or the like). For example, user account(s)/device(s) 248 may include a monitored individual's account for a decision support application, such as decision support tool(s) 270; an account for a care provider site (which may be utilized to enable electronic scheduling of appointments, for example); and online e-commerce accounts, such as Amazon.com® or a drugstore (which may be utilized to enable online ordering of treatments, for example).

Additionally, user account(s)/device(s) 248 may also include a user's calendar, appointments, application data, other user accounts, or the like. Some embodiments of user account(s)/device(s) 248 may store information across one or more databases, knowledge graphs, or data structures. As described previously, the information stored in user account(s)/device(s) 248 may be determined from data collection component 210.

Furthermore, in some embodiments, settings 249 may generally include user settings or preferences associated with one or more steps for scratch detection, sleep/wake detection, or itch/flare prediction or with one or more decision support applications, such as decision support tool(s) 270. By way of example and not limitation, such settings may include user notification tolerance thresholds, which may define when and how a user would like to be notified of a predicted flare. In some aspects, settings 249 may include user preferences for applications, such as notifications, preferred caregivers, preferred pharmacy or other stores, and over-the-counter medications. In one embodiment, calibration, initialization and settings of sensor(s) may also be stored in settings 249.

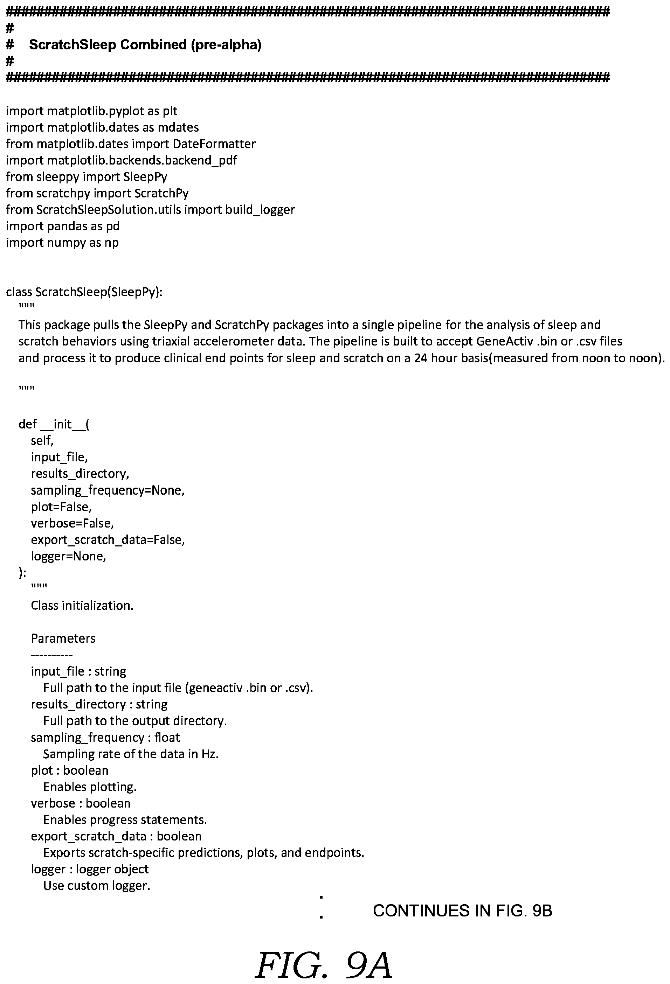

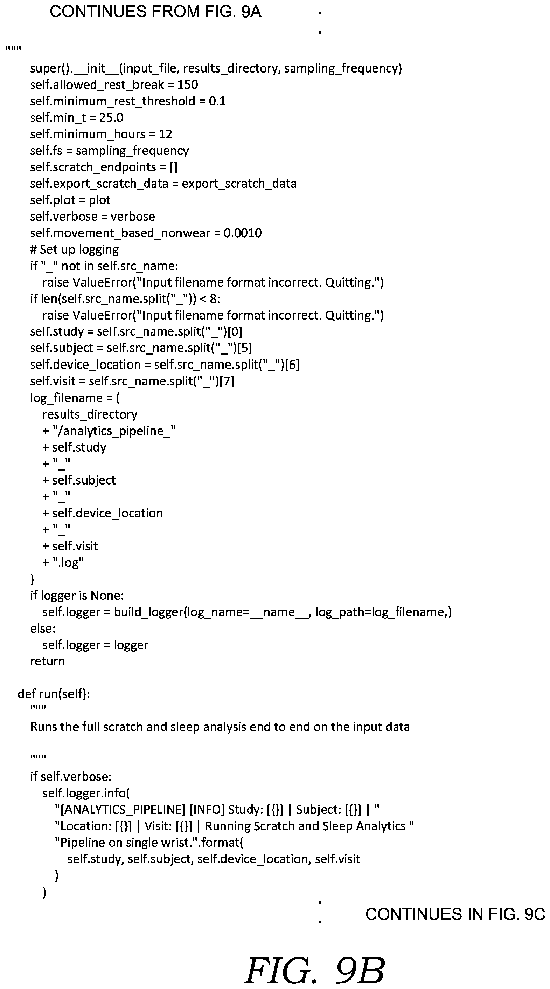

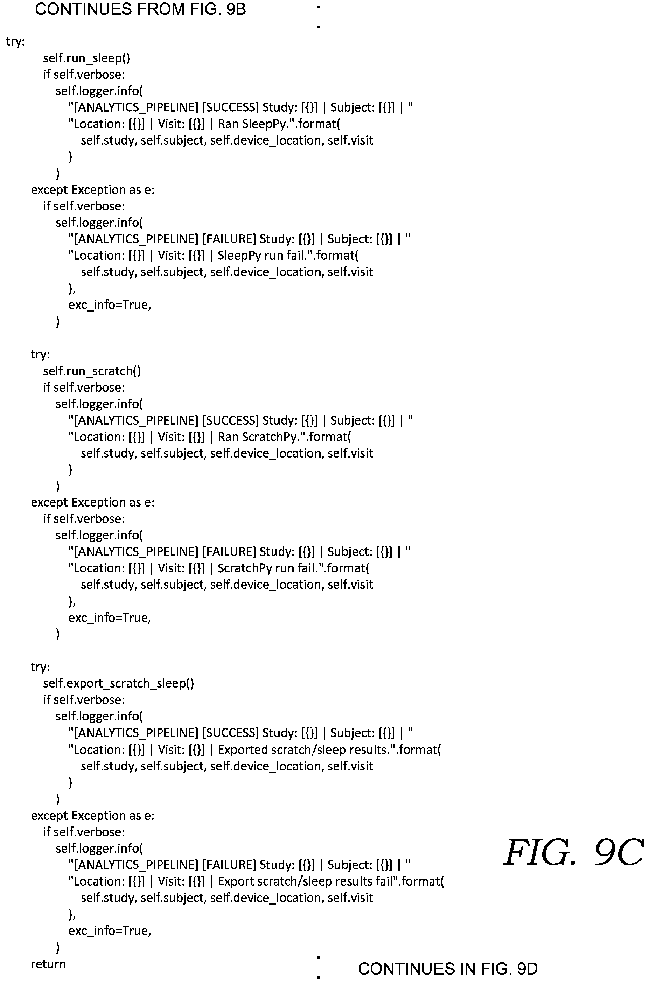

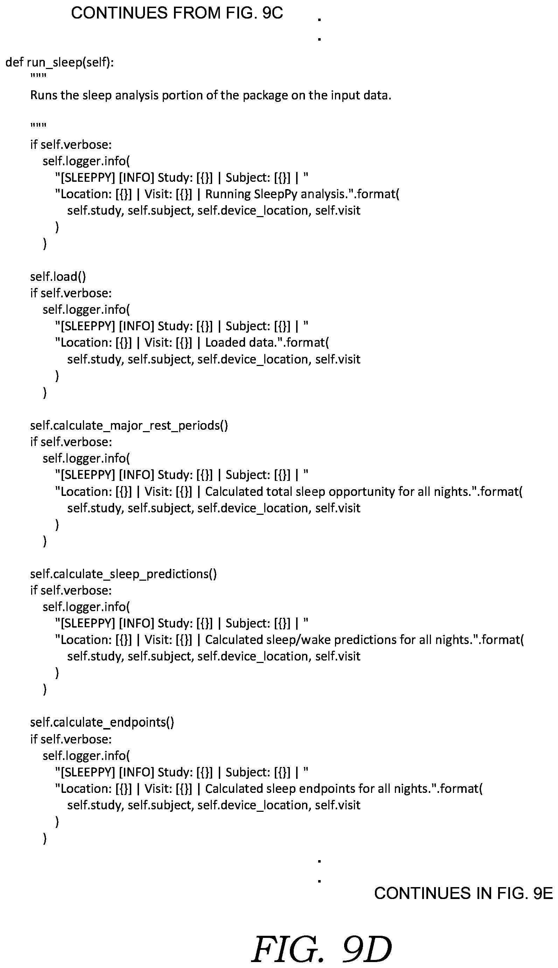

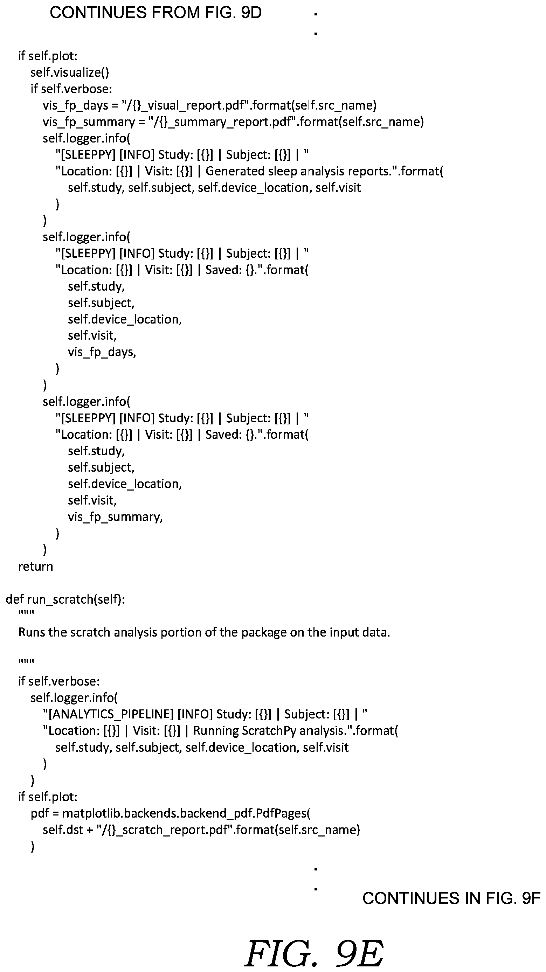

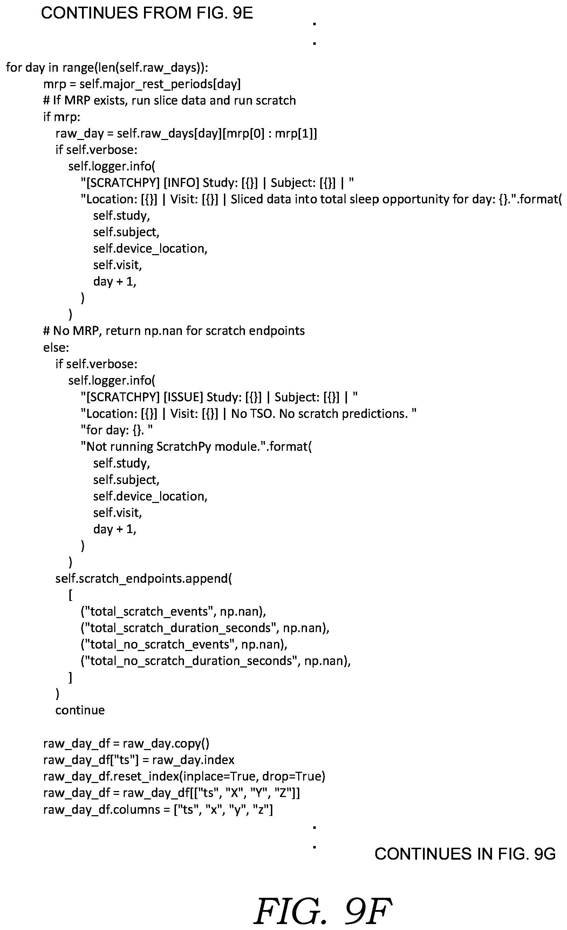

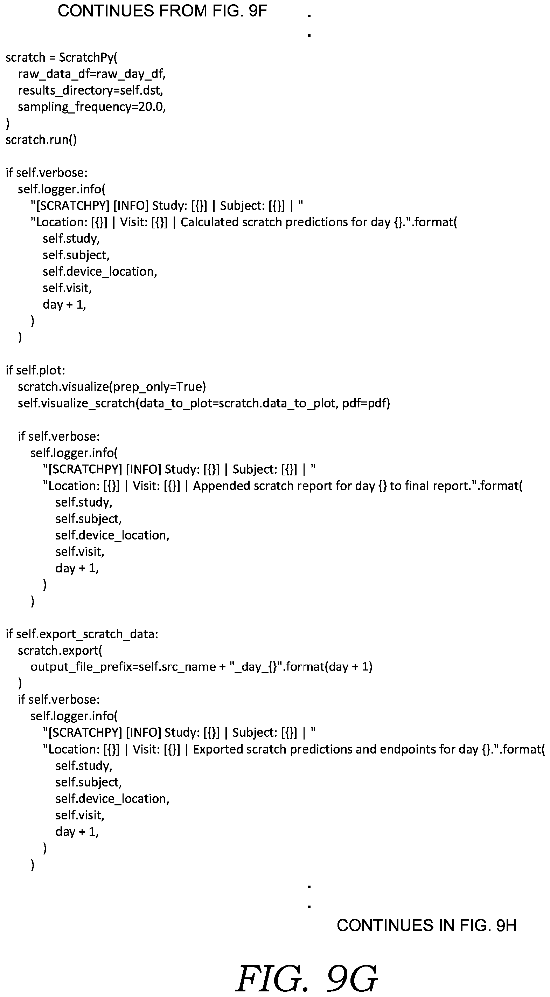

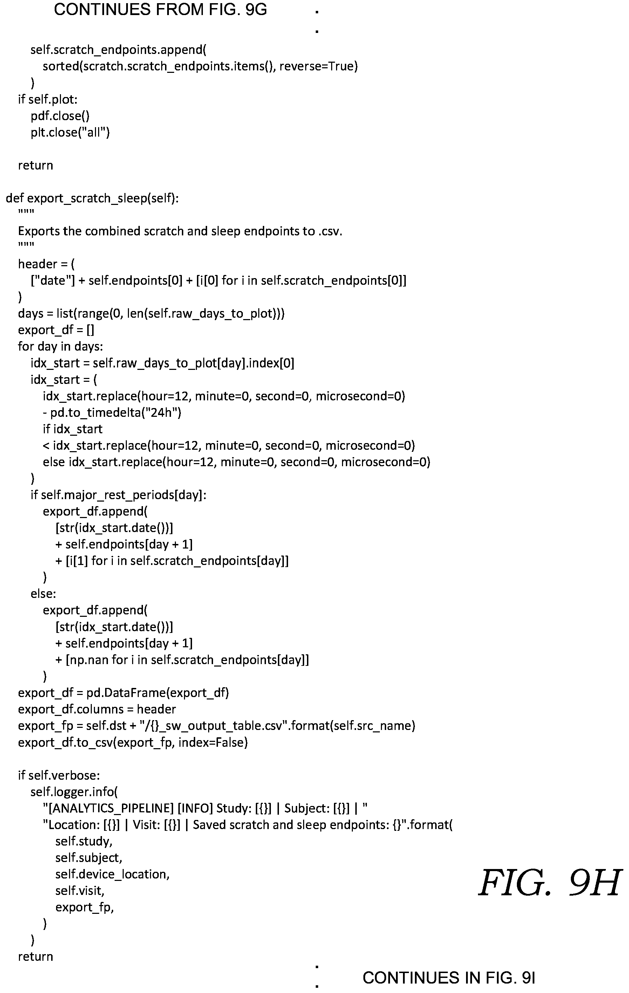

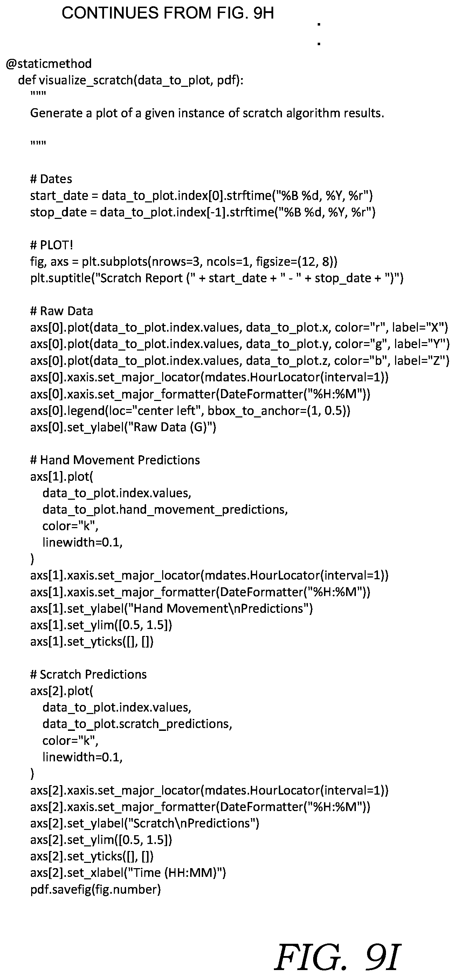

FIGS. 4A-E depict example aspects of scratch detection. FIG. 4A, for example, depicts a flow diagram illustrating an example method 400 for detecting scratch and initiating an action based on the detected scratch, in accordance with an embodiment of the disclosure. Method 400 may be performed by embodiments of one or more components of system 200, such as scratch detector 260 described in connection with FIG. 2. Further, each block or step of method 400 and other methods described herein comprises a computing process that may be performed using any combination of hardware, firmware, and/or software. For instance, various functions may be carried out by a processor executing instructions stored in a memory. The methods may also be embodied as computer-usable instructions stored on computer storage media. The methods may be provided by a stand-alone application, a service or a hosted service (stand-alone or in combination with another hosted service), or a plug-in to another product, to name a few. Accordingly, method 400 may be performed by one or more computing devices, such as a smartphone or other user device, a server, or a distributed computing platform, such as in the cloud environment. Example aspects of computer program routines covering implementations of scratch detection are illustratively depicted in FIGS. 9A-11M and, in particular, FIGS. 10A-10I.

At step 410, sensor data is received. Sensor data may include motion sensor data associated with a monitored user (or patient), such as raw accelerometer data captured by a wrist worn sensor or device. Other sensed or determined data, such as user-entered data, near body temperature data, weather-related data, and the like, may also be received as sensor data. Embodiments of step 410 may include pre-processing operations, such as applying frequency filters, segmenting data into relevant windows, such as 3-second windows, and deriving transformed signals, such as a vector magnitude, a first principal component, and a second principal component. Step 410 may be performed by sensor 103 of FIG. 1 and/or data collection component 210 of FIG. 2.

Further, at step 420, it is determined if the sensor(s) is configured for proper data acquisition. This step may include detecting whether the sensor (such as sensor 103) is being worn or not by the monitored user, or being worn in a manner to capture the intended information. Step 420 may be performed by an embodiment of sensor wear determiner 261 of FIG. 2, and an example process for performing step 420 is depicted in, and described in conjunction with, FIG. 4B. One implementation of step 420 may measure physiological parameters of the monitored user, indicating whether the sensor device (or sensor) is being worn (e.g. near body temperature, heart rate, blood pressure, galvanic skin resistance, etc.). For instance, near-body temperature may be compared to a non-wear temperature threshold, which may be 25 degrees Celsius in one exemplary embodiment. In this case, it may be determined that the sensor is not worn when the temperature is below the non-wear temperature threshold. In another implementation, a monitored user may manually indicate sensor wear, such as by pressing a button on a sensor device, initiating a mode of the sensor device and/or an application running on or communicating with the sensor device, or otherwise indicating that the sensor is being worn.

At step 430, a user sleep opportunity is determined. A user sleep opportunity may be an interval of time during which the monitored user intends to sleep or is more likely to sleep compared to outside of that interval. This determination may be made utilizing motion sensed information, such as accelerometer data. Step 430 may be performed by an embodiment of sleep opportunity determiner 262 of FIG. 2. Further, some embodiments may determine a total sleep opportunity (TSO), as described with respect to sleep opportunity determiner 262. One implementation of determining TSO is depicted in, and described in conjunction with, FIG. 4C. Other embodiments of step 430 may include determining the user sleep opportunity by determining that the lights are out for a minimum interval of time (e.g., 10 minutes) utilizing a photodetector. In some other embodiments, sleep opportunity may be determined based on sensed physiological measures, or having the user indicate to a sensor (e.g., using a button or entering a sleep mode or an awake mode) when the monitored user is going to sleep and when the user is getting up from sleep.

Additionally, method 400 (more specifically, step 430) may further include determining periods of actual sleep (and/or periods of wake) during the user sleep opportunity. This aspect of step 430 may be carried out by sleep/wake detector 230 or its subcomponents activity index determiner 232 and/or sleep/wake classifier 234 in FIG. 2. Additionally, one example process for determining sleep/wake periods is depicted in FIG. 4D.

Sleep periods may be determined by computing activity index values from accelerometer data captured within a determined total sleep opportunity (TSO). In this way, sleep/wake detection may include applying a sequence of three algorithms. Firstly, a total sleep opportunity may be detected. Secondly, activity index values may be computed from accelerometer data captured during the determined TSO, and thirdly, periods of time within the determined TSO may be classified as sleep/wake periods based on the activity index values.

Other techniques for determining sleep in accordance with an embodiment of method 400 may be based on physiological parameters that may be sensed, such as brain activity determined by a head-worn sensor, or based on a combination of a plurality of physiological parameters and motion data. For instance, step 430 may detect sleep during a period of less motion indicated in the motion data coupled with heart rate and/or respiration rate changes that are consistent with sleep. Output of a sleep (or wake) detection may be endpoints shown in the example user interface depicted in FIG. 8B.

Continuing with method 400, at step 440, a user hand motion event (which may also be referred to generally as hand movement) may be detected. Example embodiments of step 440 may detect hand motion events based on the sensor data, such as accelerometer data, acquired from a wearable device, such as a wrist-worn or finger-worn device. Step 440 may be carried out by an embodiment of hand movement detector 264 of FIG. 2.

Further, at step 450, a likely scratch event may be detected. Step 450 may be determined from sensor data corresponding to detected hand movement. In this way, embodiments of step 450 determine whether detected hand movement is a scratch event or not. Specifically, features values, such as time and frequency domain feature values, may be extracted from sensor data corresponding to detected hand movement event, and the feature values may be input into one or more machine-learning classifiers, such as a random forest classifier, to determine whether or not the detected hand movement is likely a scratch event. Step 450 may be carried out by embodiments of features extractor 266 and scratch event classifier 268.

At step 460, a detected scratch event may be recorded. This step may include storing the classification of the scratch event and related contextual information. The scratch event data may be stored in individual record 240 and accessed for decision support, such as by decision support tool(s) 270. The scratch event data may further be provided to a user and/or a clinician, as described with respect to presentation component 220 of FIG. 2.

At step 470, an action may be initiated based on the detected scratch event. Example actions may include actions, recommendations, and/or directives for alleviating itch and reducing scratch events. Step 470 may be performed by embodiments of decision support tool(s) 270 and/or presentation component in FIG. 2. For example, step 470 may include initiating steps to treat a user's pruritus (or, more specifically, atopic dermatitis) using one or more therapeutic agents based on scratch events detected utilizing a sensor on a wearable device as described with respect to decision support tool 276. Method 400 may include tracking and/or monitoring the application and use of a therapeutic agent according to a recommended or directed treatment protocol provided at step 470.

The action may include sending or otherwise electronically communicating an alert or a notification to a user via a user device, such as user devices 102a-n in FIG. 1, or to a clinician via a clinician user device, such as clinician user device 108 in FIG. 1. The notification may indicate one or more scratch events have been detected and/or other scratch endpoints, such as total scratch event count, a total scratch duration, a longest scratch event duration, and/or a ratio of the duration between scratch events to the number of scratch events. Further, in some embodiments, the notification may include a scratch score, which may be computed utilizing one or more of these scratch endpoints. In some aspects in which sleep/wake period is detected, the notification may also include sleep endpoints and/or a sleep score determined utilizing sleep endpoints.

In some embodiments, an action may further include processing the scratch event data for further decision making, which may include providing a recommendation for treatment and support based on the detected scratch events. Such a recommendation may include a recommendation to consult with a healthcare provider, continue an existing prescription or over-the-counter medicine, start using an over-the-counter medicine (which may additionally include adding the medicine to an electronic shopping list and/or e-commerce cart), adjust thermostat settings, and/or continue monitoring scratch events. One or more of these actions may be performed automatically in response to the detected scratch events and, in some embodiments, detected sleep/wake periods.

FIG. 4B depicts a diagrammatic representation of an example process 4200 for detecting sensor wear. Process 4200 may represent an example process for performing step 420 of method 400 of FIG. 4A. An embodiment of this process may be performed by sensor wear determiner 261 of FIG. 2.

FIG. 4B depicts a series of steps 4201 for detecting wear and non-wear periods. Within the series of steps 4201, at step 4210, raw tri-axial motion data is received, such as from an accelerometer. The tri-axial motion data comprises x-axis measurements, y-axis measurements, and z-axis measurements. This data may be pre-processed by applying one or more filters as previously described. The tri-axial motion data may be split into overlapping windows, such as one hour windows with 15 minute overlap. At step 4230, statistical measures for the x-axis, y-axis, and z-axis measurements for a window satisfies a non-wear threshold. As depicted in FIG. 4B, an initial non-wear determination is made for a window at step 4240 if either any the standard deviations for any two axes is less than 0.13Gs or the ranges for any two axes is less than 0.15Gs. **If** neither of those non-wear thresholds are satisfied in step 4230, the window may be initially determined to be a wear window.

At step 4250, a set of rescoring rules may be applied to determine whether or not to change the initial determination of wear or non-wear for a given window or block of windows. Further details of heuristic rules to apply for rescoring at step 4250 are discussed in conjunction with sensor wear determiner 261 of FIG. 2. At step 4260, one or more windows initially determined to be a wear window (or block of windows) may be rescored as non-wear at step 4260. In other embodiments, rescoring may alternatively or additionally include rescoring a non-wear window or block of windows to a wear window or block of windows.

FIG. 4B also includes diagram 4270 depicts initial determinations of wear and non-wear windows. In example, block 4271 includes eight windows identified as wear windows, which is followed by block 4273 comprising four windows identified as non-wear windows. Block 4273 is followed by block 4275 comprising two wear windows, which are followed by block 4277 comprising three non-wear windows. Block 4277 is followed by block 4279 comprising six wear windows. As described in conjunction with sensor determined 261 of FIG. 2, a rescoring rule may include rescoring a block of windows from wear to non-wear if the block is less than three hours and the previous block is greater than one hour . As such, block 4275 may be recorded to a non-wear block of windows.

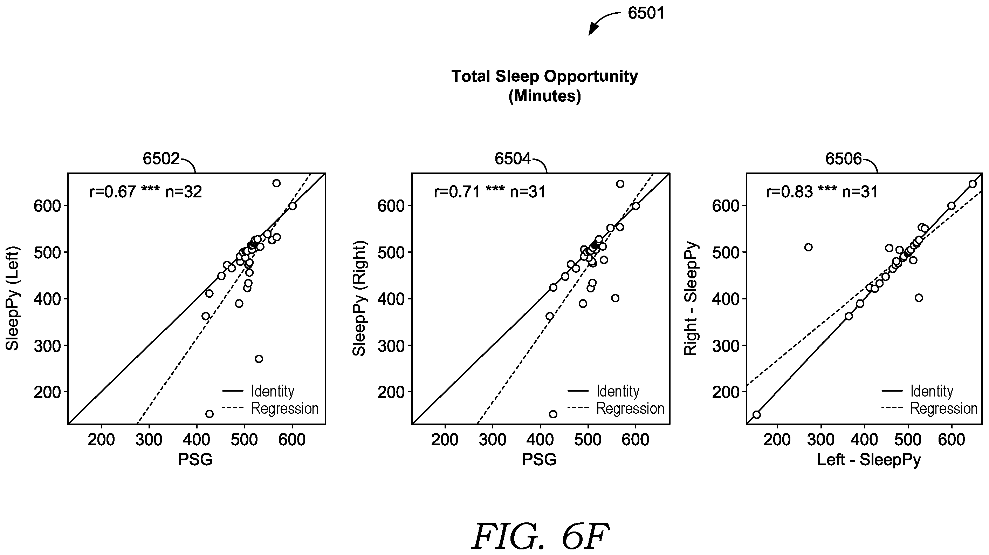

FIG. 4C depicts a diagrammatic representation of an example process 4300 for determining a user's sleep opportunity (e.g. TSO). Process 4300 may represent an example of process for performing step 430 of method 400 of FIG. 4A. Further, an embodiment of process 4300 may be performed by sleep opportunity determiner 262 of FIG. 2. Additionally, FIG. 6F depicts an aspect of performance validation for the algorithm described in process 4300 of FIG. 4C. As explained below, exemplary aspects utilizing TSO for the user's sleep opportunity. TSO advantageously captures times when a user is having difficulty sleeping, which may be a consequence of scratching. Utilizing accelerometer data to determine the sleep opportunity may further be advantageous over only using light information as individuals may spend time on their laptops or mobile devices while in a dark room and not intend to sleep. Reliably detecting sleep opportunity within which to measure scratch helps effectively determine how an individual's sleep and nighttime scratch vary on a day-to-day basis.

Process 4300 may generally include determining a user's total sleep opportunity based on the change in arm angle measured from motion data. At step 4310, rolling medians of raw tri-axial motion signal measurements are determined. For example, 5-second rolling medians of x-axis, y-axis, and z-axis measurements are determined at step 4310, and the median measurements are utilized to determine arm angles at step 4320.

At step 4330, average arm angle values may be computed for intervals (e.g., consecutive 5 seconds), and absolute differences between successive average arm angle values may be computed at step 4340. At step 4350, rolling medians of the difference between successive average arm angle values may be computed for an interval (e.g., 5 minutes). At step 4360, candidate rest periods may be determined by comparing the rolling median of the difference between successive average arm angle values to a rest threshold. For example, a candidate rest period may be detected when the median difference between successive average arm angle values is less than or equal to 0.15 multiplied by the 10th percentile value of all differences in arm angle values within the 24-hour period.

At step 4370, candidate rest periods identified as non-wear (which may be determined as described in conjunction with FIG. 4B, may be filtered out of consideration for the total sleep opportunity. At step 4380, the remaining candidate rest periods may be compared to a threshold length, such as 30 minutes, such that candidate rest periods are kept if they are greater than 30 minutes. Additionally, at step 4390, candidate periods may be grouped together if the gaps between the periods satisfy a threshold length of time, such as being less than 15 minutes. At step 4395, the longest group of candidate periods within a set time period (e.g., 24-hour period) may be determined to be the user's total sleep opportunity.

FIG. 4D depicts a diagrammatic representation of an example process 4800 for detecting user sleep periods and wake periods. Process 4800 may represent an example of the process to perform step 430 of detecting sleep and/or wake periods within an embodiment of method 400, as described in conjunction with FIG. 4A. Further, an embodiment of process 4800 may be performed by sleep/wake detector 230 of FIG. 2 or its subcomponents.

Process 4800 may detect a user's sleep/wake periods utilizing activity index values calculated from motion data. At step 4810, a filter may be applied to motion sensor data. For instance, a high-pass filter with a cutoff of 0.25 Hz may be applied to the motion. Sleep opportunity may be segmented into windows of a predetermined length, and, at step 4820, an activity index level may be computed for each window, such as one minute. Activity level values may be computed as illustrated at step 4820 in FIG. 4D.

At step 4830, a weighted sum of activity index values within a particular time period may be determined. For instance, the weighted sum for a one-minute window may be computed using activity index values over a span of 7 minutes, such as from time instances t-4 to *t*+4.

At step 4840, each weighted sum may be compared to a sleep threshold to determine whether to initial categorize the period as a sleep period. For example, the sleep threshold may be 0.5 and a window may be classified as a sleep period if the weighted sum for that period is less than 0.5. At step 4850, one or more rescoring rules may be applied to classify a period from sleep to awake and/or from awake to asleep. The rescoring rules may be as described in conjunction with sleep/wake classifier 234 of FIG. 2.

At step 4860, aggregate sleep endpoints may be determined for the total sleep opportunity. These sleep endpoints may include total sleep time (TST), percent time asleep (PTA), wake after sleep onset (WASO), sleep onset latency (SOL), and number of wake bouts (NWB). These sleep endpoints may be utilized as described with respect to decision support tool(s) 270 in FIG. 2.

FIG. 4E depicts a diagrammatic representation of example aspects of a scratch detection process 4001. Process 4001 may include classifying scratch events and, thus, may be referred to herein as a scratch classifier pipeline. Aspects of process 4001 may be performed by one or more components of system 200, such as scratch detector 260 or its subcomponents.

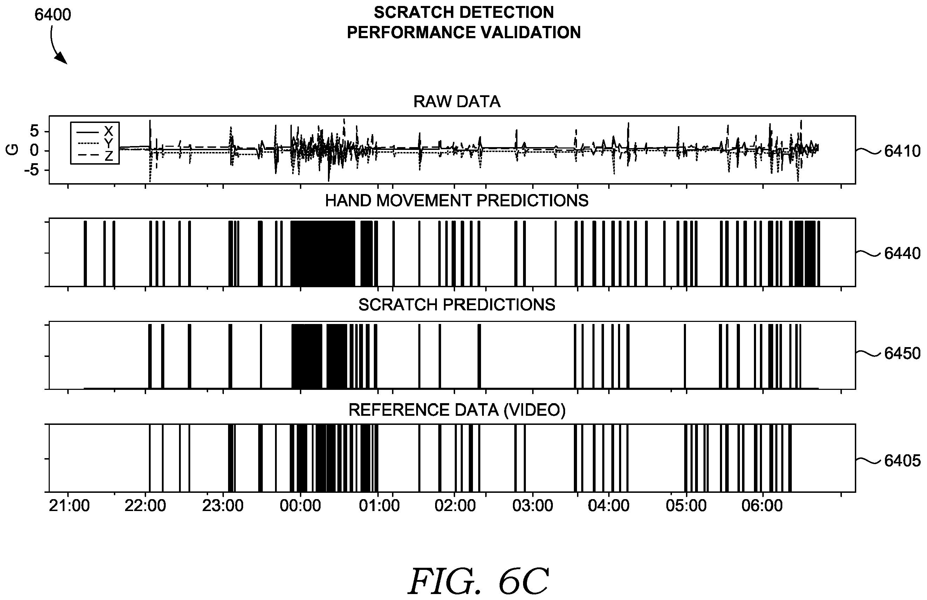

Initially, at block 4010, sensor data may be received, which may include preformatting or preprocessing raw accelerometer data. **In** some embodiments, raw data can be in the form of an example signal 6410, as depicted in FIG. 6C. As such, block 4010 may include segmenting accelerometer data during a detected total sleep opportunity (TSO) interval for a 24-hour period into 3-second non-overlapping windows, as shown in steps 4012 and 4014. **In** some embodiments, other lengths of windows, such as 1 second and 2 seconds may be utilized for segmenting. Block 4010 may be performed in accordance with embodiments of steps 410, 420, and/or 430 of FIG. 4A.

The rest of process 4001 may include generating predictions of scratch via a two-tier approach. First, the presence of hand movement is determined (see block 4040), and then those periods of hand movement are classified as either scratch events or non-scratch events (see block 4050). At block 4040, each 3-second window is passed through a heuristic hand movement detection algorithm to determine the presence of hand movement. Steps 4042 and 4044 within block 4040 may be performed by an embodiment of hand movement detector 264 of FIG. 2 and in accordance with an embodiment of step 440 of FIG. 4A.

The hand movement detection algorithm includes computing rolling (1-second) coefficient of variation (CoV), as shown at step 4042. These computed CoV values may be compared to a hand movement threshold, at step 4044. A parameter of the hand movement detection algorithm (threshold on calculated rolling coefficient of variation) may be tuned empirically based on a training dataset. For example, it may be determined that the 25th percentile of all calculated coefficient of variation values in the training dataset provides accurate results. **In** one embodiment, this threshold CoV value may be 0.023. **In** some embodiments, hand movement detection algorithm may use an example hand movement prediction signal 6440, as depicted in FIG. 6C. If hand movement is detected for the entirety of a given 3-second window at step 4044, it is sent for scratch classification.

Scratch classification is represented by block 4050. Steps within block 4050 may be performed by features extractor 266 and scratch event classifier 268 of FIG. 2 and may be performed in accordance with step 450 of FIG. 4A. **In** example embodiments, a binary machine learning (ML) classifier is trained to detect presence of scratch. The classifier may be trained in accordance with an embodiment of pipeline 600 in FIG. 6A, as described below.

An example pipeline for predicting scratch at block 4050 includes preprocessing step 4052, feature extraction 4054, classification 4056, and computing endpoints 4058. The preprocessing step 4052 may generate three processed signals by applying filtering and dimensionality reduction to raw accelerometer data. First, the raw accelerometer data may be filtered using a high-pass filter, such as a first order Butterworth Infinite Impulse Response (IIR) high-pass filter with a cutoff frequency of 0.25 Hz. Next, in order to reduce dependency on device orientation, vector magnitude and first and second principal components of the filtered signal may be computed.

At step 4054, time and frequency domain features may be computed from the processed accelerometer data. An embodiment of step 4054 may utilize 26 features as identified above with respect to features extractor 266 of FIG. 2. These features may be selected during training of a classifier, as described with respect to FIGS. 6A-6B.

At step 4056, the computed features may be run through the trained scratch classifier. **In** one embodiment, the scratch classifier is a random forest classifier. Further, the random forest classifier may include 50 estimators. The scratch classifier may determine, utilizing the computed features, whether the detected hand movement is likely a scratch event or not. Further details of step 4056 may be described with respect to scratch event classifier 268 in FIG. 2. **In** some embodiments, the scratch event classifier may predict scratch based on a detected scratch event signal 6450 that is determined at step 4056 and depicted in FIG. 6C.

At step 4058, digital endpoints of nighttime scratch (also referred to as scratch endpoints) may be derived by processing the scratch predictions during the determined sleep opportunity for each 24-hour period. The scratch endpoints may include total scratch events and total scratch duration. The sleep opportunity, such as TSO, may also be included as a digital endpoint as it is used for scratch detection. The table below summarizes some digital endpoints derived in an embodiment of step 4058.

| Endpoint | Type | Units | Description |
|---|---|---|---|
| Total sleep opportunity | Sleep | Minutes | Largest window of time where sleep is the intended behavior |
| Total scratch events | Scratch | Counts | Total scratch bouts during the total sleep opportunity window |
| Total scratch duration | Scratch | Minutes | Total time scratching during the total sleep opportunity window |

Implementations of process 4001 may be performed with only one sensor, such as a wrist-worn sensor device. Some embodiments, however, may also function with two sensors, such as when a user is wearing a device on each wrist. When there are two sensors, total scratch counts may be computed by taking the sum of contiguous 3-second bouts of predicted scratch detected from both wrists, and total scratch duration may be computed by taking the sum of the durations of all predicted scratch bouts from both wrists.

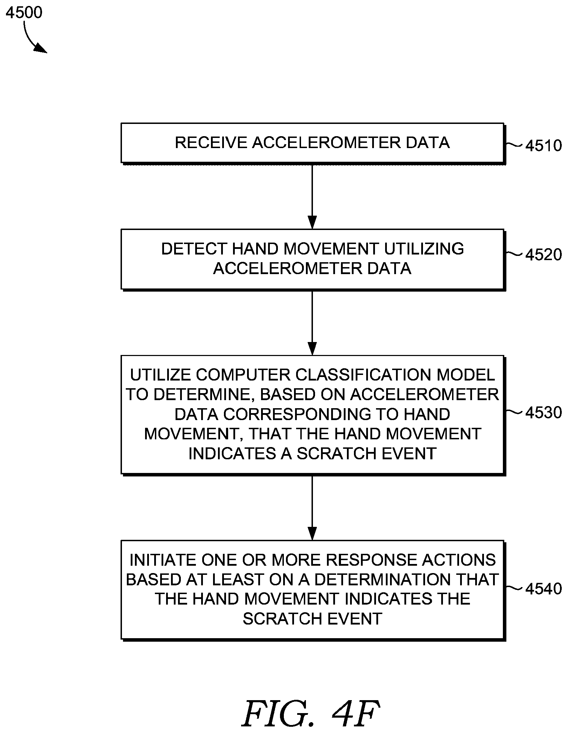

FIG. 4F depicts a flow diagram illustrating an example method 4500 for providing decision support based on scratch events, in accordance with an embodiment of the disclosure. Method 4500 may be performed by embodiments of one or more components of system 200, such as scratch detector 260 described in connection with FIG. 2. Example aspects of computer program routines covering implementations of scratch detection are illustratively depicted in FIGS. 9A-11M and, in particular, FIGS. 10A-10I.

At step 4510, accelerometer data is received. The accelerometer data may be captured by a wearable device associated with an individual (e.g., a monitored subject or patient) and located at an appendage of the individual. For example, the wearable device may be located at the individual's wrist, arm, and/or finger. Other sensed or determined data, such as user-entered data, near-body temperature data, weather-related data, and the like, may also be received as sensor data. The wearable device may include a plurality of sensors for capturing different types of data, such as accelerometer data and at least one of near-body temperature data and light data. Step 4510 may be performed by sensor 103 of FIG. 1 and/or data collection component 210 of FIG. 2. Some embodiments of step 4510 may be similar to step 410 of method 400 discussed in conjunction with FIG. 4A. Additionally, some embodiments of method 4500 may include determining if the sensor(s) is configured for proper data acquisition as described in step 420 of FIG. 4A.

At step 4520, a hand movement is detected utilizing the accelerometer data. Step 4520 may be carried out by an embodiment of hand movement detector 264 of FIG. 2. Some embodiments of steps 4520 may be similar to embodiments of step 440 of method 400.

At step 4530, a computerized classification model is utilized to determine that the hand movement indicates a scratch event. This determination may be based on the accelerometer data corresponding to the hand movement. **In** some embodiments, step 4530 includes generating a multidimensional timeseries from the accelerometer data corresponding to the hand movement and determining feature values from the multidimensional timeseries. The feature values may include at least one time-domain feature value and at least one frequency-domain feature value. The determination that the hand movement indicates the scratch event may be based on the feature values. Step 4530 may be carried out by embodiments described in connection with scratch detector 260, and more specifically embodiments described in connection with features extractor 266 and scratch event classifier 268, of FIG. 2. Additionally, some embodiments of step 4530 may be similar to embodiments of step 450 of method 400. Some embodiments of method 4500 include recording the determination of the scratch event as further described with respect to step 460 of method 400.

At step 4540, one or more response actions are initiated based on the determination that the hand movement indicates the scratch event. Example actions may include actions, recommendations, and/or directives for alleviating itch and reducing scratch events. Step 4540 may be performed by embodiments of decision support tool(s) 270 and/or presentation component in FIG. 2. For example, step 4540 may include initiating steps to treat a user's pruritus (or, more specifically, atopic dermatitis) using one or more therapeutic agents based on scratch events detected utilizing a sensor on a wearable device as described with respect to decision support tool 276. Some embodiments of step 4540 may be similar to embodiments of step 470 of method 400.

In some embodiments, the response action includes generating a graphic user interface element providing on display of a user device, such as user computer device 102a-c, patient user device 102n, or clinician user device 108 of FIG. 1, which may be performed by or in conjunction with an embodiment of presentation component 220 of FIG. 2. The graphic user interface element may include at least one of an indicator of one or more scratch endpoints (e.g., total number of scratch events and total scratch duration), and an indicator recommending that the individual seek clinical consultation based on the determination that the hand movement indicates the scratch event.

Some embodiments of method 4500 may include determining a total sleep opportunity based on the accelerometer data. The total sleep opportunity may be a period of time during which the individual lays down for a rest and when the individual gets up from the rest. The hand movement detected at step 4520 may be detected utilizing accelerometer data only corresponding to the total sleep opportunity. Some embodiments of this process may be similar to step 430 in method 400 and/or may be performed by an embodiment of sleep opportunity determiner 262 of FIG. 2. In embodiments of method 4500 in which a response action includes providing a graphic user interface element indicating one or more scratch endpoints, the scratch endpoints may be confined to total sleep opportunities (either one total sleep opportunity or multiple total sleep opportunities).

At least one of near-body temperature and light data captured by a wearable device may be used, in addition to the accelerometer data, to determine the total sleep opportunity. Additionally, this determination of the total sleep opportunity may further include determining periods of actual sleep (and/or periods of wake) during the total sleep opportunity, which may be carried out by sleep/wake detector 230 or its subcomponents, activity index determiner 232 and/or sleep/wake classifier 234, in FIG. 2. Additionally, an example process for determining sleep/wake periods is depicted in FIG. 4D.

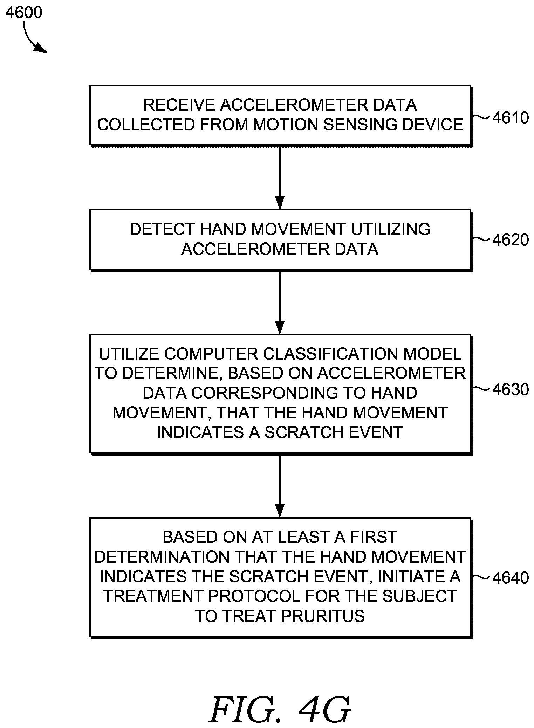

FIG. 4G depicts a flow diagram illustrating an example method 4600 for treating pruritus utilizing a motion sensing device associated with a subject, in accordance with an embodiment of the disclosure. Method 4600 may be performed by embodiments of one or more components of system 200, such as scratch detector 260 and/or decision support tools 270 described in connection with FIG. 2. Example aspects of computer program routines covering implementations of scratch detection are illustratively depicted in FIGS. 9A-11M and, in particular, FIGS. 10A-10I.

At step 4610, accelerometer data collected from a motion sensing device is received. The accelerometer data may be captured by a wearable device associated with a subject at located at the subject's appendance (e.g., at the individual's wrist, arm, and/or finger). Other sensed or determined data, such as user-entered data, near-body temperature data, light data, weather-related data, and the like, may also be received from the motion sensing device or another device having a sensor(s). The wearable device may include a plurality of sensors for capturing different types of data, such as accelerometer data and at least one of near-body temperature data and light data. Step 4610 may be performed by sensor 103 of FIG. 1 and/or data collection component 210 of FIG. 2. Some embodiments of step 4610 may be similar to embodiments of step 410 of method 400 discussed in conjunction with FIG. 4A. Additionally, some embodiments of method 4600 may include determining if the sensor(s) is configured for proper data acquisition as described in step 420 of FIG. 4A.

At step 4620, a hand movement is detected utilizing the accelerometer data. Step 4620 may be carried out by an embodiment of hand movement detector 264 of FIG. 2. Some embodiments of steps 4620 may be similar to embodiments of step 440 of method 400.

At step 4630, a computerized classification model is utilized to determine that the hand movement indicates a scratch event. This determination may be based on the accelerometer data corresponding to the hand movement. **In** some embodiments, step 4630 includes generating a multidimensional timeseries from the accelerometer data corresponding to the hand movement and determining feature values from the multidimensional timeseries. The feature values may include at least one time-domain feature value and at least one frequency-domain feature value. The determination that the hand movement indicates the scratch event may be based on the feature values. Some embodiments of step 4603 may be carried out by embodiments described in connection with scratch detector 260, and more specifically embodiments described in connection with features extractor 266 and scratch event classifier 268. Additionally, some embodiments of step 4630 may be similar to embodiments of step 450 of method 400. Some embodiments of method 4600 include recording the determination of the scratch event as further described with respect to step 460 of method 400.

At step 4640, a treatment protocol for the subject to treat pruritus may be initiated based on at least a first determination that the hand movement indicates the scratch event. Step 4640 may be performed by embodiments of decision support tool(s) 270 (e.g., tool 476) and/or presentation component 220 in FIG. 2. Some embodiments of step 4640 may be similar to embodiments of step 470 of method 400. **In** some embodiments, the subject is diagnosed based on the determination that the hand movement indicates a scratch event, and the treatment protocol may be to treat atopic dermatitis.

**In** some embodiments the treatment protocol is further based on a plurality of determination the a plurality of hand movements each indicate a scratch event. For example, the treatment protocol may be based on a pattern of scratching determined for the subject.

Some embodiments of step 4640 include determining at least one of a therapeutic agent, a dosage, and a method of administration of a therapeutic agent for determining the treatment protocol. **In** some aspects, the therapeutic agent is selected from the group consisting of: infliximab, adalimumab, belimumab, tanezumab, ranibizumab, bevacizumab, mepolizumab certolizumab, natalizumab, ustekinumab, vedolizumab, 6-mercaptopurine, hydroxychloroquine, obeticholic acid, mofetil, sodium mycophenolate, leflunomide, rituxan, solumedrol, depomedrol, betamethasone, prednisone, cyclosporin, tacrolimus, pimecrolimus, dupilumab, omalizumab, tralokinumab, etokimab, nemolizumab, Tezepelumab, lebrikizumab, fezakinumab, anti-OX40, efalizumab, etanercept, crisaborole, fluocinonide, mapracorat, hydrocortisone, desonide, alclometasone, triamcinolone, desoximetasone, loratidine, fexofenadine, desloratidine, levocetirizine, methapyrilene, cetirizine, budesonide, fluticasone, mometasone, dexamethasone, prednisolone, ciclesonide, beclomethasone, methotrexate, azathioprine, aspirin, ibuprofen, celecoxib, valdecoxib, WBI-1001 and/or MRX-6, abrocitinib, baricitinib, brepocitinib, cerdulatinib, decernotinib, delgocitinib, fedratinib, filgotinib, gandotinib, ilginatinib, itacitinib, lestaurtinib, momelotinib, oclacitinib pacritinib, peficitinib, ritlecitinib, ruxolitinib, tofacitinib, upadacitinib, THRX-212401, PF-07055087, PF-06471658, PF-07055090, ATI-502, BMS-986165, JTE052, PF-06826647, SNA 152, SHR-0302, tapinarof, and/or alitretinoin. In a preferred embodiments, the therapeutic agent is crisaborole and/or abrocitinib.

In some embodiments, initiating administration of the treatment protocol includes generating a graphic user interface element provided for display on a user device. the graphic user interface element may indicate a recommendation of the treatment protocol that is based on the first determination that the hand movement represents the scratching element. In one example, the user device is separate from the motion sensing device. For example, the motion sensing device may be an example of the user computer device 102a-c or patient user device 102n of FIG. 2 while the user device may be a clinician user device 108 of FIG. 2. Alternatively, the user device may be another user computer device 102a-c or patient user device 102 of FIG. 2. Generating the graphic user interface element may be performed by or in conjunction with an embodiment of presentation component 220 of FIG. 2. Some embodiments of method 4600 further include applying the treatment protocol to the subject based on the recommendation. Some embodiments of method 4600 may include determining a total sleep opportunity based on the accelerometer data as further described with respect to step 430 in method 400 and the hand movement used to determine the scratch event may be detected from accelerometer data corresponding to the total sleep opportunity.

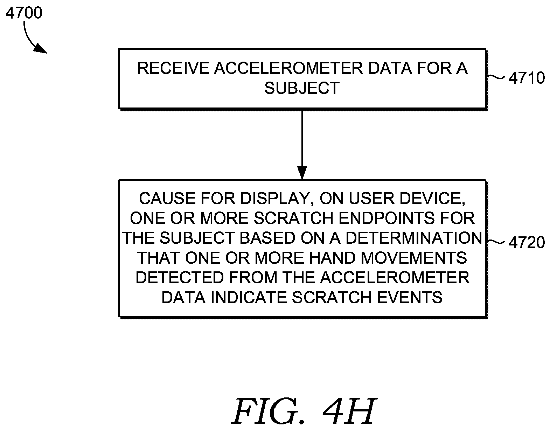

FIG. 4H depicts a flow diagram illustrating an example method 4700 utilizing scratch detection, in accordance with an embodiment of the disclosure. Method 4700 may be performed by embodiments of one or more components of system 200, such as scratch detector 260 and/or decision support tools 270 described in connection with FIG. 2. Example aspects of computer program routines covering implementations of scratch detection are illustratively depicted in FIGS. 9A-11M and, in particular, FIGS. 10A-10I.

At step 4710, accelerometer data is received for a subject. The accelerometer data may be captured by a motion sensing device, which may be a wearable device associated with subject at located at the subject's appendance (e.g., at the individual's wrist, arm, and/or finger). Other sensed or determined data, such as user-entered data, near body temperature data, light data, weather-related data, and the like, may also be received from the motion sensing device or another device having sensor(s). The wearable device may include a plurality of sensors for capturing different types of data, such as accelerometer data and at least one of near-body temperature data and light data. Step 4710 may be performed by sensor 103 of FIG. 1 and/or data collection component 210 of FIG. 2. Some embodiments of step 4710 may be similar to embodiments of step 410 of method 400 discussed in conjunction with FIG. 4A. Additionally, some embodiments of method 4700 may include determining if the sensor(s) is configured for proper data acquisition as described in step 420 of FIG. 4A. **In** some embodiments, the accelerometer data is captured by a sensor integrated into a first wearable device and a second wearable device worn contemporaneously by the subject. For example, the subject may wear a wrist-worn motion sensing device on each of the subject's wrists.

At step 4620, one or more scratch endpoints for the subject are provided for display on a user device. The scratch endpoints are based on a determination that one or more hand movements detected from the accelerometer data indicate scratch events. Detecting one or more hand movements from the accelerometer data may be performed by an embodiment of hand movement detector 264 of FIG. 2 and as described with respect to step 440 of method 400. Further, determining the one or more hand movements indicate scratch events may be done utilizing a computerized classification model by an embodiment described in connection with scratch detector 260, or more specifically features extractor 266 and scratch event classifier 268, and/or as further described with respect to step 450 of method 400. The scratch endpoints may include a total scratch event count and/or a total scratch duration, among others. The scratch endpoints may be confined to the subject's sleep opportunity (e.g., total sleep opportunity) as further described with respect to step 430 in method 400.

The graphic user interface element may be provided for display on user device that is communicatively coupled to a wearable device with sensors capturing the accelerometer data. For example, the user device may be a smart phone that is connected to a wearable device that captures the accelerometer data. Example embodiments of the user device and wearable device include user computer device 102a-c, patient user device 102n, and clinician user device 108 of FIG. 1.

Some embodiments of method 4700 include providing for display, on the user device, a treatment protocol for the subject for treating atopic dermatitis. The treatment protocol may include a therapeutic agent, a dosage, and/or a method of administration, and may be based on the one or more scratch endpoints. Example therapeutic agents that may be included in method 4700 includes the therapeutic agents described at step 4640 in method 4600.

FIG. 5 depicts a flow diagram illustrating a method 500 for flare prediction, in accordance with an embodiment of the disclosure. Method 500 may be performed by one or more components of system 200, such as flare predictor 290, including its subcomponents. Similar to method 400, each block or step of method 500 comprises a computing process that may be performed using any combination of hardware, firmware, and/or software. For instance, various functions may be carried out by a processor executing instructions stored in memory. The method may also be embodied as computer-usable instructions stored on computer storage media. The method may be provided by a stand-alone application, a service or a hosted service (stand-alone or in combination with another hosted service), or a plug-in to another product, to name a few. Accordingly, method 500 may be performed by one or more computing devices, such as a smartphone or other user device, a server, or by a distributed computing platform, such as in the cloud environment.

At step 510, user scratch patterns may be determined. Step 510 may be performed by an embodiment of scratch patterns assembler 292 of FIG. 2. Scratch patterns may be determined from a user's historical scratch event data, such as historical scratch events 244 stored in the user's individual record 240, as described in conjunction with FIG. 2. Historical scratch event data includes scratch endpoints determined from detected scratch events such as total scratch episodes counts, total scratch duration, duration between scratch events, and/or a ratio of duration between scratch events and number of scratch events. A scratch pattern may indicate a change in scratch event endpoints, such as an increase in nightly scratch episode counts or a decrease in duration between scratch events.

At step 520, contextual information may be determined. Step 520 may be performed by an embodiment of contextual data determiner 294. The determined contextual information may include weather information, such as atmospheric temperature and/or humidity; user health data, such as a user's age, weight, diagnosed conditions, past prescriptions or therapies, and current medications; and user-input data, such as a user-defined itch rating, notes, photographs of the user's skin, and/or treatment logs. **In** some embodiments, user health data may be determined from a user's profile/health record (EHR) 241 stored in the individual record 240 of FIG. 2.

At step 530, a user's itch may be determined for a future time interval. Step 530 may be performed by an embodiment of itch predictor 296. The determined future itch is a likelihood of future itching within a future time frame, such as tomorrow, the next day, or in five days. The determined future itch may include a level or magnitude, which may represent the severity level of a predicted or future itch.

A future itch may be determined at step 530 utilizing the user's scratch patterns and contextual information determined at steps 510 and 520, respectively. Various types of logic may be employed at step 530 to determine user's itch in the future. As described with respect to itch prediction logic 259 of FIG. 2, future itch may be determined utilizing rules, conditions, thresholds, associations, machine learning models, or other criteria for inferring or detecting a likelihood of itch (either generally or a level/severity of itch) occurring in the future. For example, fuzzy logic, neural network(s), finite state machine, support vector machine, logistic regression, clustering, other machine-learning techniques, similar statistical classification processes, or a combination of these may be utilized at step 530.

As may be appreciated, a user's itch may be determined for multiple future time frames, and the itch level predicted may vary within different time frames. For example, at step 530, a user may be determined to have a "low" itch level in two days, but may be determined to have a "high" itch level in five days.

At step 540, a likelihood of a flare event within a future time interval may be determined. Step 540 may be performed by an embodiment of itch predictor 296 or, more generally, flare detector 290. Determining a likelihood of a future flare event may include comparing a predicted itch level to one or more flare detection thresholds to determine whether the predicted itch level is of sufficient severity to be a flare risk. **In** some embodiments, the flare detection threshold(s) may be predetermined based on a reference population such that the flare detection threshold may be utilized for the population at large. **In** other embodiments, a flare detection threshold(s) is determined for each monitored individual. For instance, the flare detection threshold may be set based on the user's historical information, including health data such as condition and age. Further, the flare detection threshold(s) may be set by a clinician/caregiver of the user and/or adjusted by the user. This set threshold may be stored in settings 249 of individual record 240, as described in FIG. 2.

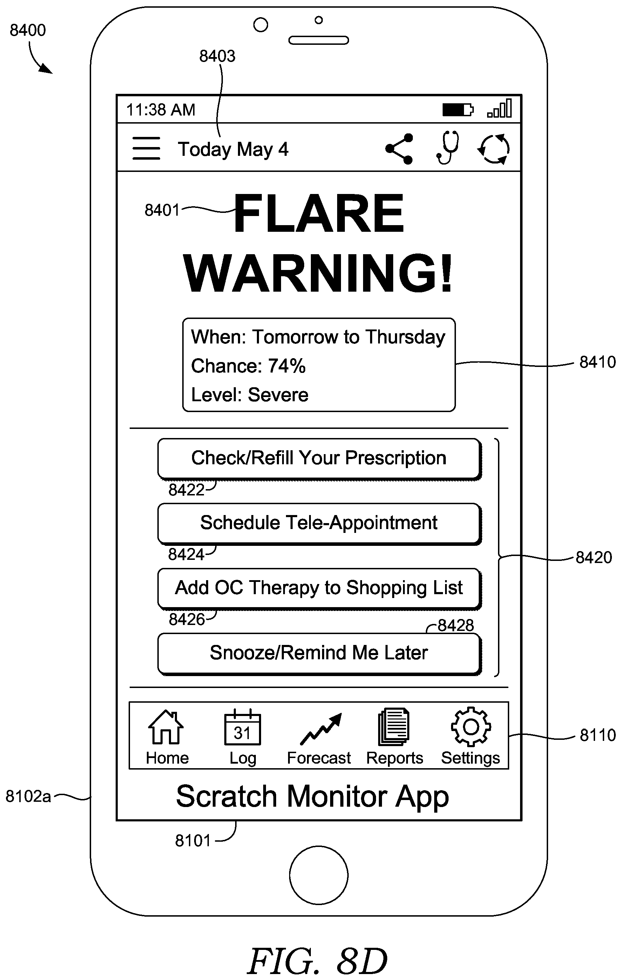

At step 550, an action may be initiated based on the determined likelihood of a flare event and/or user itch. As such, step 550 may be performed by an embodiment of flare notification generator 298 and/or decision support tool 270, such as tool(s) 272, 274, or 276. **In** some embodiments, a flare notification or an alert indicating a user's itch and/or flare risk may be generated. **In** one exemplary embodiment, where an itch level satisfies a flare detection threshold, a flare notification indicating the risk may be sent to a user device of the monitored user. **In** another exemplary embodiment, the flare notification is sent to a clinician's user device for the clinician to accordingly treat the monitored user. Example embodiments of a flare notification generated in accordance with embodiments of step 550 are described below with respect to FIGS. 8D-8F and above with respect to FIG. 3B. For instance, the flare notification may provide contextual or historical information.

Initiating an action at step 550 may also include generating recommendations or directives or initiating actions based on the itch level or flare risk. As an example, a recommendation to schedule an appointment with a caregiver, refill prescription, and/or add an over-the-counter therapy to a user's shopping list may be generated and presented to the user. Further, in some embodiments, initiating an action may include adding the prediction to a user's electronic calendar, such as in a monitoring or tracking application, or modifying a user interface element in the user's device to indicate the predicted risk within an electronic calendar. Some embodiments of step 550 include initiating steps to treat a user's pruritus (or, more specifically, atopic dermatitis) using one or more therapeutic agents, e.g., crisaborole and/or abrocitinib, based on a flare prediction generated utilizing data obtained using a sensor on a wearable device as described with respect to decision support tool 276. Method 500 may include tracking and/or monitoring the application and use of a therapeutic agent according to a recommended or directed treatment protocol provided at step 470.

Further, some embodiments of step 550 may include utilizing a response log, such as logs 246 in FIG. 2, indicating how a user responded to a notification of a predicted itch or flare risk and/or support recommendation, to improve the itch/flare predictor. For example, subsequent scratch events, itch predictions and/or flare predictions may be correlated with a prior itch or flare prediction and response, which may indicate whether the generated response resulted in an increase or decrease in scratch events and/or an increase or decrease in a predicted itch level or flare risk.

FIGS. 6A-F depict aspects of training example embodiments of a sleep detector. FIG. 6A provides a diagrammatic representation of an example process 600 for training of a scratch detector. Example process 600 is a supervised training process that generates and uses labeled data based on video annotations of accelerometer data. An example pipeline for training a scratch classifier may include steps for preprocessing (including data preprocessing at block 610 and signal preprocessing at block 620), feature engineering at block 630 and feature selection, model training and model evaluation at block 640.

Data preprocessing at block 610 includes, at step 612, alignment of video annotations to accelerometer data. To generate labels for training the scratch classifier, annotations of nighttime scratch and restless (non-scratch) movements may be created by human annotators who view thermal videos of in-clinic subject visits. Annotations may be performed by two human annotators and reviewed by an arbitrator for accuracy. Each annotation may include metadata, indicating which hand was moving (right, left, or both) in embodiments in which sensors are worn are both hands; the affected body location; as well as severity (mild, moderate, severe) of the scratch. To accurately make use of the reference video-based annotations, all annotations may be time-aligned with the accelerometer data, at step 612. Alignment of the video annotations and the accelerometer data may performed manually based on a prescribed clap event (i.e., subjects may be instructed to clap in front of a camera while wearing accelerometer devices) during each in-clinic visit.

Data preprocessing further includes, at 614, down sampling the accelerometer data to 20 Hertz (Hz), which may help maximize battery life. Data preprocessing further includes filtering the annotations, at step 616. **In** exemplary aspects, annotations of three seconds or longer may be used in training a binary classifier. If an annotation is greater than three seconds, it may be segmented into three-second windows, at step 616. **In** some embodiments, the windows may be overlapping, such as with a 50% overlap in the three-second windows. Step 616 may also include determining whether hand movement is present throughout the annotated three-second window and filtering out data that does not have hand movement throughout.

Preprocessed data from block 610 may then be passed to block 620 for signal preprocessing. Signal pre-processing steps in block 620 may be similar to preprocessing step 4052 described in connection with FIG. 4E and may include segmenting and applying filtering and dimensionality reduction to raw accelerometer data. First, x, y, and z signal segments may be segmented into 3-second windows (similar to the video annotations), at step 622. At step 624, x, y, and z data may be filtered using a high-pass filter, such as a first order Butterworth **IIR** high-pass filter with a cutoff frequency of 0.25 Hz. Next, to reduce dependency on device orientation, the transformed signals may be derived from the filtered signal, at step 626. For example, vector magnitude and first and second principal components of the filtered signal may be computed.

The transformed signals may then be passed to block 630 for feature engineering. At step 632, a total of 36 time and frequency domain features are extracted from the transformed signals for each window. These 36 features may include, but not limited to, the following:

- Root mean square (RMS) value of vector magnitude - RMS is a measure of signal energy and may be correlated with amount and intensity of motion;

- Signal range of vector magnitude - Signal range is a measure of the extremes of motion observed in a given time window of sensor data, where a higher range may indicate occurrence of a large excursion in sensor values;

- Signal entropy of vector magnitude, first principal component, and second principal component - Signal entropy may be calculated by estimating Shannon entropy of the probability mass function of a signal. Signal entropy values close to zero may indicate that the signal is periodic and smooth, whereas large negative values may indicate that the signal is irregular and non-periodic;

- Interquartile range (IQR) of auto-covariance of vector magnitude, first principal component, and second principal component - **IQR** of auto-covariance is a measure of long-range dependency or periodicity of a signal and may capture if the signal is periodic or irregular;

- Skewness of vector magnitude, first principal component, and second principal component - skewness is a measure of asymmetry in a signal;

- Dominant frequency value of first principal component and second principal component - Dominant frequency value is the value of the frequency with the highest magnitude in the normalized power spectrum of the accelerometer signal and captures the fundamental frequency of the underlying movement producing the acceleration signal;

- Dominant frequency magnitude of first principal component and second principal component - Dominant frequency magnitude captures the percentage of total signal energy in the dominant frequency;

- Ratio of dominant frequency band to total energy in spectrum of first principal component and second principal component - This feature captures periodicity of a signal by calculating a ratio of the energy in the dominant frequency component to a sum of energy in the entire frequency spectrum of a signal;

- Mean cross rate of vector magnitude, first principal component and second principal component - Mean cross rate calculates the number of times a signal changes from positive to negative and may be normalized by total signal length;

- Jerk ratio of vector magnitude, first principal component and second principal component - Jerk ratio may be calculation of smoothness of motion;

- Log dimensionless jerk of vector magnitude, first principal component, and second principal component - This feature may also be a calculation of smoothness of motion;

- SPARC of vector magnitude, first principal component, and second principal component - This feature may also be a calculation of smoothness of motion;

- Permutation entropy of vector magnitude, first principal component, and second principal component - Permutation entropy is a measure of complexity of a signal;

- Spectral flatness of first principal component and second principal component - Spectral flatness captures the amount of modulation or the level of consistency and may range from 0 to 1; and

- Spectral entropy of first principal component and second principal component - Spectral entropy may be calculated by estimating Shannon entropy of the probability mass function of the power spectrum of a signal, where values close to 1 indicate presence of white noise and values close to 0 indicate presence of periodicity in the signal.

At step 634, principal component analysis (PCA) is utilized to determine feature importance in indicating whether movement is a scratch event or not, and 36 features may be ranked according to their relative importance. **In** one embodiment, data from a random subset of 15 subjects may be selected to analyze feature importance in a scratch classifier. Feature importance may be determined from SHapley Additive exPlanations (SHAP) summary values that order the top 20 features based on their importance for detecting scratch. **In** an example embodiment, it was determined that signal periodicity, smoothness, and dominant frequency may be predominant features of a scratch classifier. Specifically, in one embodiment, a mean cross rate of the second principal component signal may be determined to be the most influential feature for an example classifier. Moreover, higher values of this feature may result in higher SHAP values, which in turn indicates a higher probability that the model would predict scratch for the given window. Measures of smoothness (spectral arc length measure (SPARC)) and dominant frequency may also be influential features to distinguish scratch movements as higher SPARC values (i.e. a smoother signal) and lower dominant frequency values tend to result in a lower probability of scratch prediction by the classifier.

After determining feature importance, feature selection and training of the machine learning model may be done in accordance with leave-one-subject-out (LOSO) validation process, as depicted by block 640. At step 642, observations may be randomly sampled to balance the positive and negative classes prior to feature selection. At step 644, feature selection may be performed utilizing recursive feature elimination with cross-validation (RFECV) using a decision tree estimator. **In** one embodiment, a subset of the following 26 features may be selected during step 644: RMS (vector magnitude); signal entropy (vector magnitude, first principal component, and second principal component); **IQR** of auto-covariance (vector magnitude, first principal component, and second principal component); skewness (first principal component and second principal component); dominant frequency value (first principal component); dominant frequency magnitude (first principal component and second principal component); mean cross rate (second principal component); jerk ratio (vector magnitude and second principal component); log dimensionless jerk (first principal component); SPARC (vector magnitude, first principal component, and second principal component); permutation entropy (vector magnitude, first principal component, and second principal component); spectral flatness (first principal component and second principal component); spectral entropy (second principal component); and signal range (vector magnitude).

FIG. 6B depicts a graphical depiction 6300 of another embodiment of feature engineering block 630. As depicted by graphical representation 6301, principal component analysis (PCA) is utilized to determine features that will most likely be utilized with the classifier to indicate a likely scratch event. Additionally, graphical representation 6351 depicts a ranking of features based on feature importance. A subset 6255 of features may be selected from the highest ranked features according to importance and, consequently, utilized for training and running the classifier. The specific subset 6255 depicted in FIG. 6B is one example subset, but it is contemplated that other subsets, such as the 26 features listed above, may be selected in other embodiments. **In** another embodiment, for instance, the highest ranked features accordance to importance and used for training and running the classifier may be mean cross rate (principal component 2), SPARC (vector magnitude), dominant frequency value (principal component 1), jerk ratio (vector magnitude), jerk ration (principal component 2), log dimensionless jerk (principal component 1), interquartile range (principal component 2), permutation entropy (principal component 2), root mean square (vector magnitude), and SPARC (principal component 2).

Continuing with FIG. 6A, at step 646, the classifier may be trained according to the selected subset of features. For instance, a random forest classifier with 50 estimators may be trained with the above 26 features. As the classifier is trained, validation of the classifier's performance is performed, at step 648. Performance of the binary classifier (i.e., accuracy, sensitivity, specificity, F1 score and area under receiver operating characteristic (ROC) curve) may be assessed using a LOSO validation routine. Additionally, during training, multiple settings for the number of estimators in the random forest classifier may be attempted and evaluated to determine performance effects. **In** one embodiment, the classifier may be trained with 25, 50, 75, or 100 estimators.

Aspects of the performance of an embodiment of a trained model are illustrated in FIGS. 6C-D. FIG. 6C provides a graphical depiction 6400 of performance validation of an exemplary trained scratch detector, in accordance with an embodiment of the present disclosure. **In** FIG. 6C, signal 6410 may be an example tri-axial accelerometer data signal that may be acquired from a monitored user's sensor(s) over time. Signal 6440 may be a hand movement detection signal that, when aligned with signal 6410, indicates parts of the accelerometer data that correspond to detected hand movements. **In** FIG. 6C, the shaded regions within signal 6440 indicate where hand movement was detected. Signal 6450 may be a scratch prediction signal that, when aligned with signal 6410, indicates parts of the accelerometer data that correspond to predicted scratch events. Finally, signal 6405 may be a video reference signal that, when aligned with signal 6410, indicates parts of the accelerometer data that correspond to times in which the video reference data was annotated as a scratch event. **In** this way, signal 6405 acts as a reference, and comparison of signal 6450 with signal 6405 indicates accuracy the scratch prediction model.

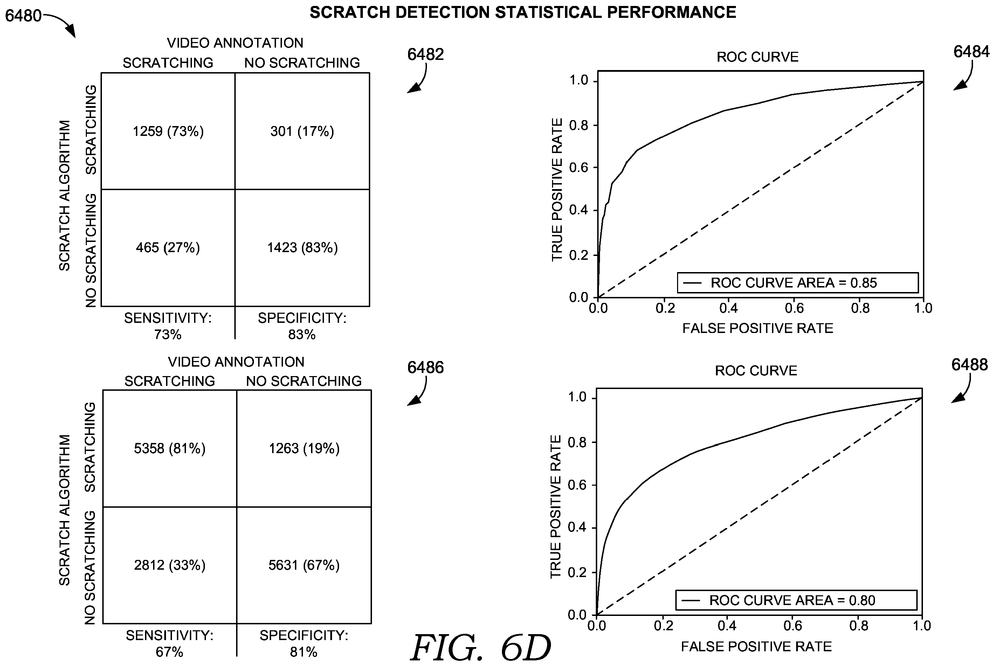

FIG. 6D depicts statistical performances 6480 of example trained scratch detectors, in accordance with some embodiments of the disclosure actually reduced to practice. FIG. 6D includes performance metrics 6482 that show the sensitivity and specificity of a first model (e.g., scratch event classifier) trained to detect scratching and non-scratching periods compared to the annotated video. FIG. 6D further includes performance graphical depiction 6484 in the form of a receiver operating characteristic (ROC) curve for the first trained scratch event classifier. The area under the curve (AUC) for the trained classifier is 0.85, as per FIG. 6D. FIG. 6D also shows performance metrics 6486 and a performance graphical depiction 6488 in the form of an ROC curve for a second model trained to detect scratching. **It** is further contemplated that any model fitting procedure or technique known to those skilled in the art may be utilized for model validation.

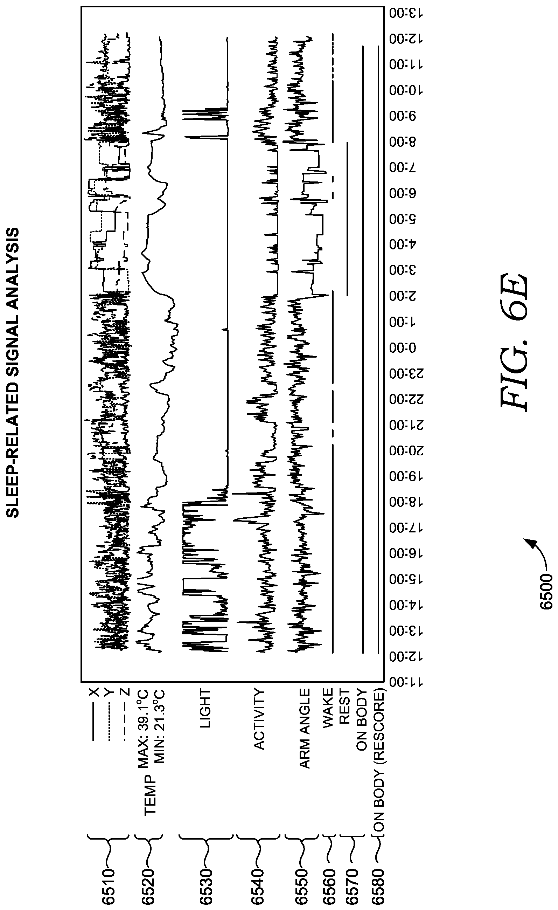

FIG. 6E depicts a time series 6500 of sleep-related signals and signal analysis, in accordance with an example embodiment of sleep/wake detection. Accelerometer signal 6510 is a tri-axial accelerometer data signal, which may be received from a sensor, such as sensor 103 of FIG. 1. Temperature signal 6520 indicates the near-body temperature of a monitored user. On body signal(s) 6580 indicates when a sensor is being worn and may be output by an embodiment of sensor wear determiner 261 of FIG. 2. On body signal(s) 6580 may be derived from accelerometer signal 6510, as described in connection with FIG. 4B. Additionally, or alternatively, temperature signal 6520 may be utilized to generate on body signal(s) 6580, as described in step 420 of FIG. 4A. FIG. 6E depicts two on body signals 6580; "on body" may represent an initial signal and "on body (rescore)" may depict the signal after being rescored, as described in connection with sensor wear determiner 261 of FIG. 2.

Further, in FIG. 6E, light signal 6530 indicates ambient light amounts detected by a sensor. Arm angle signal 6550 indicates a change in arm signal over time and may be derived from accelerometer signal 6510, as described in connection with FIG. 4C. Arm angle signal 6550 may be an output of an embodiment of sleep opportunity determiner 262 of FIG. 2. Rest signal 6570 indicates periods in which it is determined that a user is at rest or intending to sleep. As such, rest signal 6570 may represent the sleep opportunity and may be output by sleep opportunity determiner 262 of FIG. 2. Rest signal 6570 may be derived from arm angle signal 6550, as described in FIG. 4C. **In** other embodiments, rest signal 6570 may be derived from light signal 6530, either alone, or in conjunction with accelerometer signal 6510 or arm angle signal 6550.

Further, in FIG. 6E, activity signal 6540 indicates activity index values and may be derived from accelerometer signal 6510, as described in connection with FIG. 4D. Activity signal 6540 may be an output of an embodiment of activity index determiner 232 of FIG. 2. Further, wake signal 6560 indicates periods during which a user is detected as being awake, which also indicates when the user is asleep corresponding to periods when wake signal 6560 is not observed in FIG. 6E. Wake signal 6560 may be derived from activity signal 6540 and may be output of an embodiment of sleep/wake detector 230 or, more specifically, sleep/wake classifier 234.

FIG. 6F depicts performance validation 6501 of an example sleep opportunity algorithm such as that described in connection with FIG. 4C, and some embodiments of sleep opportunity determiner 262 in FIG. 2 and step 430 in FIG. 4A.

**In** this performance validation 6501, determinations of rest utilizing a total sleep opportunity (TSO) algorithm disclosed herein is compared against determinations of rest utilizing polysomnography (PSG), which is represented as PSG TSO. The PSG determinations represent the base or reference that is compared with the TSO as determined by embodiments of the present disclosure, such as TSO detected by the process 4300 of FIG. 4C.

Graphs 6502 and 6504 indicate performance of the disclosed TSO algorithm determined by sensor data from the left wrist and right wrist, respectively. Graph 6506 shows the agreement between left-wrist and right-wrist based determinations of TSO. Specifically, graph 6506 indicates that the agreement is strong or correlated, which means that the TSO algorithm disclosed herein may be sufficiently accurate for a single-wrist operation - either left-wrist based or right-wrist based (either dominant or non-dominant) detection of TSO. Using this technique, embodiments of the present disclosure may be used more accurately for a single-wrist operation, which represents an improvement over conventional technologies that required a dual-wrist operation. Additionally, because the algorithm for detecting TSO may also be utilized in scratch detection, as described with respect to FIG. 4A, by confirming the accuracy of the TSO through the performance validation shown in the graphs 6502, 6504, and 6506, the disclosed nighttime scratch detection algorithm (e.g., as described in FIGS. 4A and 4E) is more accurate.

FIG. 7A illustratively depicts a graph 7400 of accelerometer signals indicating detected hand movement. The accelerometer signals are separated into three axes signals (x, y, and z) and are similar to accelerometer signal 6510 of FIG. 6E. Vertical bars in graph 7400 (at time instances of approximately 21:53:13.5 and 21:53:19 in FIG. 7A) represent the beginning and end, respectively, of detected hand movement. As such, the vertical bars indicating the hand movement may be an example output of hand movement detector 264 in FIG. 2, from step 440 of FIG. 4A, and/or from step 4040 in FIG. 4E.

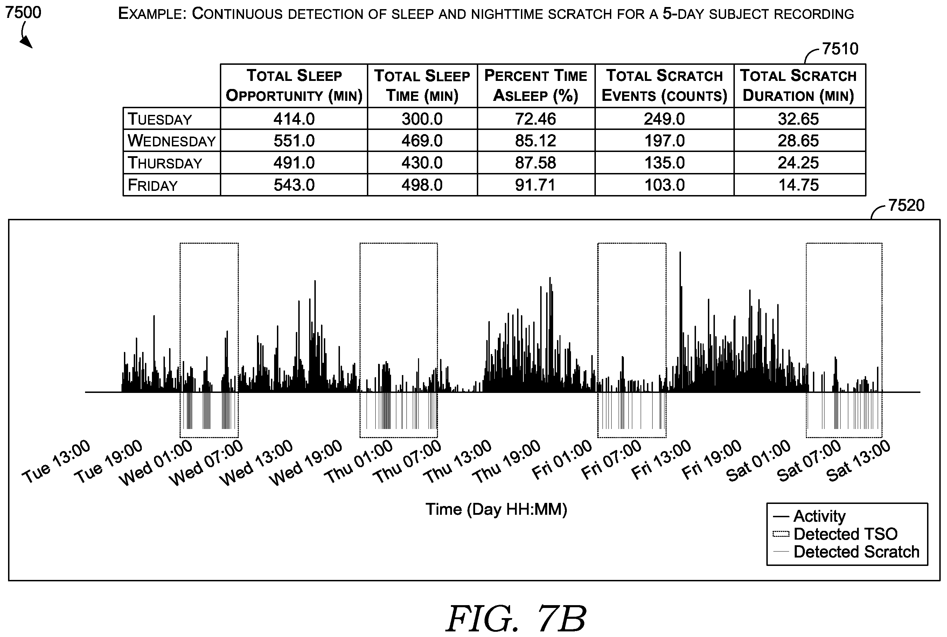

FIG. 7B depicts example results 7500 of scratch detection over five days using example embodiments of the algorithms described in connection with FIGS. 4A, 4C, 4E, and 4D. FIG. 7B includes a summary table 7510 of the detection results, and a graphical representation 7520. The results in FIG. 7B are for nighttime scratch detection over a 5-day recording of sensor data. As described with respect to FIGS. 4A and 4E, the scratch events are detected during a sleep opportunity and thus, represent nighttime scratching. Additionally, sleep/wake periods may be detected by an embodiment of sleep/wake detector 230 in FIG. 2. Summary table 7510 includes digital sleep endpoints as well as scratch endpoints. **In** some embodiments of a scratch monitor application shown in FIG. 8B, the "charts" tab 8230 may include tables, charts or graphs such as summary table 7510 or graph 7520.

FIGS. 8A-8E illustratively depict various example screenshots from a computing device, showing aspects of example graphical user interfaces (GUIs) for a computer software application or app. **In** particular, the example embodiments of GUIs depicted in the screenshots of FIGS. 8A-8E are for a computer decision support application, which in these examples is referred to as the "scratch monitor app." The scratch monitor app 8101 (a computer software application) may include an implementation of decision support app 105a or 105b and/or may include an implementation of one or more decision support tool(s) 270, as described in connection with FIGS. 1 and 2, respectively.

With reference to FIG. 8A, aspects of a GUI 8100 are illustratively provided, showing an example embodiment of a computer software application 8101 (sometimes referred to herein as the "scratch monitor app") for providing decision support for users having atopic dermatitis, pruritus, or similar condition. Example computer software application 8101 may be operating on (and GUI 8100 may be displayed on) a user computing device 8102a, which may be embodied as a user device 102a-102n, described in connection with FIG. 1. At a high level, the example scratch monitor app 8101 may be used for, among other purposes, accessing, viewing, tracking, supplementing, and/or reporting the scratch-detection and/or sleep-related data for a user that is detected by the embodiments of the technologies described herein. Some embodiments of scratch monitor app 8101 may further or alternatively provide functionality related to flare prediction and itch prediction.

**In** some embodiments, it is contemplated that a prescribed or recommended standard of care for a patient diagnosed with atopic dermatitis (or similar condition) may comprise utilizing an embodiment of the scratch monitor app 8101, which may operate on the user/patient's own computing device, such as a smartwatch, a mobile device, or other user device 102a-102n, or may be provided to the user/patient via the patient's healthcare provider or pharmacy.

**In** particular, as described herein, conventional solutions for monitoring and tracking user scratching, such as requiring users to monitor and report scratching, may suffer from being subjective and non-uniform, less accurate, inconsistently captured, and other deficiencies. However, embodiments of the technologies described herein may provide objective and/or uniform, consistent, and more accurate means of monitoring, detecting, and tracking scratch (and sleep) related data for a user. As a result, these embodiments thereby enable reliable use of these technologies for patients who are prescribed certain medicines. **In** this way, a doctor or a healthcare provider may issue an order that includes a patient taking a medicine and using a computer decision support app (e.g., scratch monitor app 8101) to, among other things, track and determine precise efficacy of the prescribed treatment. Moreover, the use of the computer decision support app (e.g., scratch monitor app 8101), as part of the standard of care for a patient who is administered or prescribed a particular medicine, supports the effective treatment of the patient. The effective treatment, in some embodiments, is achieved by enabling the healthcare provider to better understand the efficacy of a prescribed medicine, modify a dosage, change a particular prescribed medicine, or instruct the patient to cease using it because it is no longer needed due to the patient's condition having improved.

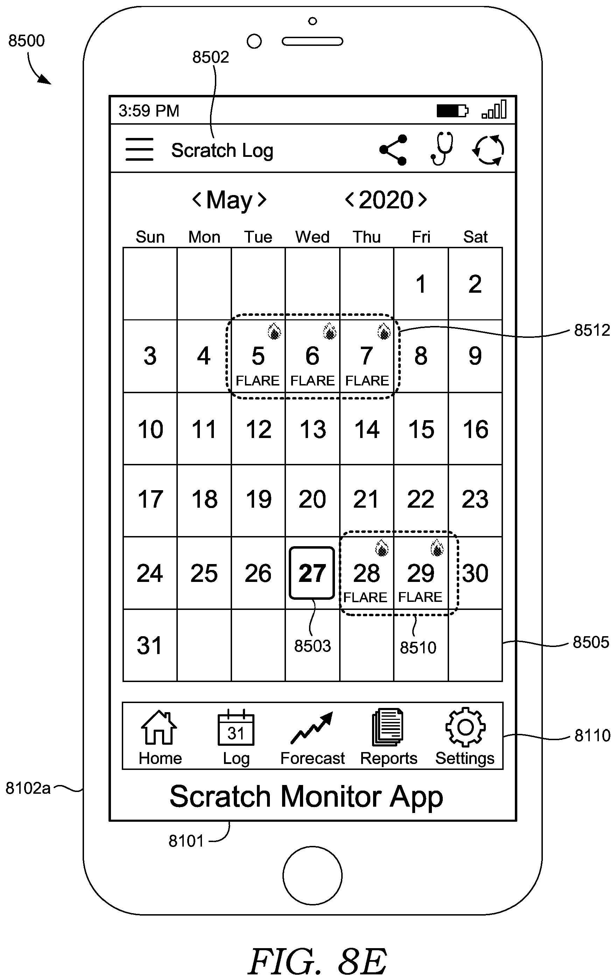

Further, continuing with FIG. 8A, the scratch monitor app 8101 depicted in GUI 8100 includes an icon menu 8110 comprising various user-selectable icons 8111, 8112, 8113, 8114, and 8115, which correspond to various additional functionalities provided by the scratch monitor app 8101. **In** particular, selecting these icons may navigate a user to various services or tools provided via the scratch monitor app 8101. By way of example and without limitation, home icon 8111 may navigate the user to a home screen, which may include a calendar view 8105 depicted in GUI 8100, one of the example GUIs described in connection with FIGS. 8B, 8C, 8D, or 8E, a welcome screen (not shown), which may include one or more commonly utilized services or tools provided by scratch monitor app 8101, or any other view (not shown).

Selecting log icon 8112 can navigate the user to a scratch log tool (which may be indicated by a descriptor for a scratch log 8201) that comprises functionality to facilitate scratch or sleep related detection, tracking, and/or monitoring. **In** an embodiment, scratch log 8201 comprises calendar view 8105 or an alternative calendar view 8505 depicted in FIG. 8E. Functionality associated with scratch log 8201 or log icon 8112 may also include a GUI and tools or services for daily tracking and monitoring, such as that described in connection with FIG. 8B. Selecting forecast icon 8113 can navigate the user to a scratch forecast or an itch forecast related GUI that may include one or more tools and services related to itch prediction. Additional details of the forecast functionality associated with forecast icon 8113 are described in connection with FIG. 8C. Selecting reports icon 8114 can navigate the user to a GUI for viewing and generating various reports of the scratch detection and/or sleep related data detected by the embodiments described herein. Selecting settings icon 8115 may navigate the user to a user-setting configuration mode that can enable specifying various user preferences, settings, or configurations of scratch monitor app 8101, aspects of the sleep and scratch related detection, user care/treatment, or other settings. **In** some embodiments, at least a portion of settings may be configured by the user's healthcare provider or a clinician. Some settings accessible via settings icon 8115 may include settings discussed in connection with settings 249 of FIG. 2.

The example scratch monitor app 8101 depicted in GUI 8100 includes a header region 8109 located near the top of GUI 8100. In particular, this example header region 8109 includes a hamburger icon 8103, descriptor 8201 showing "Scratch Log", a share icon 8104, a stethoscope icon 8106, and a cycle icon 8108. Selecting hamburger icon 8103 may provide the user access to a menu of other services, features, or functionalities of scratch monitor app 8101, and may further include access to help, app version information, and access to secure user-account sign-in/sign-off functionality. Descriptor 8201 showing "Scratch Log" indicates to the user a mode, a feature set or an aspect of scratch monitor app 8101 to which the user has navigated. Here the descriptor 8201 indicates that the user is in the scratch log functionality of scratch monitor app 8101, which may have been accessed by selecting the log icon 8112. Share icon 8104 may be selected for sharing various data, reports, user-provided annotations or observations (e.g., notes or photos). For example, share icon 8104 may facilitate enabling the user to email a report of recent nights' scratch events to a caregiver of the user. In some embodiments, share icon 8104 may facilitate sharing aspects of the various data captured, displayed, or accessed via scratch monitor app 8101 on social media or with other similar users. Selecting stethoscope icon 8106 can provide the user with various communication or connection options to the user's healthcare provider. For example, selecting stethoscope icon 8106 may initiate functionality to facilitate scheduling a tele-appointment, sharing or uploading data to a medical record (e.g., profile/health data (EHR) 241) of the user for access by the user's healthcare provider, or accessing a healthcare provider's online portal for additional services. In some embodiments, selecting stethoscope icon 8106 may initiate functionality for the user to communicate specific data, such as the data that the user is currently viewing, to the user's healthcare provider, or may ping the user's healthcare provider to request them to look at the user's data. Finally, selecting cycle icon 8108 may cause a refresh or update to the views and/or data displayed via scratch monitor app 8101 so that the view is current with regards to the available data. In some embodiments, selecting cycle icon 8108 may refresh data pulled from a sensor (or from a computer application associated with data collection from a sensor, such as sensor 103 in FIG. 1) and/or from a cloud data store (e.g., an online data account) associated with the user.

Scratch monitor app 8101 depicted in GUI 8100 may also include calendar view 8105. Embodiments of calendar view 8105 can facilitate accessing or displaying the detected and interpreted sleep and/or scratch related data for the user. For example, by selecting a particular date of the calendar view 8105, the user may be presented with a daily (or nightly) summary of the data for that date, such as provided by a GUI 8200, described in connection with FIG. 8B. In some embodiments of calendar view 8105, indicators or information may be displayed on dates of the calendar, indicating scratch-related or sleep-related information associated with that date. For example, an alternative calendar view 8505 described in FIG. 8E depicts flame indicators on dates associated with a flare.

Turning now to FIG. 8B, another aspect of scratch monitor app 8101 is depicted including GUI 8200. GUI 8200 includes user interface (UI) elements for displaying or receiving scratch-related or sleep-related data, and corresponds to the log functionality indicated by log icon 8112. In particular, GUI 8200 depicts an example of a nightly summary 8202 of data for the user, and may be an example of information that is displayed to user upon selecting a particular calendar date from calendar view 8105 (FIG. 8A), or information that is presented to the user upon selecting the log icon 8112 from menu 8110. GUI 8200 includes a descriptor 8203 indicating that the nightly summary 8202 is for the date Sunday, January 12.

As shown in this example GUI 8200 of scratch monitor app 8101, the log functionality includes five selectable tabs: scores 8210, charts 8230, photo 8240, notes 8250, and treatment 8260. As per GUI 8200, as shown in FIG. 8B, the tab for scores 8210 is selected, and thus, various scores and metrics are presented to the user. In particular, scores 8210 may comprise a scratching score 8212, a sleep score 8216, and a visual summary 8218 (of detected user activity and scratch events, as shown in FIG. 8B) corresponding to user data detected overnight on Sunday, January 12. In some embodiments, the scores may be presented as numbers, categories, colors, or a combination of these features. For example, here the scratching score 8212 is "36" and may be colored green to indicate that it is a desirable score for the user. Sleep score 8216 is presented as a category "Very Good" but could alternatively be presented as a number or a color.

In some embodiments, scratching score 8212 may be displayed with various scratch-related analytics data 8213. By way of example and without limitation, data 8213 may include: a scratch trend, which indicates whether the user's scratching is increasing, decreasing, or remaining unchanged over recent nights (e.g., past 3 nights, 5 nights, or a week); a number of nightly or daily scratch events detected (e.g., 12 scratch events); total scratch time, which represents a cumulative total of the time of detected overnight scratch events (e.g., 84 seconds); the average duration of the detected scratch events (e.g., 7 seconds); and the duration of the longest detected scratch (e.g., 12 seconds). Similarly sleep score 8216 may be displayed with various sleep-related analytics data 8217. By way of example and without limitation, data 8217 may include: a sleep percentage, which represents a ratio of the user's detected sleep time over their sleep opportunity (e.g., TSO) time interval (here shown as 86%); total sleep time (TST), sleep onset latency (SOL, measured in minutes); wake after sleep onset (WASO, measured in minutes); and a number of wake bouts (NWB). Other sleep-related metrics may also be presented, and in some embodiments, a user may customize information that is displayed including scores, metrics, and visual summary 8218, by configuring the settings (e.g., via settings icon 8115). Similarly, in some embodiments, other related data such as temperature or humidity data may be displayed alongside the score(s).

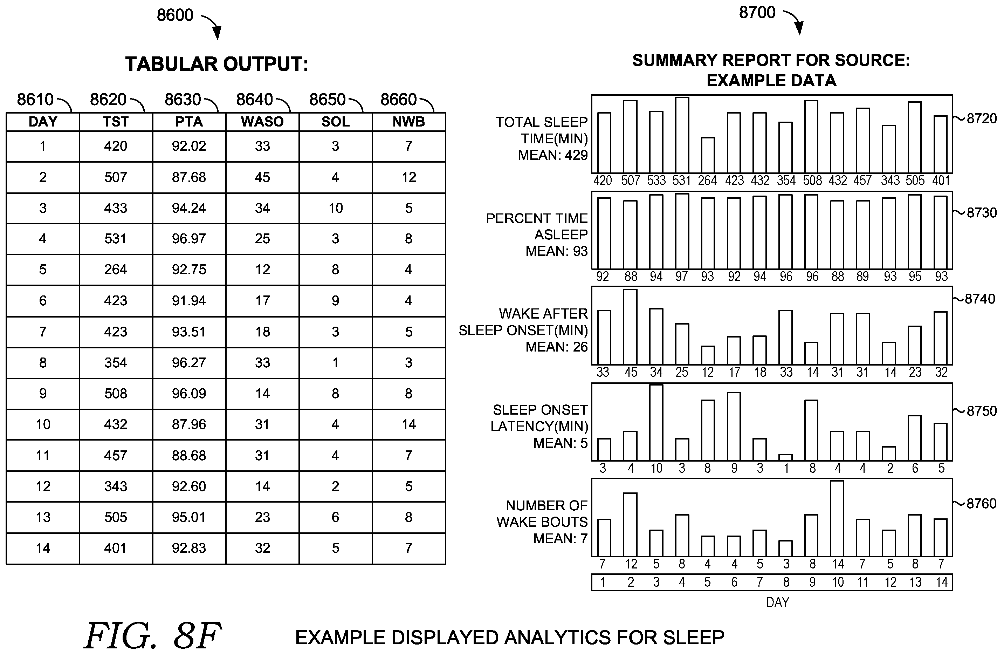

Continuing with GUI 8200 shown in FIG. 8B, the tab for charts 8230 may be selected by the user to create or display various charts, graphs, or interpreted scratch-related or sleep-related data (e.g., summaries and trends analyses) for the user. Examples of the charts that may be presented via charts 8230 is depicted in FIG. 8F, which shows various analytics data for sleep as a table 8600 and charts 8700. Another example of charts that may be created or displayed via charts 8230 is provided in FIG. 7B.

Continuing with FIG. 8B, selecting tabs for photo 8240 and notes 8250 can navigate the user to functionality for scratch monitor app 8101 (or, more specifically, log functionality associated with log icon 8112) for receiving and displaying observational data from a user or a caregiver for that particular date. Examples of observational data may include notes and/or photos documenting or relating to the user's scratching or sleep. In some embodiments, notes 8250 include a UI for receiving text (or audio or video recordings) from the user. In some aspects, UI functionality for notes 8250 may comprise a GUI showing a human body configured to receive input from the user indicating areas of the user's body affected by itching or scratching. In addition, or alternatively, some embodiments of notes 8250 may include UI input functionality for the user to specify a subjective rating of the itching or scratching they experienced over the nightly time interval.

In some embodiments, the users may enter other contextual information, such as their location, weather, and any physical activity that they engaged in during the day, for example, into notes 8250. In some instances, data such as user location and weather may be determined automatically, such as by using location sensors on the user computing device 8102a and looking up the weather information for the user device location. In some embodiments, as described in connection with contextual data determiner 294 (FIG. 2), these user-provided observations may be analyzed for contextual information that then may be utilized for generating forecasts or decision support information for the user.

In some embodiments, photo 8240 can comprise a UI for receiving photo(s) or video(s) from the user. Photo 8240 may also comprise functionality for snapping photos or videos on the user computing device 8102a on which scratch monitor app 8101 operates. For example, for a given day, the user may select notes 8250 to add a note indicating the user did not sleep well and scratched all night. The user also, or alternatively, may snap a photo on user computing device 8102a to be logged for this data, after selecting the tab for photo 8240. The photo may be of a lesion or an otherwise-affected area of the user's skin.

Selecting the tab indicating treatment 8260 on GUI 8200 may navigate the user to a UI within scratch monitor app 8101 with functionality for the user to specify details such as whether the user applied (or took) treatment for that date. For example, the user may specify that their prescription topical medication is applied on the affected area of the user's body. It is also contemplated that, in some embodiments, smart pillboxes or smart containers, which may include so-called internet-of-things (IoT) functionality, may automatically detect that a user has accessed medicine stored within a container and may communicate an indication to scratch monitor app 8101 indicating that the user has applied treatment on that date. In some embodiments, the tab for treatment 8260 may comprise a UI, enabling the user to specify their treatment, for instance, by selecting check-boxes indicating the kind of treatment the user followed on that date (e.g., applied OC lotion, took a bath, avoided exposure to sun, applied topical (or ingested oral) prescription medication, and so on).

Turning now to FIG. 8C, another aspect of example scratch monitor app 8101 is depicted including a GUI 8300. GUI 8300 includes various UI elements for displaying itch forecast(s) and related information for the user. As described herein, some embodiments may determine an itch forecast representing a user's expected itching (or scratching) to occur at a future time or over a future time interval (for example, as described in connection with FIG. 5 and flare predictor 290 of FIG. 2). As further described herein, the itch forecast may be personalized to a user and may be based on the user's historical scratch pattern. In some instances, the itch forecast further may be based on contextual data, such as weather, user observations, health or physiological data, or other contexts. Alternatively, according to other embodiments described herein, the itch forecast may be determined according to predetermined rules or conditions, and thus not personalized to a particular user. Alternatively, according to other embodiments, the itch forecast may be determined based on data of other users who are similar to the particular user of scratch monitor app 8101. Additional details of determining the itch forecast are provided in connection with flare predictor 290 of FIG. 2 and method 500 of FIG. 5.

Example GUI 8300 includes a descriptor 8303 indicating a current date the user is accessing the forecast functionality of scratch monitor app 8101 (e.g., Today, Tuesday, March 17, 2020) and user's itch forecast 8301. As shown in FIG. 8C, menu 8110 indicates that the forecast icon 8113 is selected, which may present the user with GUI 8300 depicting the user's itch forecast 8301. The example itch forecast 8301 depicted in GUI 8300 may comprise information that predicts the user's itch or scratching for one or more future intervals of time. In particular, in the example of GUI 8300, itch forecast 8301 includes a daily (or nightly) itch forecast 8310 for the next 3 days. As shown in FIG. 8C, low itching is shown for Wednesday, March 18, moderate itching for Thursday, March 19, and high itching forecasted for Friday, March 20. Example itch forecast 8301 may further include an itch forecast trend 8320, indicating a trend for the user's itching (or scratching) in the near future. As shown in this example, the user's itch forecast trend 8320 is increasing, which is consistent with the daily (or nightly) itch forecast 8310.

In some embodiments, and in the example embodiment depicted in GUI 8300, itch forecast 8301 further includes a user recommendation 8330. Here, the recommendation advises the user to "use your topical treatment every day, as directed." User recommendation 8330 may include recommendations and/or directives for treating pruritus using one or more therapeutic agents, such as the agents discussed with respect to decision support tool 276. **In** some instances, the user may select or click on user recommendation 8330 to view the recommendation or additional details about the recommendation. The recommendation displayed or accessed via user recommendation 8330 may correspond to the specific itch forecast for the user and/or information available of the user's behavior or treatment regimen. This information may be provided by the user, the user's caregiver or a healthcare provider, or received as observational or treatment-related data, such as described in connection with FIG. 8B. In some embodiments, the recommendation may be determined using rules, conditions, and/or input received from the user's healthcare provider.

In some embodiments and in the example embodiment depicted in GUI 8300, itch forecast 8301 further includes a viewing functionality 8340 for viewing alternative forecasts (with or without treatment). For example, by selecting a treatment button 8341, daily itch forecast 8310 may be determined and presented to the user based on the user using treatment over the future time interval. Similarly, by selecting a no-treatment button 8343, daily itch forecast 8310 may be determined and presented to the user based on the user not using treatment over the future time interval. In particular, the user's treatment may be determined as part of contextual data (such as by contextual data determiner 294, discussed in connection with system 200 of FIG. 2) from the user's profile/health data (EHR) 241, information entered via treatment tab 8260, or specified by the user or a caregiver/healthcare provider (e.g., in the settings 8110). Different and alternative forecasts may be determined based on historical data of the user, where the user did or did not use treatment, based on similar users using or not using the treatment, or may be first computed based on available data, and then modified accordingly. For example, an itch forecast for a user, with or without treatment, may be determined and then modified such as by scaling a predicted number of scratch events up or down by a multiplier (e.g., up to a forty percent increase in scratch events, within 48 hours, if the user stops using treatment). In this way, viewing functionality 8340 may determine and present alternative itch forecasts for the user.

Turning now to FIG. 8D, another aspect of example scratch monitor app 8101 is depicted including a GUI 8400. GUI 8400 includes various UI elements for displaying a flare alert notification (e.g., a flare notification 8401). In some instances, related information is displayed additionally or alternatively. As described herein, flare notification 8401 may indicate to the user that predicted future itch (or scratch events) for the user is likely to surpass a threshold so as to become a flare. As described above in connection with method 500 of FIG. 5 and flare predictor 290 of FIG. 2, some embodiments may determine a future likelihood of a flare event for the user.

Example GUI 8400 includes a descriptor 8403 indicating the current date (e.g., Today, Monday May 4) and flare notification 8401 alerting the user for a likely future flare event. In the example embodiment depicted in GUI 8400, additional information may be presented in addition to flare notification 8401, such as a recommendation (not shown, e.g., avoid exposure to sunlight) and/or flare notification details 8410. In particular, in this example, flare notification details 8410 indicate when the flare is likely to occur (e.g., the future time interval between the next day and Thursday), the likelihood of the flare event occurring (e.g., 74% likelihood, which may be determined as described in connection to flare predictor 290 of FIG. 2 or method 500 of FIG. 5), and/or the severity or level of the flare (e.g., "severe"). In some instances, although the severity may vary within the future time interval, the severity may be the highest possible severity predicted for the user, to enable the user to prepare for the worst possible outcome.

In the example embodiment depicted in GUI 8400, flare notification 8401 further includes one or more response options 8420 to facilitate a user's response to the flare notification. For example, response options 8420 may include an option 8422 to check/refill the user's prescription, an option 8424 to schedule a tele-appointment (or in-person appointment) with the user's healthcare provider;, or an option 8426 to automatically add an over-the-counter (OC) therapy (e.g., cortisone cream, calamine lotion, etc.) to the user's electronic shopping list. In embodiments where purchasing or store-account information is specified in user account(s)/device(s), selecting option 8426 may automatically purchase the item for the user and deliver it to the user's address or make it available for pickup. In some embodiments, the particular OC therapy may be specified by the user or healthcare provider. For example, OC therapy may be defined via treatment tab 8260, settings 8115, user's profile/health data (EHR) 241 (FIG. 2), or based on past user purchases, which may be determined as contextual data from contextual data determiner 294 and/or from user accounts/devices 248, such as purchase history or email/electronic receipts of the user. As shown in FIG. 8D, an option 8428 may snooze flare notification 8401 for a period of time or initiate a functionality to remind the user of the flare at a future time. In some embodiments, flare notification 8401 is provided to the user in the morning and/or evening to increase the likelihood that a user can take actions to mitigate the flare event (e.g., put on treatment at night, or schedule time to go to the pharmacy or discuss with healthcare provider during that day).

Upon selecting response option 8424, for scheduling a tele-appointment, it is contemplated that in many instances, a user may not have time to schedule a physical (in-person) appointment after receiving flare notification 8401 before the flare event happens. Therefore, a tele-appointment, which may include initiating video conference with user's healthcare provider using a camera on user computing device 8102a, provides a more timely solution for the user. Some embodiments of flare prediction, however, may forecast flares weeks in advance, and hence, physical appointment can be done as an alternate solution.

Turning now to FIG. 8E, another aspect of example scratch monitor app 8101 is depicted including a GUI 8500. GUI 8500 depicts an aspect of a scratch log 8502, which may be an additional or alternative depiction of scratch log 8201 of GUI 8100 (described in FIG. 8A). In particular, GUI 8500 includes a calendar view 8505 that depicts a current date indicator 8503 (indicating the current date as May 27). Calendar view 8505 also includes indicators of flare events, including indicators of future flare events 8510 (shown as occurring on May 28 and May 29) and an indicator of past flare events 8512 (shown as having occurred on May 5 to May 7). Some embodiments (not shown) of calendar view 8505 or GUI 8500 may also depict past or historical events based on whether the user would have used or not used treatment, similar to the alternative views of the itch forecast described in conjunction with FIG. 8C. For example, an alternative view based on when the user is not using the treatment may show additional flares that the user is likely to have experienced without treatment.

FIG. 8F depicts example analytics for sleep detection, which includes digital sleep endpoints, that may be presented to the user, via chart tab 8230, as described in connection with FIG. 8B. Specifically, the example analytics includes example table 8600 including sleep-related data for a series of days 8610, including total sleep time (TST) 8620, percent time asleep (PTA) 8630, wake after sleep onset (WASO) 8640, sleep onset latency (SOL) 8650, and number of wake bouts (NWB) 8660. The example analytics in FIG. 8F further include a set of charts 8700. Charts 8700 include graphical depictions of the sleep-related data shown in table 8600 for each day from the list of days 8610. As shown in FIG. 8F, charts 8700 depict total sleep time (TST) 8720, percent time asleep (PTA) 8730, wake after sleep onset (WASO) 8740, sleep onset latency (SOL) 8750, and number of wake bouts (NWB) 8760. Table 8600 and charts 8700 may be presented to a user one at a time or simultaneously.

FIGS. 9A-11M depict example embodiments of computer program routines for detecting scratch-related and sleep-related data for the user, as described herein. In particular, FIGS. 9A-9I depict aspects of an example computer program for controlling sleep-detection related and scratch-detection related routines. As such, computer program routine in FIGS. 9A-9I may be utilized to perform method 400 of FIG. 4A, method 4800 of FIG. 4D, and/or method 4001 of FIG. 4E. FIGS. 10A-10I depict aspects of an example computer program for detecting scratch events and related information, which may be utilized to perform method 400 of FIG. 4A and/or method 4001 of FIG. 4E. FIGS. 11A-11M depict aspects of an example computer program for detecting sleep-related information, including total sleep opportunity (TSO), which may be utilized in performing some embodiments of step 430 in FIG. 4A, method 4300 of FIG. 4C, and/or method 4800 of FIG. 4D.

Accordingly, various aspects of technology directed to systems and methods for detecting scratch and predicting flares are provided. It is understood that various features, sub-combinations, and modifications of the embodiments described herein are of utility and may be employed in other embodiments without reference to other features or sub-combinations. Moreover, the order and sequences of steps shown in the example methods or process are not meant to limit the scope of the present disclosure in any way, and in fact, the steps may occur in a variety of different sequences within embodiments hereof. Such variations and combinations thereof are also contemplated to be within the scope of embodiments of this disclosure.

Having described various implementations, an exemplary computing environment suitable for implementing embodiments of the disclosure is now described. With reference to FIG. 12, an exemplary computing device is provided and referred to generally as a computing device 1200. The computing device 1200 is one example of a suitable computing environment and is not intended to suggest any limitation as to the scope of use or functionality of embodiments of the disclosure. Neither should the computing device 1200 be interpreted as having any dependency or requirement relating to any one or combination of components illustrated.

Embodiments of the disclosure may be described in the general context of computer code or machine-useable instructions, including computer-useable or computer-executable instructions, such as program modules, being executed by a computer or other machine, such as a personal data assistant, a smartphone, a tablet PC, or other handheld or wearable device, such as a smart watch. Generally, program modules, including routines, programs, objects, components, data structures, and the like, refer to code that performs particular tasks or implements particular abstract data types. Embodiments of the disclosure may be practiced in a variety of system configurations, including handheld devices, consumer electronics, general-purpose computers, or specialty computing devices. Embodiments of the disclosure may also be practiced in distributed computing environments, where tasks are performed by remote-processing devices that are linked through a communications network. In a distributed computing environment, program modules may be located in both local and remote computer storage media including memory storage devices.

With reference to FIG. 12, computing device 1200 includes a bus 1210 that directly or indirectly couples various devices including a memory 1212, one or more processor(s) 1214, one or more presentation component(s) 1216, one or more input/output (I/O) port(s) 1218, one or more I/O components 1220, and an illustrative power supply 1222. Some embodiments of computing device 1200 may further include one or more radios 1224. Bus 1210 represents one or more busses (such as an address bus, a data bus, or a combination thereof). Although various blocks of FIG. 12 are shown with lines for the sake of clarity, in reality, these blocks represent logical, not necessarily actual, components. For example, one may consider a presentation component such as a display device to be an I/O component. Also, a processor may have a memory. FIG. 12 is merely illustrative of an exemplary computing device that can be used in connection with one or more embodiments of the present disclosure. Distinction is not made between such categories as "workstation," "server," "laptop," or "handheld device," as all are contemplated within the scope of FIG. 12 and with reference to "computing device."

Computing device 1200 typically includes a variety of computer-readable media. Computer-readable media can be any available media that can be accessed by computing device 1200 and includes both volatile and nonvolatile, and removable and non-removable media. By way of example, and not limitation, computer-readable media may comprise computer storage media and communication media. Computer storage media includes both volatile and nonvolatile, removable and non-removable media implemented in any method or technology for storage of information such as computer-readable instructions, data structures, program modules, or other data. Computer storage media includes, but is not limited to, Random-access memory (RAM), Read-only memory (ROM), electrically erasable programmable read-only memory (EEPROM), flash memory or other memory technology, Compact Disc Read-Only Memory (CD-ROM), digital versatile disks (DVDs) or other optical disk storage, magnetic cassettes, magnetic tape, magnetic disk storage or other magnetic storage devices, or any other medium, which can be used to store the desired information and can be accessed by computing device 1200. Computer storage media does not comprise signals per se. Communication media typically embodies computer-readable instructions, data structures, program modules, or other data in a modulated data signal such as a carrier wave or other transport mechanism and includes any information delivery media. The term "modulated data signal" means a signal that has one or more of its characteristics set or changed in such a manner as to encode information in the signal. By way of example, and not limitation, communication media includes wired media, such as a wired network or a direct-wired connection, and wireless media, such as acoustic, radio frequency (RF), infrared, and other wireless media. Combinations of any of the above should also be included within the scope of computer-readable media.

Memory 1212 includes computer storage media in the form of volatile and/or nonvolatile memory. The memory may be removable, non-removable, or a combination thereof. Exemplary hardware devices include for example solid-state memory, hard drives, and optical-disc drives. Computing device 1200 includes one or more processor(s) 1214 that reads data from various devices such as memory 1212 or I/O components 1220. Presentation component(s) 1216 presents data indications to a user or other device. Exemplary presentation component(s) 1216 may include a display device, a speaker, a printing component, a vibrating component, and the like.

The I/O port(s) 1218 allow computing device 1200 to be logically coupled to other devices, including I/O components 1220, some of which may be built in. Illustrative components include a microphone, a joystick, a game pad, a satellite dish, a scanner, a printer, or a wireless device. The I/O components 1220 may provide a natural user interface (NUI) that processes air gestures, voice, or other physiological inputs generated by a user. In some instances, inputs may be transmitted to an appropriate network element for further processing. An NUI may implement any combination of speech recognition, touch and stylus recognition, facial recognition, biometric recognition, gesture recognition (both on screen and adjacent to the screen), air gestures, head and eye tracking, and touch recognition associated with displays on the computing device 1200. The computing device 1200 may be equipped with depth cameras, such as stereoscopic camera systems, infrared camera systems, RGB camera systems, and combinations of these, for gesture detection and recognition. Additionally, the computing device 1200 may be equipped with accelerometers or gyroscopes that enable detection of motion. The output of the accelerometers or gyroscopes may be provided to the display of the computing device 1200 to render immersive augmented reality or virtual reality.

Some embodiments of computing device 1200 may include one or more radio(s) 1224 (or similar wireless communication components). The radio(s) 1224 transmits and receives radio or wireless communications. The computing device 1200 may be a wireless terminal adapted to receive communications and media over various wireless networks. Computing device 1200 may communicate via wireless protocols, such as code division multiple access ("CDMA"), global system for mobiles ("GSM"), time division multiple access ("TDMA"), or other wireless means, to communicate with other devices. The radio communications may be a short-range connection, a long-range connection, or a combination of both. Herein, "short" and "long" types of connections do not refer to the spatial relation between two devices. Instead, these connection types are generally referring to short range and long range as different categories, or types, of connections (i.e., a primary connection and a secondary connection). A short-range connection may include, by way of example and not limitation, a Wi-Fi® connection to a device (e.g., mobile hotspot) that provides access to a wireless communications network, such as a Wireless Local Area Network (WLAN) connection using the 802.11 protocol; a Bluetooth connection to another computing device is another example of a short-range connection; or a near-field communication. A long-range connection may include a connection using, by way of example and not limitation, one or more of CDMA, General Packet Radio Service (GPRS), GSM, TDMA, and 802.16 protocols.

The following embodiments represent example aspects of concepts contemplated by the disclosure hereinn. Any one of the following embodiments may be combined in a multiple dependent manner to depend from one or more other embodiments. Further, any combination of embodiments hat explicitly depend from a previous embodiment may be combined while staying within the scope of aspects contemplated herein. The following embodiments are illustrative in nature and are not limiting.

In some embodiments, a system for providing decision support based on scratch events, such as the systems described in any of embodiments disclosed herein, comprises: a processor; and a computer memory having computer executable instructions stored thereon for performing operations when executed by the processor. The operations comprise: receiving accelerometer data for an individual; detecting a hand movement utilizing the accelerometer data; utilizing a computerized classification model to determine, based on the accelerometer data corresponding to the hand movement, that the hand movement indicates a scratch event; and initiating one or more response actions based at least on a determination that the hand movement indicates the scratch event. Among other benefits, these embodiments may provide an assessment of pruritus with greater accuracy and reliability (as compared to conventional solutions) based on accelerometer data acquired in a way to reduce burden on the user and increase user compliance. Using computerized classification models with the accelerometer data to detect scratch events helps remove bias and subjectivity, further improving accuracy and reliability. These classifiers help to provide reliable computer decision support tools that are based on detected scratch data, thereby improving recommendations for treatment and/or responses to scratching.

In the above embodiment of the system, the operations performed by the processor executing the computer executable instructions further comprise: generating a multidimensional timeseries from the accelerometer data corresponding to the hand movement; and determining a plurality of feature values from the multidimensional timeseries. The plurality of feature values include at least one time-domain feature value and at least one frequency-domain feature value. The determination that the hand movement is the scratch event is based on the plurality of feature values.

In any combination of the above embodiments of the system, the accelerometer data is captured by a wearable device located at an appendage of the individual. For example, the wearable device may be located on a wrist, finger, and/or arm. Using wearable device may enable continuous (or semi-continuous, periodic, as-needed, or as-it-becomes-available) data capturing that is less intrusive than other types of monitoring, which may be beneficial in monitoring individuals in populations with typically lower compliance rates, such as children.

In any combination of the above embodiments of the system, the operations performed by the processor executing the computer executable instructions further comprise determining a total sleep opportunity based on the accelerometer data. The total sleep opportunity comprises a period of time between when the individual lays down for a rest and when the individual gets up from the rest. The hand movement is detected utilizing accelerometer data corresponding to the total sleep opportunity. In this way, the scratch event detected may be considered nighttime scratching or scratching during a period in which the individual intends to sleep. This detection helps track scratching during peak pruritus time or even when an individual is unaware of the scratching. As such, scratch events detected, in accordance with embodiments of this disclosure, may provide more accurate measures of the individual's current condition (e.g., pruritus and atopic dermatitis).

In any combination of the above embodiments of the system, the accelerometer data is captured by a wearable device having a plurality of sensors, wherein the wearable device further captures at least one of near-body temperature data and light data. The total sleep opportunity is determined further based on the at least one of near-body temperature data and light data.

In any combination of the above embodiments of the system, the computerized classification model utilized to determine that the hand movement indicates the scratch event comprises at least one of an ensemble of machine learning models and a random forest classifier. For example, the computerized classification model may be an ensemble of machine learning models in which at least one model is a random forest classifier. Compared to other scratch detection approaches these embodiments yield results that are more interpretable, when compared to the recurrent neural network approaches, and, therefore, better capable of being modified or refined for particular contexts. Additionally, these may be quicker and less computationally burdensome than other approaches.

In any combination of the above embodiments of the system, the one or more response actions comprises generating a graphic user interface element provided for display on a user device. The graphic user interface element includes at least one of an indicator of one or more scratch endpoints comprising a total number of scratch events and a total scratch duration; and an indicator recommending that the individual seek clinical consultation based on the determination that the hand movement indicates the scratch event. Scratch endpoints may represent novel digital endpoints that are useful in quantitatively and objectively measuring pruritus or, more specifically, atopic dermatitis. Further, generating the graphic user interface element to provide for display on a user device, either with the scratch endpoint indicator(s) and/or the recommendation for clinical consultation promotes better treatment compliance for the individual being monitored and enables clinician's to make informed decisions with respect to treatment.

In any combination of the above embodiments of the system, the total number of scratch events and the total scratch duration are each determined for a total sleep opportunity that is determined based on the accelerometer data received for the individual. The total sleep opportunity comprises a period of time between when the individual lays down for a rest and when the individual gets up from the rest. In this way, the scratch event detected may be considered nighttime scratching or scratching during a period in which the individual intends to sleep. This detection helps track scratching during peak pruritus time or even when an individual is unaware of the scratching. As such, scratch events detected, in accordance with embodiments of this disclosure, may provide more accurate measures of the individual's current condition (e.g., pruritus and atopic dermatitis).

In some embodiments, a method for treating pruritus utilizing a motion sensing device associated with a subject is provided. The subject may comprise a human subject for which treatment of pruritus is sought. The method may comprise: receiving accelerometer data collected from the motion sensing device; detecting a hand movement utilizing the accelerometer data; utilizing a computerized classification model to determine, based on the accelerometer data corresponding to the hand movement, that the hand movement indicates a scratch event; and, based on at least a first determination that the hand movement indicates the scratch event, initiating a treatment protocol for the subject to treat pruritus. Among other benefits, these embodiments may provide an assessment of pruritus with greater accuracy and reliability (as compared to conventional solutions) based on accelerometer data acquired in a way to reduce burden on the user and increase user compliance. Using computerized classification models with the accelerometer data to detect scratch events helps remove bias and subjectivity, further improving accuracy and reliability. These classifiers help to provide reliable computer decision support tools that are based on detected scratch data, thereby improving recommendations for treatment and/or responses to scratching. As such, these embodiments may more effectively treat and manage pruritus (including in the form of atopic dermatitis) than conventional measures.

In the above embodiment of the method, initiating the treatment protocol is further based on a plurality of determinations that a plurality of hand movements each indicate a scratch event. Initiating the treatment protocol includes determining at least one of a therapeutic agent, a dosage, and a method of administration of the therapeutic agent.

In any combination of the above embodiments of the method, the therapeutic agent is selected from the group consisting of: infliximab, adalimumab, belimumab, tanezumab, ranibizumab, bevacizumab, mepolizumab certolizumab, natalizumab, ustekinumab, vedolizumab, 6-mercaptopurine, hydroxychloroquine, obeticholic acid, mofetil, sodium mycophenolate, leflunomide, rituxan, solumedrol, depomedrol, betamethasone, prednisone, cyclosporin, tacrolimus, pimecrolimus, dupilumab, omalizumab, tralokinumab, etokimab, nemolizumab, Tezepelumab, lebrikizumab, fezakinumab, anti-OX40, efalizumab, etanercept, crisaborole, fluocinonide, mapracorat, hydrocortisone, desonide, alclometasone, triamcinolone, desoximetasone, loratidine, fexofenadine, desloratidine, levocetirizine, methapyrilene, cetirizine, budesonide, fluticasone, mometasone, dexamethasone, prednisolone, ciclesonide, beclomethasone, methotrexate, azathioprine, aspirin, ibuprofen, celecoxib, valdecoxib, WBI-1001 and/or MRX-6, abrocitinib, baricitinib, brepocitinib, cerdulatinib, decernotinib, delgocitinib, fedratinib, filgotinib, gandotinib, ilginatinib, itacitinib, lestaurtinib, momelotinib, oclacitinib pacritinib, peficitinib, ritlecitinib, ruxolitinib, tofacitinib, upadacitinib, THRX-212401, PF-07055087, PF-06471658, PF-07055090, ATI-502, BMS-986165, JTE052, PF-06826647, SNA 152, SHR-0302, tapinarof, and/or alitretinoin.

In a preferred embodiment of any combination of the above embodiments, the therapeutic agent is selected from the group consisting of: crisaborole and abrocitinib.

In any combination of the above embodiments of the method, initiating administration of the treatment protocol includes generating a graphic user interface element provided for display on a user device. The graphic user interface element indicates a recommendation of the treatment protocol that based on the first determination that the hand movement represents the scratch event. This embodiment helps promote better treatment compliance for the subject and enables clinician's to make informed decisions with respect to treatment protocol for the subject.

In any combination of the above embodiments of the method, the user device is separate from the motion sensing device. For example, the user device may be a user computing device that is separate from the motion sensing device. One advantage of this embodiment allows the motion sensing device to be more portable and less bulky as it may be desirable for the display on the user device to be larger than what is permitted by a wearable device. Additionally, in some aspects, the user device may be a clinician user device and having that separate from the motion sensing device allows the data to be collected outside of the clinical setting, thereby improving the quality of the data and subject compliance.

In any combination of the above embodiments of the method, the method further comprises applying the treatment protocol to the subject based on the recommendation.

In any combination of the above embodiments of the method, the motion sensing device comprises a wearable device worn at an appendage of the subject. For example, the motion sensing device may be a wearable device worn at the subject's finger, wrist, or arm. Using wearable device may enable continuous (or semi-continuous, periodic, as-needed, or as-it-becomes-available) data capturing that is less intrusive than other types of monitoring, which may be beneficial in monitoring individuals in populations with typically lower compliance rates, such as children.

In any combination of the above embodiments of the method, the subject is diagnosed with atopic dermatitis based on the determination that the hand movement indicates a scratch event, and the treatment protocol is to treat atopic dermatitis.

In some embodiment, one or more computer storage media having computer-executable instructions embodied thereon that, when executed by one or more processors, cause the one or more processors to perform operations. The operations comprise: receiving accelerometer data for a subject; and causing for display, on a user device, one or more scratch endpoints for the subject based a determination that one or more hand movements detected from the accelerometer data indicate scratch events. The subject may comprise a human subject for which treatment of pruritus is sought. Among other benefits, these embodiments may provide an assessment of pruritus with greater accuracy and

43

reliability (as compared to conventional solutions) based on accelerometer data acquired in a way to reduce burden on the user and increase user compliance. Using computerized classification models with the accelerometer data to detect scratch events helps remove bias and subjectivity, further improving accuracy and reliability. These classifiers help to provide reliable computer decision support tools that are based on detected scratch data, thereby improving recommendations for treatment and/or responses to scratching. Further, scratch endpoints may represent novel digital endpoints that are useful in quantitatively and objectively measuring pruritus or, more specifically, atopic dermatitis. The graphic user interface element provided for display on a user device with the scratch endpoint indicator(s) promotes better treatment compliance for the individual being monitored and enables clinician's to make informed decisions with respect to treatment.

In the above embodiment of the computer storage media, accelerometer data is received from one or more sensors integrated into a wearable device that is communicatively coupled to the user device. Using wearable device may enable continuous (or semi-continuous, periodic, as-needed, or as-it-becomes-available) data capturing that is less intrusive than other types of monitoring, which may be beneficial in monitoring individuals in populations with typically lower compliance rates, such as children.

In any combination of the above embodiments of the computer storage media, the accelerometer data is captured by sensors integrated into a first wearable device and a second wearable device worn contemporaneously by the subject.

In any combination of the above embodiments of the computer storage media, the operations further comprise causing to display, on the user device, a treatment protocol for the subject for treating atopic dermatitis, the treatment protocol being based on the one or more scratch endpoints.

Many different arrangements of the various components depicted, as well as components not shown, are possible without departing from the scope of the claims below. Embodiments of the disclosure have been described with the intent to be illustrative rather than restrictive. Alternative embodiments will become apparent to readers of this disclosure after and because of reading it. Alternative means of implementing the aforementioned can be completed without departing from the scope of the claims below. Certain features and sub-combinations are of utility and may be employed without reference to other features and sub-combinations and are contemplated within the scope of the claims.

## Claims

1. A system for providing decision support based on scratch events, the system comprising: a processor; and a computer memory having computer executable instructions stored thereon for performing operations when executed by the processor, the operations comprising: receiving accelerometer data for an individual; detecting a hand movement utilizing the accelerometer data; utilizing a computerized classification model to determine, based on the accelerometer data corresponding to the hand movement, that the hand movement indicates a scratch event, wherein the model calculates a rolling coefficient of variation (CoV) based on the accelerometer data for the determination of hand movements; and initiating one or more response actions based at least on a determination that the hand movement indicates the scratch event.

2. The system of claim 1, wherein the operations performed by the processor executing the computer executable instructions further comprise: generating a multidimensional timeseries from the accelerometer data corresponding to the hand movement; and determining a plurality of feature values from the multidimensional timeseries, the plurality of feature values including at least one time-domain feature value and at least one frequency-domain feature value, wherein the determination that the hand movement is the scratch event is based on the plurality of feature values.

3. The system of claim 1, wherein the accelerometer data is captured by a wearable device located at an appendage of the individual.

4. The system of claim 1, wherein the operations performed by the processor executing the computer executable instructions further comprise determining a total sleep opportunity based on the accelerometer data, the total sleep opportunity comprising a period of time between when the individual lays down for a rest and when the individual gets up from the rest, wherein the hand movement is detected utilizing accelerometer data corresponding to the total sleep opportunity.

5. The system of claim 4, wherein the accelerometer data is captured by a wearable device having a plurality of sensors, wherein the wearable device further captures at least one of near-body temperature data and light data, the total sleep opportunity being determined further based on the at least one of near-body temperature data and light data.

6. The system of claim 1, wherein the computerized classification model utilized to determine that the hand movement indicates the scratch event comprises at least one of an ensemble of machine learning models and a random forest classifier.

7. The system of claim 1, wherein the one or more response actions comprises generating a graphic user interface element provided for display on a user device, the graphic user interface element including at least one of: an indicator of one or more scratch endpoints comprising a total number of scratch events and a total scratch duration; and an indicator recommending that the individual seek clinical consultation based on the determination that the hand movement indicates the scratch event.

8. The system of claim 7, wherein the total number of scratch events and the total scratch duration are each determined for a total sleep opportunity that is determined based on the accelerometer data received for the individual, the total sleep opportunity comprising a period of time between when the individual lays down for a rest and when the individual gets up from the rest.

9. One or more computer storage media having computer-executable instructions embodied thereon that, when executed by one or more processors, cause the one or more processors to perform operations comprising: receiving accelerometer data for a subject; and causing for display, on a user device, one or more scratch endpoints for the subject based a determination that one or more hand movements detected from the accelerometer data indicate scratch events.

10. The computer storage media of claim 9, wherein the accelerometer data is received from one or more sensors integrated into a wearable device that is communicatively coupled to the user device.

11. The computer storage media of claim 9, wherein the accelerometer data is captured by sensors integrated into a first wearable device and a second wearable device worn contemporaneously by the subject.

12. The computer storage media of claim 9, wherein the operations further comprise causing to display, on the user device, a treatment protocol for the subject for treating atopic dermatitis, the treatment protocol being based on the one or more scratch endpoints.


**Patentansprüche**

1. Ein System zur Entscheidungsunterstützung auf der Grundlage von Kratzereignissen, wobei das System umfasst: einen Prozessor; und einen Computerspeicher mit darauf gespeicherten computerausführbaren Anweisungen zur Durchführung von Operationen bei Ausführung durch den Prozessor, wobei die Operationen umfassen: Empfangen von Beschleunigungsmesserdaten für eine Person; Erfassen einer Handbewegung unter Verwendung der Beschleunigungsmesserdaten; Verwenden eines computergestützten Klassifizierungsmodells, um auf der Grundlage der der Handbewegung entsprechenden Beschleunigungsmesserdaten zu bestimmen, dass die Handbewegung ein Kratzereignis anzeigt, wobei das Modell einen gleitenden Variationskoeffizienten (CoV) auf der Grundlage der Beschleunigungsmesserdaten zur Bestimmung von Handbewegungen berechnet; und Einleiten einer oder mehrerer Reaktionsmaßnahmen, die zumindest auf einer Bestimmung basieren, dass die Handbewegung das Kratzereignis anzeigt.

2. Das System nach Anspruch 1, wobei die von dem Prozessor bei der Ausführung der computerausführbaren Anweisungen durchgeführten Operationen ferner umfassen: Erzeugen einer mehrdimensionalen Zeitreihe aus den der Handbewegung entsprechenden Beschleunigungsmesserdaten; und Bestimmen einer Vielzahl von Merkmalswerten aus der mehrdimensionalen Zeitreihe, wobei die Vielzahl von Merkmalswerten mindestens einen Zeitbereichs-Merkmalswert und mindestens einen Frequenzbereichs-Merkmalswert umfasst, wobei die Bestimmung, dass die Handbewegung das Kratzereignis ist, auf der Vielzahl von Merkmalswerten basiert.

3. Das System nach Anspruch 1, wobei die Beschleunigungsmesserdaten von einer tragbaren Vorrichtung erfasst werden, die sich an einer Gliedmaße der Person befindet.

4. Das System nach Anspruch 1, wobei die von dem Prozessor bei der Ausführung der computerausführbaren Anweisungen durchgeführten Operationen ferner das Bestimmen einer Gesamtschlafgelegenheit auf der Grundlage der Beschleunigungsmesserdaten umfassen, wobei die Gesamtschlafgelegenheit eine Zeitspanne zwischen dem Zeitpunkt, zu dem sich die Person zur Ruhe legt, und dem Zeitpunkt, zu dem die Person von der Ruhe aufsteht, umfasst, wobei die Handbewegung unter Verwendung von Beschleunigungsmesserdaten erfasst wird, die der Gesamtschlafgelegenheit entsprechen.

5. Das System nach Anspruch 4, wobei die Beschleunigungsmesserdaten von einer tragbaren Vorrichtung mit einer Vielzahl von Sensoren erfasst werden, wobei die tragbare Vorrichtung ferner mindestens eines von körpernahen Temperaturdaten und Lichtdaten erfasst, wobei die Gesamtschlafgelegenheit ferner auf der Grundlage des mindestens einen von körpernahen Temperaturdaten und Lichtdaten bestimmt wird.

6. Das System nach Anspruch 1, wobei das computergestützte Klassifizierungsmodell, das verwendet wird, um zu bestimmen, dass die Handbewegung das Kratzereignis anzeigt, mindestens eines von einem Ensemble von maschinellen Lernmodellen und einem Random-Forest-Klassifikator umfasst.

7. Das System nach Anspruch 1, wobei die eine oder die mehreren Reaktionsmaßnahmen das Erzeugen eines grafischen Benutzeroberflächenelements umfassen, das zur Anzeige auf einem Benutzergerät bereitgestellt wird, wobei das grafische Benutzeroberflächenelement mindestens eines der Folgenden einschließt: einen Indikator für einen oder mehrere Kratz-Endpunkte, die eine Gesamtzahl von Kratzereignissen und eine Gesamtkratzdauer umfassen; und einen Indikator, der empfiehlt, dass die Person eine klinische Beratung aufsucht, basierend auf der Bestimmung, dass die Handbewegung das Kratzereignis anzeigt.

8. Das System nach Anspruch 7, wobei die Gesamtzahl der Kratzereignisse und die Gesamtkratzdauer jeweils für eine Gesamtschlafgelegenheit bestimmt werden, die auf der Grundlage der für die Person empfangenen Beschleunigungsmesserdaten bestimmt wird, wobei die Gesamtschlafgelegenheit eine Zeitspanne zwischen dem Zeitpunkt, zu dem sich die Person zur Ruhe legt, und dem Zeitpunkt, zu dem die Person von der Ruhe aufsteht, umfasst.

9. Ein oder mehrere Computerspeichermedien mit darauf verkörperten computerausführbaren Anweisungen, die bei Ausführung durch einen oder mehrere Prozessoren den einen oder die mehreren Prozessoren veranlassen, Operationen durchzuführen, die umfassen: Empfangen von Beschleunigungsmesserdaten für ein Subjekt; und Veranlassen der Anzeige eines oder mehrerer Kratz-Endpunkte für das Subjekt auf einem Benutzergerät, basierend auf einer Bestimmung, dass eine oder mehrere aus den Beschleunigungsmesserdaten erfasste Handbewegungen Kratzereignisse anzeigen.

10. Die Computerspeichermedien nach Anspruch 9, wobei die Beschleunigungsmesserdaten von einem oder mehreren Sensoren empfangen werden, die in eine tragbare Vorrichtung integriert sind, die kommunikativ mit dem Benutzergerät gekoppelt ist.

11. Die Computerspeichermedien nach Anspruch 9, wobei die Beschleunigungsmesserdaten von Sensoren erfasst werden, die in eine erste tragbare Vorrichtung und eine zweite tragbare Vorrichtung integriert sind, die gleichzeitig von dem Subjekt getragen werden.

12. Die Computerspeichermedien nach Anspruch 9, wobei die Operationen ferner das Veranlassen der Anzeige eines Behandlungsprotokolls für das Subjekt zur Behandlung von atopischer Dermatitis auf dem Benutzergerät umfassen, wobei das Behandlungsprotokoll auf dem einen oder den mehreren Kratz-Endpunkten basiert.

**Revendications**

1. Un système d'aide à la décision basé sur des événements de grattage, le système comprenant : un processeur, et une mémoire d'ordinateur ayant des instructions exécutables par ordinateur stockées sur celle-ci pour exécuter des opérations lorsqu'elles sont exécutées par le processeur, les opérations comprenant : la réception de données d'accéléromètre pour un individu ; la détection d'un mouvement de la main en utilisant les données d'accéléromètre ; l'utilisation d'un modèle de classification informatisé pour déterminer, sur la base des données d'accéléromètre correspondant au mouvement de la main, que le mouvement de la main indique un événement de grattage, dans lequel le modèle calcule un coefficient de variation glissant (CoV) sur la base des données d'accéléromètre pour la détermination des mouvements de la main ; et le lancement d'une ou plusieurs actions de réponse sur la base au moins d'une détermination que le mouvement de la main indique l'événement de grattage.

2. Le système selon la revendication 1, dans lequel les opérations exécutées par le processeur exécutant les instructions exécutables par ordinateur comprennent en outre : la génération d'une série temporelle multidimensionnelle à partir des données d'accéléromètre correspondant au mouvement de la main ; et la détermination d'une pluralité de valeurs de caractéristiques à partir de la série temporelle multidimensionnelle, la pluralité de valeurs de caractéristiques incluant au moins une valeur de caractéristique du domaine temporel et au moins une valeur de caractéristique du domaine fréquentiel, dans lequel la détermination que le mouvement de la main est l'événement de grattage est basée sur la pluralité de valeurs de caractéristiques.

3. Le système selon la revendication 1, dans lequel les données d'accéléromètre sont capturées par un dispositif portable situé au niveau d'un appendice de l'individu.

4. Le système selon la revendication 1, dans lequel les opérations exécutées par le processeur exécutant les instructions exécutables par ordinateur comprennent en outre la détermination d'une opportunité de sommeil totale sur la base des données d'accéléromètre, l'opportunité de sommeil totale comprenant une période de temps entre le moment où l'individu se couche pour se reposer et le moment où l'individu se lève du repos, dans lequel le mouvement de la main est détecté en utilisant des données d'accéléromètre correspondant à l'opportunité de sommeil totale.

5. Le système selon la revendication 4, dans lequel les données d'accéléromètre sont capturées par un dispositif portable ayant une pluralité de capteurs, dans lequel le dispositif portable capture en outre au moins l'une parmi des données de température proche du corps et des données de luminosité, l'opportunité de sommeil totale étant déterminée en outre sur la base de l'au moins une parmi les données de température proche du corps et les données de luminosité.

6. Le système selon la revendication 1, dans lequel le modèle de classification informatisé utilisé pour déterminer que le mouvement de la main indique l'événement de grattage comprend au moins l'un parmi un ensemble de modèles d'apprentissage automatique et un classificateur de forêt aléatoire.

7. Le système selon la revendication 1, dans lequel la ou les actions de réponse comprennent la génération d'un élément d'interface utilisateur graphique prévu pour l'affichage sur un dispositif utilisateur, l'élément d'interface utilisateur graphique incluant au moins l'un parmi : un indicateur d'un ou plusieurs critères d'évaluation du grattage comprenant un nombre total d'événements de grattage et une durée totale de grattage ; et un indicateur recommandant que l'individu recherche une consultation clinique sur la base de la détermination que le mouvement de la main indique l'événement de grattage.

8. Le système selon la revendication 7, dans lequel le nombre total d'événements de grattage et la durée totale de grattage sont chacun déterminés pour une opportunité de sommeil totale qui est déterminée sur la base des données d'accéléromètre reçues pour l'individu, l'opportunité de sommeil totale comprenant une période de temps entre le moment où l'individu se couche pour se reposer et le moment où l'individu se lève du repos.

9. Un ou plusieurs supports de stockage informatique ayant des instructions exécutables par ordinateur incorporées sur ceux-ci qui, lorsqu'elles sont exécutées par un ou plusieurs processeurs, amènent le ou les processeurs à exécuter des opérations comprenant : la réception de données d'accéléromètre pour un sujet ; et le fait de provoquer l'affichage, sur un dispositif utilisateur, d'un ou plusieurs critères d'évaluation du grattage pour le sujet sur la base d'une détermination qu'un ou plusieurs mouvements de la main détectés à partir des données d'accéléromètre indiquent des événements de grattage.

10. Les supports de stockage informatique selon la revendication 9, dans lesquels les données d'accéléromètre sont reçues d'un ou plusieurs capteurs intégrés dans un dispositif portable qui est couplé de manière communicative au dispositif utilisateur.

11. Les supports de stockage informatique selon la revendication 9, dans lesquels les données d'accéléromètre sont capturées par des capteurs intégrés dans un premier dispositif portable et un second dispositif portable portés simultanément par le sujet.

12. Les supports de stockage informatique selon la revendication 9, dans lesquels les opérations comprennent en outre le fait de provoquer l'affichage, sur le dispositif utilisateur, d'un protocole de traitement pour le sujet pour le traitement de la dermatite atopique, le protocole de traitement étant basé sur le ou les critères d'évaluation du grattage.

FIG. 1

FIG. 2

FIG. 3A

FIG. 3B

400

```
┌─────────────────────────────────────┐
│         RECEIVE SENSOR DATA          │~410
└─────────────────────────────────────┘
                  ↓
┌─────────────────────────────────────┐
│   DETERMINE SENSOR(S) CONFIGURED     │~420
│     FOR PROPER DATA ACQUISITION      │
└─────────────────────────────────────┘
                  ↓
┌─────────────────────────────────────┐
│    DETERMINE USER SLEEP OPPORTUNITY  │~430
└─────────────────────────────────────┘
                  ↓
┌─────────────────────────────────────┐
│     DETECT USER HAND MOTION EVENT    │~440
└─────────────────────────────────────┘
                  ↓
┌─────────────────────────────────────┐
│      DETECT LIKELY SCRATCH EVENT     │~450
└─────────────────────────────────────┘
                  ↓
┌─────────────────────────────────────┐
│        RECORD SCRATCH EVENT          │~460
└─────────────────────────────────────┘
                  ↓
┌─────────────────────────────────────┐
│           INITIATE ACTION            │~470
└─────────────────────────────────────┘
```

*FIG. 4A*

EP 4 167 838 B1

EXAMPLE SENSOR CONFIGURATION (E.G., WEAR DETECTION)

4210 — RAW X, Y, Z

4220 — 1 HOUR WINDOWS, 15 MINUTE OVERLAP

4230 — ANY2(STD(X, Y, Z)) < 0.013Gs OR ANY2(RANGE(X, Y, Z)) < 0.15Gs

4240 — NON-WEAR

4250 — RESCORING RULES*

4260 — NON-WEAR RESCORED

RESCORING IN ACTION:

| A | B | C | D | E |
| WEAR | NON-WEAR | WEAR | NON-WEAR | WEAR |

4271  4273  4275  4277  4279

FIG. 4B

4300

EXAMPLE DETERMINE USER SLEEP OPPORTUNITY (E.G., TSO)

4310
5-SECOND ROLLING MEDIAN OF RAW SIGNAL (X, Y, Z)

4320
$$arm\_angle_z = \left( tan^{-1} \frac{a_z}{\sqrt{a_x^2 + a_y^2}} \right) \cdot \frac{180}{\pi}$$

4330
CONSECUTIVE 5 SECOND AVERAGE

4340
ABSOLUTE DIFFERENCE BETWEEN SUCCESSIVE VALUES

4370
NON-WEAR FILTERING: DROP CANDIDATE PERIODS WITH DETECTED NON-WEAR

4360
CANDIDATE REST PERIOD: DETECT WHEN VALUES
$$\leq max(0.15 \times 10^{th} percentile)$$

4350
ROLLING MEDIAN OF 5 MINUTE WINDOW

4380
KEEP CANDIDATE REST PERIODS > 30 MINUTES

4390
INCLUDE 15 MINUTE OR LESS GAP BETWEEN CANDIDATE PERIODS

4395
TAKE THE LONGEST CANDIDATE OF A DAY AS TSO

*FIG. 4C*

EP 4 167 838 B1

4800

EP 4 167 838 B1

EXAMPLE USER SLEEP/WAKE DETECTION

4810

HIGH PASS
FILTER > 0.25

4820

ACTIVITY INDEX AT MINUTE LEVEL

$$A_t = \sqrt{max\left(\frac{1}{3}\left(\sum_{m}^{3}\sigma_{im}^2(t) - \bar{\sigma}_t^2\right), 0\right)}$$

4830

$$D_0 = 0.243 \cdot (W_{-4}A_{-4} + W_{-3}A_{-3} + W_{-2}A_{-2} + W_{-1}A_{-1} + W_0A_0 + W_{+1}A_{+1} + W_{+2}A_{+2})$$

4840

SLEEP =
$$D_0 < 0.5$$

4850

WEBSTER'S
RESCORING
RULES

4860

**AGGREGATE ENDPOINTS DURING TSO**
TOTAL SLEEP TIME, PERCENT TIME ASLEEP, WAKE AFTER SLEEP
ONSET, SLEEP ONSET LATENCY, NUMBER OF WAKE BOUTS

*FIG. 4D*

4001

SCRATCH DETECTOR (CLASSIFICATION PIPELINE)

4010

4012
RAW SIGNAL (X, Y, Z)
20Hz SLICED TO TSO
WINDOW

4014
RAW SIGNAL WINDOWED
IN 3-S NON-OVERLAPPING
WINDOWS

HAND MOVEMENT
DETECTION

4040

4042
COMPUTE ROLLING (1-S)
COEFFICIENT OF
VARIATION (CoV)

4044
$Hand\ Movement = CoV > 0.023$

SCRATCH
CLASSIFICATION

4050

4052
**PREPROCESS DATA**
1. HIGH PASS FILTER (> 0.25)
2. VECTOR MAGNITUDE OF (X, Y, Z)
3. $1^{ST}$ AND $2^{ND}$ PRINCIPAL
COMPONENT OF (X, Y, Z)

4054
**FEATURE EXTRACTION**
COMPUTE 26 SELECTED
TIME AND FREQUENCY
DOMAIN FEATURES

4058
**ENDPOINTS**
TOTAL SCRATCH
COUNTS AND DURATION

4056
RUN COMPUTED FEATURES
THROUGH BINARY SCRATCH
CLASSIFIER

*FIG. 4E*

4500

```
┌─────────────────────────────────────┐
│      RECEIVE ACCELEROMETER DATA      │──4510
└─────────────────────────────────────┘
                    │
                    ▼
┌─────────────────────────────────────┐
│    DETECT HAND MOVEMENT UTILIZING    │──4520
│         ACCELEROMETER DATA           │
└─────────────────────────────────────┘
                    │
                    ▼
┌─────────────────────────────────────┐
│ UTILIZE COMPUTER CLASSIFICATION MODEL│
│ TO DETERMINE, BASED ON ACCELEROMETER │
│   DATA CORRESPONDING TO HAND         │──4530
│ MOVEMENT, THAT THE HAND MOVEMENT     │
│     INDICATES A SCRATCH EVENT        │
└─────────────────────────────────────┘
                    │
                    ▼
┌─────────────────────────────────────┐
│ INITIATE ONE OR MORE RESPONSE ACTIONS│
│ BASED AT LEAST ON A DETERMINATION THAT│──4540
│   THE HAND MOVEMENT INDICATES THE    │
│           SCRATCH EVENT              │
└─────────────────────────────────────┘
```

*FIG. 4F*

4600

RECEIVE ACCELEROMETER DATA
COLLECTED FROM MOTION SENSING DEVICE ~4610

DETECT HAND MOVEMENT UTILIZING
ACCELEROMETER DATA ~4620

UTILIZE COMPUTER CLASSIFICATION MODEL
TO DETERMINE, BASED ON ACCELEROMETER
DATA CORRESPONDING TO HAND
MOVEMENT, THAT THE HAND MOVEMENT
INDICATES A SCRATCH EVENT ~4630

BASED ON AT LEAST A FIRST
DETERMINATION THAT THE HAND MOVEMENT
INDICATES THE SCRATCH EVENT, INITIATE A
TREATMENT PROTOCOL FOR THE SUBJECT
TO TREAT PRURITUS ~4640

*FIG. 4G*

4700

RECEIVE ACCELEROMETER DATA FOR A SUBJECT ～4710

CAUSE FOR DISPLAY, ON USER DEVICE, ONE OR MORE SCRATCH ENDPOINTS FOR THE SUBJECT BASED ON A DETERMINATION ～4720 THAT ONE OR MORE HAND MOVEMENTS DETECTED FROM THE ACCELEROMETER DATA INDICATE SCRATCH EVENTS

*FIG. 4H*

500

DETERMINE USER SCRATCH PATTERN(S) ~510

DETERMINE CONTEXTUAL INFORMATION ~520

DETERMINE USER ITCH FOR FUTURE TIME INTERVAL(S) ~530

DETERMINE LIKELIHOOD OF FLARE EVENT ~540

INITIATE ACTION ~550

*FIG. 5*

SCRATCH CLASSIFIER MODEL TRAINING PIPELINE

DATA PREPROCESSING

610

612
ALIGNMENT OF VIDEO ANNOTATIONS TO ACCELEROMETER DATA

614
DOWNSAMPLE TO 20Hz

616
**FILTER ANNOTATIONS**
1. SCRATCH/RESTLESS ANNOTATIONS > 3s
2. HAND MOVEMENT PRESENT THROUGHOUT ANNOTATION

SIGNAL PREPROCESSING

620

622
SEGMENT (X, Y, Z) INTO 3-SECOND WINDOW

624
HIGH-PASS FILTER (>0.25)

626
**TRANSFORM SIGNALS**
1. VECTOR MAGNITUDE OF (X, Y, Z)
2. $1^{ST}$ AND $2^{ND}$ PRINCIPAL COMPONENTS OF (X, Y, Z)

FEATURE ENGINEERING

630

632
EXTRACT 36 TIME AND FREQUENCY DOMAIN FEATURES

634
PCA AND FEATURE WEIGHTS RANKING

LEAVE ONE SUBJECT OUT DEVELOPMENT

640

642
BALANCE POSITIVE AND NEGATIVE CLASSES 50:50

644
RFECV FEATURE SELECTION WITH *DECISION TREE*

**VALIDATION PERFORMANCE**
1. CONFUSION MATRIX
2. AUC ROC

646
TRAIN RANDOM FOREST CLASSIFIER (50 TREES)

648

600

*FIG. 6A*

6301

**2 COMPONENT PCA OF FEATURE SET**

● HAND MOVEMENT
  NO SCRATCH
□ SCRATCH

6351

FEATURE SELECTION

**RECURSIVE FEATURE ELIMINATION WITH CROSS VALIDATION (RFECV)**

TOP 10 FEATURES

6300

*FIG. 6B*

EP 4 167 838 B1

SCRATCH DETECTION
PERFORMANCE VALIDATION

6400

*FIG. 6C*

EP 4 167 838 B1

**SCRATCH DETECTION STATISTICAL PERFORMANCE**

6480

6482

VIDEO ANNOTATION

| | SCRATCHING | NO SCRATCHING |
|---|---|---|
| SCRATCHING | 1259 (73%) | 301 (17%) |
| NO SCRATCHING | 465 (27%) | 1423 (83%) |
| | SENSITIVITY: 73% | SPECIFICITY: 83% |

SCRATCH ALGORITHM

6484

ROC CURVE

ROC CURVE AREA = 0.85

6486

VIDEO ANNOTATION

| | SCRATCHING | NO SCRATCHING |
|---|---|---|
| SCRATCHING | 5358 (81%) | 1263 (19%) |
| NO SCRATCHING | 2812 (33%) | 5631 (67%) |
| | SENSITIVITY: 67% | SPECIFICITY: 81% |

SCRATCH ALGORITHM

6488

ROC CURVE

ROC CURVE AREA = 0.80

*FIG. 6D*

SLEEP-RELATED SIGNAL ANALYSIS

6510 {
— X
········ Y
·— — Z

6520 { TEMP MAX: 39.1°C
MIN: 21.3°C

6530 { LIGHT

6540 { ACTIVITY

6550 { ARM ANGLE

6560 { WAKE
6570 { REST
ON BODY
6580 { ON BODY (RESCORE)

11:00 12:00 13:00 14:00 15:00 16:00 17:00 18:00 19:00 20:00 21:00 22:00 23:00 0:00 1:00 2:00 3:00 4:00 5:00 6:00 7:00 8:00 9:00 10:00 11:00 12:00 13:00

*FIG. 6E*

6500

EP 4 167 838 B1

*FIG. 6F*

*FIG. 7A*

7500

EXAMPLE: CONTINUOUS DETECTION OF SLEEP AND NIGHTTIME SCRATCH FOR A 5-DAY SUBJECT RECORDING

|  | TOTAL SLEEP OPPORTUNITY (MIN) | TOTAL SLEEP TIME (MIN) | PERCENT TIME ASLEEP (%) | TOTAL SCRATCH EVENTS (COUNTS) | TOTAL SCRATCH DURATION (MIN) |
|---|---|---|---|---|---|
| TUESDAY | 414.0 | 300.0 | 72.46 | 249.0 | 32.65 |
| WEDNESDAY | 551.0 | 469.0 | 85.12 | 197.0 | 28.65 |
| THURSDAY | 491.0 | 430.0 | 87.58 | 135.0 | 24.25 |
| FRIDAY | 543.0 | 498.0 | 91.71 | 103.0 | 14.75 |

7510

7520

Time (Day HH:MM)

— Activity
⊡ Detected TSO
— Detected Scratch

*FIG. 7B*

EP 4 167 838 B1

*FIG. 8A*

8200

8203

1:37 PM

☰  < Sun Jan 12 >  ⌲  🩺  🔄

8202 — **Nightly Summary**  ✏️ Edit

8210 — 📋 Scores

☾ **Scratching Score: 36** [*Green*] — 8212

- Your Scratch Trend: ➚
- Scratching Events: **12**
- Total Scratch Time: **84 sec**
- Ave. Scratch Duration: **7 sec**
- Longest Scratch: **12 sec**

⎫ — 8213

8230 — 📊 Charts

☾ **Sleep Score: Very Good** — 8216

- Sleep Percentage: 86%
- Total Sleep Time: 7 hr 25 min

⎫ — 8217

SOL: 3 | WASO: 33 | NWB: 6

8240 — 📷 Photo

8250 — 📄 Notes

Activity

Detected Scratch

Sun 22:00  Mon 00:00  Mon 02:00  Mon 04:00  Mon 06:00

— 8218

8260 — 🧴 Treatment

8112

🏠 Home  📅31 **Log**  📈 Forecast  📄 Reports  ⚙️ Settings — 8110

**Scratch Monitor App**

8102a

8101

*FIG. 8B*

8300

8303

3:14 PM

Today Tue Mar 17

8301

# Your Itch Forecast

Itch Forecast Trend: Increasing — 8320

| Low | Moderate | High |
|---|---|---|
| **Wed**<br>Mar 18 | **Thu**<br>Mar 19 | **Fri**<br>Mar 20 |

— 8310

**Recommendation:** Use your topical treatment every day, as directed. — 8330

[With Treatment]          [Without Treatment]

8341          Viewing →          8343

8340

8113

| Home | Log | Forecast | Reports | Settings |

— 8110

Scratch Monitor App

8102a

8101

## FIG. 8C

8400

8403

11:38 AM

☰ Today May 4

8401 **FLARE WARNING!**

When: Tomorrow to Thursday
Chance: 74%
Level: Severe

— 8410

Check/Refill Your Prescription

8422

Schedule Tele-Appointment

8424

Add OC Therapy to Shopping List

8426
8428

Snooze/Remind Me Later

— 8420

Home | Log | Forecast | Reports | Settings

— 8110

8102a

**Scratch Monitor App**

8101

*FIG. 8D*

FIG. 8E

**TABULAR OUTPUT:**

8600

| DAY | TST | PTA | WASO | SOL | NWB |
|-----|-----|-----|------|-----|-----|
| 1 | 420 | 92.02 | 33 | 3 | 7 |
| 2 | 507 | 87.68 | 45 | 4 | 12 |
| 3 | 433 | 94.24 | 34 | 10 | 5 |
| 4 | 531 | 96.97 | 25 | 3 | 8 |
| 5 | 264 | 92.75 | 12 | 8 | 4 |
| 6 | 423 | 91.94 | 17 | 9 | 4 |
| 7 | 423 | 93.51 | 18 | 3 | 5 |
| 8 | 354 | 96.27 | 33 | 1 | 3 |
| 9 | 508 | 96.09 | 14 | 8 | 8 |
| 10 | 432 | 87.96 | 31 | 4 | 14 |
| 11 | 457 | 88.68 | 31 | 4 | 7 |
| 12 | 343 | 92.60 | 14 | 2 | 5 |
| 13 | 505 | 95.01 | 23 | 6 | 8 |
| 14 | 401 | 92.83 | 32 | 5 | 7 |

(8610 — DAY; 8620 — TST; 8630 — PTA; 8640 — WASO; 8650 — SOL; 8660 — NWB)

8700

**SUMMARY REPORT FOR SOURCE: EXAMPLE DATA**

8720 — TOTAL SLEEP TIME(MIN) MEAN: 429
420 507 533 531 264 423 432 354 508 432 457 343 505 401

8730 — PERCENT TIME ASLEEP MEAN: 93
92 88 94 97 93 92 94 96 96 88 89 93 95 93

8740 — WAKE AFTER SLEEP ONSET(MIN) MEAN: 26
33 45 34 25 12 17 18 33 14 31 31 14 23 32

8750 — SLEEP ONSET LATENCY(MIN) MEAN: 5
3 4 10 3 8 9 3 1 8 4 4 2 6 5

8760 — NUMBER OF WAKE BOUTS MEAN: 7
7 12 5 8 4 4 5 3 8 14 7 5 8 7

1 2 3 4 5 6 7 8 9 10 11 12 13 14
DAY

*FIG. 8F*    EXAMPLE DISPLAYED ANALYTICS FOR SLEEP

EP 4 167 838 B1

```
##############################################################################
#
#    ScratchSleep Combined (pre-alpha)
#
##############################################################################

import matplotlib.pyplot as plt
import matplotlib.dates as mdates
from matplotlib.dates import DateFormatter
import matplotlib.backends.backend_pdf
from sleeppy import SleepPy
from scratchpy import ScratchPy
from ScratchSleepSolution.utils import build_logger
import pandas as pd
import numpy as np


class ScratchSleep(SleepPy):
    """

    This package pulls the SleepPy and ScratchPy packages into a single pipeline for the analysis of sleep and
    scratch behaviors using triaxial accelerometer data. The pipeline is built to accept GeneActiv .bin or .csv files
    and process it to produce clinical end points for sleep and scratch on a 24 hour basis(measured from noon to noon).

    """

    def __init__(
        self,
        input_file,
        results_directory,
        sampling_frequency=None,
        plot=False,
        verbose=False,
        export_scratch_data=False,
        logger=None,
    ):
        """
        Class initialization.

        Parameters
        ----------
        input_file : string
            Full path to the input file (geneactiv .bin or .csv).
        results_directory : string
            Full path to the output directory.
        sampling_frequency : float
            Sampling rate of the data in Hz.
        plot : boolean
            Enables plotting.
        verbose : boolean
            Enables progress statements.
        export_scratch_data : boolean
            Exports scratch-specific predictions, plots, and endpoints.
        logger : logger object
            Use custom logger.
```

## FIG. 9A

## CONTINUES FROM FIG. 9A

```
    """
        super().__init__(input_file, results_directory, sampling_frequency)
        self.allowed_rest_break = 150
        self.minimum_rest_threshold = 0.1
        self.min_t = 25.0
        self.minimum_hours = 12
        self.fs = sampling_frequency
        self.scratch_endpoints = []
        self.export_scratch_data = export_scratch_data
        self.plot = plot
        self.verbose = verbose
        self.movement_based_nonwear = 0.0010
        # Set up logging
        if "_" not in self.src_name:
            raise ValueError("Input filename format incorrect. Quitting.")
        if len(self.src_name.split("_")) < 8:
            raise ValueError("Input filename format incorrect. Quitting.")
        self.study = self.src_name.split("_")[0]
        self.subject = self.src_name.split("_")[5]
        self.device_location = self.src_name.split("_")[6]
        self.visit = self.src_name.split("_")[7]
        log_filename = (
            results_directory
            + "/analytics_pipeline_"
            + self.study
            + "_"
            + self.subject
            + "_"
            + self.device_location
            + "_"
            + self.visit
            + ".log"
        )
        if logger is None:
            self.logger = build_logger(log_name=__name__, log_path=log_filename,)
        else:
            self.logger = logger
        return

    def run(self):
        """
        Runs the full scratch and sleep analysis end to end on the input data

        """
        if self.verbose:
            self.logger.info(
                "[ANALYTICS_PIPELINE] [INFO] Study: [{}] | Subject: [{}] | "
                "Location: [{}] | Visit: [{}] | Running Scratch and Sleep Analytics "
                "Pipeline on single wrist.".format(
                    self.study, self.subject, self.device_location, self.visit
                )
            )
```

CONTINUES IN FIG. 9C

# *FIG. 9B*

CONTINUES FROM FIG. 9B

```
try:
    self.run_sleep()
    if self.verbose:
        self.logger.info(
            "[ANALYTICS_PIPELINE] [SUCCESS] Study: [{}] | Subject: [{}] | "
            "Location: [{}] | Visit: [{}] | Ran SleepPy.".format(
                self.study, self.subject, self.device_location, self.visit
            )
        )
except Exception as e:
    if self.verbose:
        self.logger.info(
            "[ANALYTICS_PIPELINE] [FAILURE] Study: [{}] | Subject: [{}] | "
            "Location: [{}] | Visit: [{}] | SleepPy run fail.".format(
                self.study, self.subject, self.device_location, self.visit
            ),
            exc_info=True,
        )


try:
    self.run_scratch()
    if self.verbose:
        self.logger.info(
            "[ANALYTICS_PIPELINE] [SUCCESS] Study: [{}] | Subject: [{}] | "
            "Location: [{}] | Visit: [{}] | Ran ScratchPy.".format(
                self.study, self.subject, self.device_location, self.visit
            )
        )
except Exception as e:
    if self.verbose:
        self.logger.info(
            "[ANALYTICS_PIPELINE] [FAILURE] Study: [{}] | Subject: [{}] | "
            "Location: [{}] | Visit: [{}] | ScratchPy run fail.".format(
                self.study, self.subject, self.device_location, self.visit
            ),
            exc_info=True,
        )


try:
    self.export_scratch_sleep()
    if self.verbose:
        self.logger.info(
            "[ANALYTICS_PIPELINE] [SUCCESS] Study: [{}] | Subject: [{}] | "
            "Location: [{}] | Visit: [{}] | Exported scratch/sleep results.".format(
                self.study, self.subject, self.device_location, self.visit
            )
        )
except Exception as e:
    if self.verbose:
        self.logger.info(
            "[ANALYTICS_PIPELINE] [FAILURE] Study: [{}] | Subject: [{}] | "
            "Location: [{}] | Visit: [{}] | Export scratch/sleep results fail".format(
                self.study, self.subject, self.device_location, self.visit
            ),
            exc_info=True,
        )
return
```

*FIG. 9C*

CONTINUES FROM FIG. 9C    .

      .

```python
def run_sleep(self):
    """

    Runs the sleep analysis portion of the package on the input data.

    """
    if self.verbose:
        self.logger.info(
            "[SLEEPPY] [INFO] Study: [{}] | Subject: [{}] | "
            "Location: [{}] | Visit: [{}] | Running SleepPy analysis.".format(
                self.study, self.subject, self.device_location, self.visit
            )
        )

    self.load()
    if self.verbose:
        self.logger.info(
            "[SLEEPPY] [INFO] Study: [{}] | Subject: [{}] | "
            "Location: [{}] | Visit: [{}] | Loaded data.".format(
                self.study, self.subject, self.device_location, self.visit
            )
        )

    self.calculate_major_rest_periods()
    if self.verbose:
        self.logger.info(
            "[SLEEPPY] [INFO] Study: [{}] | Subject: [{}] | "
            "Location: [{}] | Visit: [{}] | Calculated total sleep opportunity for all nights.".format(
                self.study, self.subject, self.device_location, self.visit
            )
        )

    self.calculate_sleep_predictions()
    if self.verbose:
        self.logger.info(
            "[SLEEPPY] [INFO] Study: [{}] | Subject: [{}] | "
            "Location: [{}] | Visit: [{}] | Calculated sleep/wake predictions for all nights.".format(
                self.study, self.subject, self.device_location, self.visit
            )
        )

    self.calculate_endpoints()
    if self.verbose:
        self.logger.info(
            "[SLEEPPY] [INFO] Study: [{}] | Subject: [{}] | "
            "Location: [{}] | Visit: [{}] | Calculated sleep endpoints for all nights.".format(
                self.study, self.subject, self.device_location, self.visit
            )
        )
```

      .

      .

## FIG. 9D

CONTINUES FROM FIG. 9D    .

                                          .

```
    if self.plot:
        self.visualize()
        if self.verbose:
            vis_fp_days = "/{}_visual_report.pdf".format(self.src_name)
            vis_fp_summary = "/{}_summary_report.pdf".format(self.src_name)
            self.logger.info(
                "[SLEEPPY] [INFO] Study: [{}] | Subject: [{}] | "
                "Location: [{}] | Visit: [{}] | Generated sleep analysis reports.".format(
                    self.study, self.subject, self.device_location, self.visit
                )
            )
            self.logger.info(
                "[SLEEPPY] [INFO] Study: [{}] | Subject: [{}] | "
                "Location: [{}] | Visit: [{}] | Saved: {}.".format(
                    self.study,
                    self.subject,
                    self.device_location,
                    self.visit,
                    vis_fp_days,
                )
            )
            self.logger.info(
                "[SLEEPPY] [INFO] Study: [{}] | Subject: [{}] | "
                "Location: [{}] | Visit: [{}] | Saved: {}.".format(
                    self.study,
                    self.subject,
                    self.device_location,
                    self.visit,
                    vis_fp_summary,
                )
            )
        return

def run_scratch(self):
    """
    Runs the scratch analysis portion of the package on the input data.

    """
    if self.verbose:
        self.logger.info(
            "[ANALYTICS_PIPELINE] [INFO] Study: [{}] | Subject: [{}] | "
            "Location: [{}] | Visit: [{}] | Running ScratchPy analysis.".format(
                self.study, self.subject, self.device_location, self.visit
            )
        )
    if self.plot:
        pdf = matplotlib.backends.backend_pdf.PdfPages(
            self.dst + "/{}_scratch_report.pdf".format(self.src_name)
        )
```

                                          .

                                          .

CONTINUES IN FIG. 9F

# *FIG. 9E*

CONTINUES FROM FIG. 9E

.

.

```
for day in range(len(self.raw_days)):
    mrp = self.major_rest_periods[day]
    # If MRP exists, run slice data and run scratch
    if mrp:
        raw_day = self.raw_days[day][mrp[0] : mrp[1]]
        if self.verbose:
            self.logger.info(
                "[SCRATCHPY] [INFO] Study: [{}] | Subject: [{}] | "
                "Location: [{}] | Visit: [{}] | Sliced data into total sleep opportunity for day: {}.".format(
                    self.study,
                    self.subject,
                    self.device_location,
                    self.visit,
                    day + 1,
                )
            )
    # No MRP, return np.nan for scratch endpoints
    else:
        if self.verbose:
            self.logger.info(
                "[SCRATCHPY] [ISSUE] Study: [{}] | Subject: [{}] | "
                "Location: [{}] | Visit: [{}] | No TSO. No scratch predictions. "
                "for day: {}. "
                "Not running ScratchPy module.".format(
                    self.study,
                    self.subject,
                    self.device_location,
                    self.visit,
                    day + 1,
                )
            )
        self.scratch_endpoints.append(
            [
                ("total_scratch_events", np.nan),
                ("total_scratch_duration_seconds", np.nan),
                ("total_no_scratch_events", np.nan),
                ("total_no_scratch_duration_seconds", np.nan),
            ]
        )
        continue

    raw_day_df = raw_day.copy()
    raw_day_df["ts"] = raw_day.index
    raw_day_df.reset_index(inplace=True, drop=True)
    raw_day_df = raw_day_df[["ts", "X", "Y", "Z"]]
    raw_day_df.columns = ["ts", "x", "y", "z"]
```

.

.

.

CONTINUES IN FIG. 9G

# *FIG. 9F*

CONTINUES FROM FIG. 9F　　.

　　　　　　　　　　　．

```
scratch = ScratchPy(
    raw_data_df=raw_day_df,
    results_directory=self.dst,
    sampling_frequency=20.0,
)
scratch.run()

if self.verbose:
    self.logger.info(
        "[SCRATCHPY] [INFO] Study: [{}] | Subject: [{}] | "
        "Location: [{}] | Visit: [{}] | Calculated scratch predictions for day {}.".format(
            self.study,
            self.subject,
            self.device_location,
            self.visit,
            day + 1,
        )
    )

if self.plot:
    scratch.visualize(prep_only=True)
    self.visualize_scratch(data_to_plot=scratch.data_to_plot, pdf=pdf)

    if self.verbose:
        self.logger.info(
            "[SCRATCHPY] [INFO] Study: [{}] | Subject: [{}] | "
            "Location: [{}] | Visit: [{}] | Appended scratch report for day {} to final report.".format(
                self.study,
                self.subject,
                self.device_location,
                self.visit,
                day + 1,
            )
        )

if self.export_scratch_data:
    scratch.export(
        output_file_prefix=self.src_name + "_day_{}".format(day + 1)
    )
    if self.verbose:
        self.logger.info(
            "[SCRATCHPY] [INFO] Study: [{}] | Subject: [{}] | "
            "Location: [{}] | Visit: [{}] | Exported scratch predictions and endpoints for day {}.".format(
                self.study,
                self.subject,
                self.device_location,
                self.visit,
                day + 1,
            )
        )
```

　　　　　　　　　　　．

　　　　　　　　　　　．

CONTINUES IN FIG. 9H

*FIG. 9G*

CONTINUES FROM FIG. 9G      .

.

```
        self.scratch_endpoints.append(
            sorted(scratch.scratch_endpoints.items(), reverse=True)
        )
    if self.plot:
        pdf.close()
        plt.close("all")

    return

def export_scratch_sleep(self):
    """
    Exports the combined scratch and sleep endpoints to .csv.
    """
    header = (
        ["date"] + self.endpoints[0] + [i[0] for i in self.scratch_endpoints[0]]
    )
    days = list(range(0, len(self.raw_days_to_plot)))
    export_df = []
    for day in days:
        idx_start = self.raw_days_to_plot[day].index[0]
        idx_start = (
            idx_start.replace(hour=12, minute=0, second=0, microsecond=0)
            - pd.to_timedelta("24h")
            if idx_start
            < idx_start.replace(hour=12, minute=0, second=0, microsecond=0)
            else idx_start.replace(hour=12, minute=0, second=0, microsecond=0)
        )
        if self.major_rest_periods[day]:
            export_df.append(
                [str(idx_start.date())]
                + self.endpoints[day + 1]
                + [i[1] for i in self.scratch_endpoints[day]]
            )
        else:
            export_df.append(
                [str(idx_start.date())]
                + self.endpoints[day + 1]
                + [np.nan for i in self.scratch_endpoints[day]]
            )
    export_df = pd.DataFrame(export_df)
    export_df.columns = header
    export_fp = self.dst + "/{}_sw_output_table.csv".format(self.src_name)
    export_df.to_csv(export_fp, index=False)

    if self.verbose:
        self.logger.info(
            "[ANALYTICS_PIPELINE] [INFO] Study: [{}] | Subject: [{}] | "
            "Location: [{}] | Visit: [{}] | Saved scratch and sleep endpoints: {}".format(
                self.study,
                self.subject,
                self.device_location,
                self.visit,
                export_fp,
            )
        )
    return
```

*FIG. 9H*

CONTINUES IN FIG. 9I

82

CONTINUES FROM FIG. 9H   .

.

```
@staticmethod
  def visualize_scratch(data_to_plot, pdf):
    """

    Generate a plot of a given instance of scratch algorithm results.

    """

    # Dates
    start_date = data_to_plot.index[0].strftime("%B %d, %Y, %r")
    stop_date = data_to_plot.index[-1].strftime("%B %d, %Y, %r")

    # PLOT!
    fig, axs = plt.subplots(nrows=3, ncols=1, figsize=(12, 8))
    plt.suptitle("Scratch Report (" + start_date + " - " + stop_date + ")")

    # Raw Data
    axs[0].plot(data_to_plot.index.values, data_to_plot.x, color="r", label="X")
    axs[0].plot(data_to_plot.index.values, data_to_plot.y, color="g", label="Y")
    axs[0].plot(data_to_plot.index.values, data_to_plot.z, color="b", label="Z")
    axs[0].xaxis.set_major_locator(mdates.HourLocator(interval=1))
    axs[0].xaxis.set_major_formatter(DateFormatter("%H:%M"))
    axs[0].legend(loc="center left", bbox_to_anchor=(1, 0.5))
    axs[0].set_ylabel("Raw Data (G)")

    # Hand Movement Predictions
    axs[1].plot(
        data_to_plot.index.values,
        data_to_plot.hand_movement_predictions,
        color="k",
        linewidth=0.1,
    )
    axs[1].xaxis.set_major_locator(mdates.HourLocator(interval=1))
    axs[1].xaxis.set_major_formatter(DateFormatter("%H:%M"))
    axs[1].set_ylabel("Hand Movement\nPredictions")
    axs[1].set_ylim([0.5, 1.5])
    axs[1].set_yticks([], [])

    # Scratch Predictions
    axs[2].plot(
        data_to_plot.index.values,
        data_to_plot.scratch_predictions,
        color="k",
        linewidth=0.1,
    )
    axs[2].xaxis.set_major_locator(mdates.HourLocator(interval=1))
    axs[2].xaxis.set_major_formatter(DateFormatter("%H:%M"))
    axs[2].set_ylabel("Scratch\nPredictions")
    axs[2].set_ylim([0.5, 1.5])
    axs[2].set_yticks([], [])
    axs[2].set_xlabel("Time (HH:MM)")
    pdf.savefig(fig.number)
```

*FIG. 9I*

```
###################################################################
#
#   Scratch Detection (pre-alpha)
#
###################################################################

import numpy as np
import pandas as pd
import datetime
import matplotlib.pyplot as plt
from matplotlib.dates import DateFormatter
import matplotlib.dates as mdates
import pkg_resources as pkr
from joblib import load
from motion_detection_algorithm import detect_motion_from_acceleration
from scratchpy import helper_functions


class ScratchPy:
    def __init__(
        self,
        raw_data_df,
        results_directory,
        sampling_frequency=20.0,
        verbose=False,
    ):
        """

        Scratch algorithm object. This object houses the procedure for computing scratch predictions from a wrist-worn
        accelerometer device.

        Parameters
        ----------
        raw_data_df : Pandas DataFrame
            DataFrame of raw accelerometer data. Measured in G's. Columns = [ts {datetime}, x {float}, y {float}, z {float}]
        results_directory : str
            File path to output directory
        verbose : bool
            Bool to allow for printing of results
        """
                                             .
                                             .
```

CONTINUES IN FIG. 10B

# FIG. 10A

CONTINUES FROM FIG. 10A  :
                         :

```
# Raw data to process
self.raw_data = raw_data_df
# Results directory to save final results
self.results_directory = results_directory
# Window size to process data (non-overlapping windows)
self.window_size = 3.0
# Properties of hand movement motion detection algorithm
self.motion_detection_filter_cutoff = 6
self.motion_detection_threshold = 0.022558169349678834
# Sampling frequency of raw data
self.fs = sampling_frequency
# Total number of accelerometer samples in a given window
self.no_samples_per_window = int(self.fs * self.window_size)
# Load serialized scratch model
model_file_path = pkr.resource_filename(
    "scratchpy.model", "model_trained_all_subjects.joblib"
)
self.scratch_model = load(model_file_path)
# Selected features for Scratch model
self.selected_features = [
    "PC2_iqr",
    "PC2_spectral_entropy",
    "PC1_iqr",
    "PC2_spectral_flatness",
    "PC1_dom_freq_magnitude",
    "PC2_sparc",
    "PC2_mean_cross_rate",
    "PC2_jerk_ratio",
    "PC2_signal_entropy",
    "PC2_dom_freq_magnitude",
    "mag_jerk_ratio",
    "PC1_spectral_flatness",
    "mag_sparc",
    "PC1_skewness",
    "PC1_permutation_entropy",
    "mag_rms",
    "PC1_signal_entropy",
    "PC2_permutation_entropy",
    "mag_signal_entropy",
    "PC1_ldl_jerk",
    "PC2_skewness",
    "mag_range",
    "PC1_sparc",
    "mag_permutation_entropy",
    "PC1_dom_freq_value",
    "mag_iqr",
]
```
                                 .

                                 .

# FIG. 10B

CONTINUES FROM FIG. 10B

```python
        # Hand movement predictions
        self.hand_movement_predictions = None
        # Include print statements outlining run of package
        self.verbose = verbose
        # Total recording duration
        self.recording_duration_minutes = None
        # Epoch Results
        self.epoch_timestamps = []
        self.epoch_scratch_predictions = []
        # Endpoint Results
        self.scratch_endpoints = None
        # Output filename prefix
        self.output_file_prefix = ""
        # Data for plotting
        self.data_to_plot = None

    def quality_check_input_data(self):
        """
        Run a series of quality checks on input data.
        """
        # -------------- #
        # [CHECK] Sampling rate is 20 Hz
        # -------------- #
        if self.fs != 20.0:
            print(
                "[QC ERROR] Sampling frequency not equal to 20 hz. Required 20hz input sampling rate."
            )
            return False


        # -------------- #
        # [CHECK] Raw data columns = ["ts", "x", "y", "z"]
        # -------------- #
        if self.raw_data.columns.tolist() != ["ts", "x", "y", "z"]:
            print(
                "[QC ERROR] Incorrect raw data column format. "
                "Columns of raw data must be formatted as: ['ts', 'x', 'y', 'z']"
            )
            return False


        # -------------- #
        # [CHECK] Raw data timestamp is datetime
        # -------------- #
        if not isinstance(self.raw_data.ts.values[0], np.datetime64):
            print("[QC ERROR] Timestamps of raw data are not np.datetime64.")
            return False
```

CONTINUES IN FIG. 10D

# FIG. 10C

CONTINUES FROM FIG. 10C

```
# -------------- #
    # [CHECK] duration of recording >= 3 seconds
    # -------------- #
    start_ts = self.raw_data.ts.values[0]
    stop_ts = self.raw_data.ts.values[-1]
    total_dur = (pd.to_datetime(stop_ts) - pd.to_datetime(start_ts)).total_seconds()
    if total_dur < 3.0:
        # Minimum of 3 seconds of data required to run algorithm
        print("[QC ERROR] Not enough data. Minimum recording duration is 3 seconds.")
        return False
    else:
        self.recording_duration_minutes = total_dur / 60.0

    return True

def run(self):
    """
    Run the scratch algorithm module.
    """
    # -------------- #
    # Run quality checks on input data
    # -------------- #
    if self.verbose:
        print("Running quality checks...")
    qc_result = self.quality_check_input_data()
    if not qc_result:
        return

    # -------------- #
    # 1. Run motion detection algorithm (hand movement)
    # -------------- #
    if self.verbose:
        print("Running hand movement detector...")
    hand_movement_predictions, _ = detect_motion_from_acceleration(
        sensor_data=self.raw_data,
        fs=self.fs,
        filter_cutoff=self.motion_detection_filter_cutoff,
        cov_threshold=self.motion_detection_threshold,
    )
    # Convert to DataFrame
    hand_movement_predictions_df = pd.DataFrame()
    hand_movement_predictions_df["hand_movement_predictions"] = pd.Series(
        hand_movement_predictions
    )
    self.hand_movement_predictions = hand_movement_predictions_df

    # Remove first 1 second of raw data (due to rolling CoV calculation)
    self.raw_data = self.raw_data.loc[self.fs :]
    self.raw_data.reset_index(drop=True, inplace=True)
```

CONTINUES IN FIG. 10E

## FIG. 10D

CONTINUES FROM FIG. 10D

```
# ------------- #
# 2. Window raw data and hand movement predictions into 3 second windows
# ------------- #
if self.verbose:
    print("Windowing data...")
windowed_data = helper_functions.non_overlapping_windows(
    df=self.raw_data, window_size=self.no_samples_per_window
)
windowed_hand_movement = helper_functions.non_overlapping_windows(
    df=hand_movement_predictions_df, window_size=self.no_samples_per_window
)
total_windows = windowed_hand_movement.shape[0]


# ------------- #
# 3. Iterate over each window and compute scratch prediction
# ------------- #
if self.verbose:
    print("Running model on each window...")
epoch_timestamps = []
epoch_scratch_predictions = []
for window in range(0, total_windows):
    # Get hand movement predictions for window
    hand_movement_window = windowed_hand_movement[window]
    hand_movement_window = hand_movement_window.reshape(
        self.no_samples_per_window,
    )

    raw_data_window = pd.DataFrame(
        windowed_data[window], columns=["ts", "x", "y", "z"]
    )
    # Assemble timestamps for window
    epoch_timestamps.extend(
        [
            raw_data_window.ts[0],
            raw_data_window.ts[0] + datetime.timedelta(seconds=1),
            raw_data_window.ts[0] + datetime.timedelta(seconds=2),
        ]
    )
```

# FIG. 10E

CONTINUES FROM FIG. 10E

```
# -------------- #
        # 4. Check if window is 100% hand movement
        # -------------- #
        if (np.unique(hand_movement_window) == [1])[0]:
            # Window is 100% hand movement
            # Signal preprocessing
            preprocessed_data = helper_functions.signal_preprocess_data(
                sliced_data=raw_data_window, fs=self.fs
            )

            # Signal Features
            ftc = helper_functions.compute_features(
                preprocessed_data=preprocessed_data, fs=self.fs
            )
            # Slice features for selected features
            ftc = ftc[self.selected_features]

            # Run through model
            prediction = list(self.scratch_model.predict(ftc.values))
            epoch_scratch_predictions.extend(prediction * int(self.window_size))

        else:
            # Window is not 100% hand movement => No Scratch
            epoch_scratch_predictions.extend([0] * int(self.window_size))

    # Set Epoch Results
    self.epoch_timestamps = epoch_timestamps
    self.epoch_scratch_predictions = epoch_scratch_predictions

    # Calculate and set endpoint results
    scratch_endpoints = helper_functions.calculate_scratch_endpoints(
        epoch_scratch_predictions,
    )

    self.scratch_endpoints = scratch_endpoints

def visualize(self, output_file_prefix="", prep_only=False):
    """"""
    Generate a plot of a given instance of scratch algorithm results.
    Plot will be extracted to user defined output directory.

    Parameters
    ----------
    output_file_prefix : str
        Identifier to add as prefix to save filename.
    prep_only : bool
        Flag for preparing plot data only, deactivates plotting or saving.

    """"""
```

# FIG. 10F

CONTINUES FROM FIG. 10F

```
# ------------- #
    # [CHECK] Necessary data is available
    # ------------- #

    if not len(self.epoch_scratch_predictions):
        print("[ERROR] Cannot export, no scratch predictions...")
        return

    if output_file_prefix:
        self.output_file_prefix = output_file_prefix

    plot_save_filepath = (
        self.results_directory
        + "/"
        + self.output_file_prefix
        + "_scratch_report.png"
    )

    # Plot Results
    if self.verbose and not prep_only:
        print("Generating plot...")
    elif self.verbose and prep_only:
        print("Generating plot data...")
    # Assemble predictions in 3 second windows instead of 1
    predictions_plot = self.epoch_scratch_predictions[::3]
    # Assemble predictions based on number of samples in window (1 pred per sample)
    preds_by_samples = []
    for i in predictions_plot:
        preds_by_samples.extend([i] * self.no_samples_per_window)

    # Slice raw data to number of samples that give full windows
    raw_data_to_plot = self.raw_data.iloc[: len(preds_by_samples)]

    # Slice hand movement predictions to number of samples that give full windows
    hm_preds = self.hand_movement_predictions.iloc[: len(preds_by_samples)]

    # Final DataFrame housing raw data, hand movement predictions, scratch predictions to plot
    data_to_plot = pd.concat([raw_data_to_plot, hm_preds], axis=1)
    data_to_plot["scratch_predictions"] = pd.Series(preds_by_samples)
    data_to_plot["ts"] = pd.to_datetime(data_to_plot["ts"])
    data_to_plot.set_index(keys="ts", drop=True, inplace=True)
    self.data_to_plot = data_to_plot

    # Prepare dataframe only
    if prep_only:
        if self.verbose:
            print("Plot data generated.")
        return

    # Dates
    start_date = data_to_plot.index[0].strftime("%Y-%m-%d")
    stop_date = data_to_plot.index[-1].strftime("%Y-%m-%d")

    # PLOT!
    fig, axs = plt.subplots(nrows=3, ncols=1, figsize=(12, 8))
    plt.suptitle("Scratch Report (" + start_date + " - " + stop_date + ")")
```

*FIG. 10G*

CONTINUES IN FIG. 10H

```
# Raw Data
axs[0].plot(data_to_plot.index.values, data_to_plot.x, color="r", label="X")
axs[0].plot(data_to_plot.index.values, data_to_plot.y, color="g", label="Y")
axs[0].plot(data_to_plot.index.values, data_to_plot.z, color="b", label="Z")
axs[0].xaxis.set_major_locator(mdates.HourLocator(interval=1))
axs[0].xaxis.set_major_formatter(DateFormatter("%H:%M"))
axs[0].legend(loc="center left", bbox_to_anchor=(1, 0.5))
axs[0].set_ylabel("Raw Data (G)")

# Hand Movement Predictions
axs[1].plot(
    data_to_plot.index.values,
    data_to_plot.hand_movement_predictions,
    color="k",
    linewidth=0.1,
)
axs[1].xaxis.set_major_locator(mdates.HourLocator(interval=1))
axs[1].xaxis.set_major_formatter(DateFormatter("%H:%M"))
axs[1].set_ylabel("Hand Movement\nPredictions")
axs[1].set_ylim([0.5, 1.5])
axs[1].set_yticks([], [])

# Scratch Predictions
axs[2].plot(
    data_to_plot.index.values,
    data_to_plot.scratch_predictions,
    color="k",
    linewidth=0.1,
)
axs[2].xaxis.set_major_locator(mdates.HourLocator(interval=1))
axs[2].xaxis.set_major_formatter(DateFormatter("%H:%M"))
axs[2].set_ylabel("Scratch\nPredictions")
axs[2].set_ylim([0.5, 1.5])
axs[2].set_yticks([], [])
axs[2].set_xlabel("Time (HH:MM)")

plt.savefig(plot_save_filepath)
if self.verbose:
    print("\tSaved: " + plot_save_filepath)

def export(self, output_file_prefix=""):
    """
    Export the results of the scratch algorithm module. Endpoints and epoch (1 second) predictions will
    be extracted to user defined output directory.

    Parameters
    ----------
    output_file_prefix : str
        Identifier to add as prefix to save filename.
    """
```

# FIG. 10H

CONTINUES FROM FIG. 10H    .
                           .

```
# -------------- #
# [CHECK] Necessary data is available
# -------------- #

if not len(self.epoch_scratch_predictions):
    print("[ERROR] Cannot export, no scratch predictions...")
    return

if output_file_prefix:
    self.output_file_prefix = output_file_prefix

if self.verbose:
    print("Exporting results...")

epoch_predictions_save_filepath = (
    self.results_directory
    + "/"
    + self.output_file_prefix
    + "_scratch_epoch_predictions.csv"
)
endpoint_predictions_save_filepath = (
    self.results_directory
    + "/"
    + self.output_file_prefix
    + "_scratch_endpoint_predictions.csv"
)

# Epoch Predictions
epoch_predictions = pd.DataFrame()
epoch_predictions["ts"] = pd.Series(self.epoch_timestamps)
epoch_predictions["predictions"] = pd.Series(self.epoch_scratch_predictions)
epoch_predictions.to_csv(epoch_predictions_save_filepath, index=False)
if self.verbose:
    print("\tSaved: ", epoch_predictions_save_filepath)

# Endpoint Predictions
save_endpoints = {}
for i in self.scratch_endpoints:
    save_endpoints[i] = [self.scratch_endpoints[i]]
endpoint_predictions = pd.DataFrame.from_dict(save_endpoints)
endpoint_predictions.to_csv(endpoint_predictions_save_filepath, index=False)
if self.verbose:
    print(
        "\tSaved: ", endpoint_predictions_save_filepath,
    )
```

*FIG. 10I*

```
#####################################################################
#
#   Sleep Detection & Determine TSO  (pre-alpha)
#
#####################################################################

import pandas as pd
import numpy as np
import seaborn as sns
import matplotlib.pyplot as plt
import matplotlib.dates as mdates
import matplotlib.backends.backend_pdf
from sleeppy.majorrest import MajorRestPeriod
from sleeppy.colekripke import ColeKripke
from inertial_sensor_routines.gnactv import tabular_conversion
from sleeppy.utils import (
    downsample,
    resample_by_interpolation,
    bin2df,
    high_pass_filter,
    activity_index,
    load_geneactiv_csv,
    non_overlapping_windows,
    total_sleep_time,
    percent_time_asleep,
    number_of_wake_bouts,
    wake_after_sleep_onset,
    sleep_onset_latency,
    build_logger,
)

sns.set()

class SleepPy(object):

    def __init__(
        self,
        input_file,
        results_directory,
        sampling_frequency=None,
        aws_object=None,
        start_buffer="0s",
        stop_buffer="0s",
        start_time="",
        stop_time="",
        temperature_threshold=25.0,
        minimum_rest_block=30,
        allowed_rest_break=60,
        minimum_rest_threshold=0.1,
        maximum_rest_threshold=1.0,
        minimum_rest_period=None,
        movement_based_nonwear=None,
        minimum_hours=6,
        downsample=True,
        plot=True,
        fast_load=True,
        logger=None,
    ):
```

*FIG. 11A*

CONTINUES FROM FIG. 11A :

"""
Class initialization.

Parameters
----------
input_file : string
    Full path to the file to be processed.
results_directory : string
    Full path to the directory where the results should be saved.
sampling_frequency : float
    Sampling frequency of the input data, required if not using fast load.
aws_object : data object
    Data object to be processed from aws (in place of source file path).
start_buffer : string
    Number of seconds to ignore from the beginning of the recording. format: "0s"
stop_buffer : string
    Number of seconds to ignore at the end of the recording. format: "0s"
start_time : string
    Time stamp from which to start looking at data. format: "%Y-%m-%d %H:%M:%S:%f"
stop_time : string
    Time stamp at which to stop looking at data. format: "%Y-%m-%d %H:%M:%S:%f"
temperature_threshold : float
    Lowest temperature for which a data point is considered valid.
minimum_rest_block : int
    Number of minutes required to consider a rest period valid.
allowed_rest_break : int
    Number of minutes allowed to interrupt major rest period.
minimum_rest_threshold : float
    Minimum allowed threshold for determining major rest period.
maximum_rest_threshold : float
    Maximum allowed threshold for determining major rest period.
minimum_rest_period : float
    Minimum length allowed for major rest period.
movement_based_nonwear : float
    Threshold for movement based nonwear.
minimum_hours : float
    Minimum number of hours required to consider a day useable.
downsample : boolean
    True triggers downsampling to 20hz.
plot : boolean
    True triggers plotting of data.
fast_load : boolean
    True triggers inertial sensor routines fortran loading of bin data. Locates sampling frequency in data.
logger : logger object
    Replace default logger with logger object if desired.
"""

CONTINUES IN FIG. 11C

FIG. 11B

CONTINUES FROM FIG. 11B    :
                           :
                           .

```
if aws_object is not None:
    self.src = aws_object
else:
    self.src = input_file
self.extension = input_file.split(".")[-1]
self.dst = results_directory
self.src_name = input_file.split("/")[-1][0:-4]
self.sub_dst = results_directory
self.fs = sampling_frequency
self.window_size = 60
self.high_pass_cutoff = 0.25
self.start_buffer = start_buffer
self.stop_buffer = stop_buffer
self.start_time = start_time
self.stop_time = stop_time
self.min_t = temperature_threshold
self.minimum_rest_block = minimum_rest_block
self.allowed_rest_break = allowed_rest_break
self.minimum_rest_threshold = minimum_rest_threshold
self.maximum_rest_threshold = maximum_rest_threshold
self.minimum_rest_period = minimum_rest_period
self.movement_based_nonwear = movement_based_nonwear
self.minimum_hours = minimum_hours
self.downsample = downsample
self.plot = plot
self.fast_load = fast_load
if logger is None:
    self.logger = build_logger(log_name=__name__)
else:
    self.logger = logger

# DATA
self.raw_days = []
self.raw_days_to_plot = []
self.arm_angle_days = []
self.arm_angle_thresholds = []
self.rest_period_days = []
self.activity_index_days = []
self.sleep_wake_prediction_days = []
self.major_rest_periods = []
self.endpoints = [
    [
        "total_sleep_time",
        "percent_time_asleep",
        "waso",
        "sleep_onset_latency",
        "number_wake_bouts",
        "total_sleep_opportunity",
    ]
]
```

:    CONTINUES IN FIG. 11D
.

# FIG. 11C

CONTINUES FROM FIG. 11C

```
def run(self):
    """
    Runs the full package end to end on the input data.

    """
    if self.fast_load:
        self.logger.info("Running fast loader. Inferring sampling frequency...")
    else:
        self.logger.info("Loading data...")
    self.load()
    self.logger.info("Detecting major rest periods...")
    self.calculate_major_rest_periods()
    self.logger.info("Making sleep predictions...")
    self.calculate_sleep_predictions()
    self.logger.info("Calculating sleep endpoints...")
    self.calculate_endpoints()
    if self.plot:
        self.logger.info("Building plots...")
        self.visualize()
    self.logger.info("Exporting results...")
    self.export()

    return

def load(self):
    """
    Loads full data into 24 hour days.

    """
    # FULL DATA
    data = (
        load_geneactiv_csv(self.src)
        if self.extension == "csv"
        else (
            bin2df(self.src)
            if not self.fast_load
            else tabular_conversion(
                self.src, acc_units="g", save_file=None, return_sampling_rate=True,
            )
        )
    )

    if self.fast_load:
        df = data[0]
        self.fs = data[1]
        self.logger.info(f"Detected frequency: {data[1]}")
    else:
        df = data

    # CHECK DATA LENGTH
    if ((len(df) / float(self.fs)) / 3600.0) <= self.minimum_hours:
        exception_string = f"Total length of data did not meet your minimum time requirement of {self.minimum_hours}
hours"
        raise Exception(exception_string)
```

*FIG. 11D*

CONTINUES IN FIG. 11E

CONTINUES FROM FIG. 11D

```
# DOWNSAMPLE
if self.downsample:
    self.logger.info(f"Downsampling data from {self.fs} to 20hz.")
    if self.fs > 20.0:
        if (self.fs / 20.0).is_integer():
            df = downsample(df, int(self.fs) // 20)
        else:
            df = resample_by_interpolation(df, int(self.fs), 20)
        self.fs = 20.0

# APPLY BUFFERS
df = df.loc[
    df.index[0]
    + pd.Timedelta(self.start_buffer) : df.index[-1]
    - pd.Timedelta(self.stop_buffer)
]

# APPLY START AND STOP TIMES
if self.start_time and self.stop_time:
    self.start_time = pd.to_datetime(
        self.start_time, format="%Y-%m-%d %H:%M:%S:%f"
    )
    self.stop_time = pd.to_datetime(
        self.stop_time, format="%Y-%m-%d %H:%M:%S:%f"
    )
    df = df.loc[self.start_time : self.stop_time]
elif self.start_time:
    self.start_time = pd.to_datetime(
        self.start_time, format="%Y-%m-%d %H:%M:%S:%f"
    )
    df = df.loc[self.start_time :]
elif self.stop_time:
    self.stop_time = pd.to_datetime(
        self.stop_time, format="%Y-%m-%d %H:%M:%S:%f"
    )
    df = df.loc[: self.stop_time]

# SPLIT BY DAY
self.logger.info("Splitting raw data into 24 hour periods...")
self.raw_days = [
    day[1]
    for day in df.groupby(pd.Grouper(level=0, freq="24h", base=12))
    if ((len(day[1]) / float(self.fs)) / 3600.0) >= self.minimum_hours
]

# MAKE SURE WE ARE LEFT WITH USEFUL DAYS
if not self.raw_days:
    exception_string = f"No processable days found. Please examine your input data. Ensure there are enough hours
based on your minimum criteria of {self.minimum_hours} hours."
    raise Exception(exception_string)

# DAYS FOR PLOTTING
self.raw_days_to_plot = [day.resample("60s").median() for day in self.raw_days]
return
```

*FIG. 11E*

CONTINUES IN FIG. 11F

CONTINUES FROM FIG. 11E ⋮

```python
def calculate_major_rest_periods(self):
    """
    Gets the major rest periods for each 24 hour day.
    """
    for day in self.raw_days:
        mrp = MajorRestPeriod(
            day.copy(),
            self.fs,
            minimum_rest_block=self.minimum_rest_block,
            minimum_rest_threshold=self.minimum_rest_threshold,
            allowed_rest_break=self.allowed_rest_break,
            temperature_threshold=self.min_t,
            maximum_rest_threshold=self.maximum_rest_threshold,
            minimum_rest_period=self.minimum_rest_period,
            movement_based_nonwear=self.movement_based_nonwear,
        )
        mrp.run()
        self.arm_angle_days.append(mrp.df_angle)
        self.arm_angle_thresholds.append(mrp.arm_angle_thresh)
        self.rest_period_days.append(mrp.rest_periods)
        self.major_rest_periods.append(mrp.mrp)
    return


def calculate_sleep_predictions(self):
    """
    Gets the sleep/wake predictions for each 24 hour day.
    """
    for day in self.raw_days:
        # PREPROCESS, ACTIVITY INDEX
        day = day.copy()[["X", "Y", "Z"]]
        windowed = non_overlapping_windows(day.copy(), int(60 * self.fs))
        filtered = high_pass_filter(
            windowed,
            sampling_rate=self.fs,
            hp_cutoff=self.high_pass_cutoff,
            order=3,
        )
        ai = activity_index(filtered)

        # SLEEP WAKE PREDICTIONS
        ck = ColeKripke(ai.flatten())
        predictions = ck.predict()

        # ADD TIME BACK
        idx = pd.date_range(day.index[0], day.index[-1], freq="60s")[
            0 : len(predictions)
        ]
        predictions = pd.DataFrame(predictions)
        predictions.index = idx
        ai = pd.DataFrame(ai)
        ai.index = idx

        # STORE DATA
        self.activity_index_days.append(ai)
        self.sleep_wake_prediction_days.append(predictions)
    return
```

*FIG. 11F*

⋮ CONTINUES IN FIG. 11G

CONTINUES FROM FIG. 11F

```
def calculate_endpoints(self):
    """
    Calculates the clinical sleep endpoints for each 24 hour day.

    """
    for day in range(len(self.sleep_wake_prediction_days)):
        mrp = self.major_rest_periods[day]
        if mrp:
            predictions = self.sleep_wake_prediction_days[day][
                mrp[0] : mrp[1]
            ].values
            self.endpoints.append(
                [
                    total_sleep_time(predictions),
                    percent_time_asleep(predictions),
                    wake_after_sleep_onset(predictions),
                    sleep_onset_latency(predictions),
                    number_of_wake_bouts(predictions),
                    int((mrp[1] - mrp[0]).total_seconds() / 60.0),
                ]
            )
        else:
            self.endpoints.append([np.nan] * 6)
    return

def visualize(self):
    """
    Builds all visual reports and exports them to pdf.

    """
    # OUTPUT PDF FOR ALL DAYS
    pdf = matplotlib.backends.backend_pdf.PdfPages(
        self.dst + "/{}_visual_report.pdf".format(self.src_name)
    )

    # PROCESS DAYS
    days = list(range(0, len(self.raw_days_to_plot)))
    for day in days:
        # LOAD DATA, GET SHARED AXIS
        raw = self.raw_days_to_plot[day]
        idx_start = raw.index[0]
        idx_start = (
            idx_start.replace(hour=12, minute=0, second=0, microsecond=0)
            - pd.to_timedelta("24h")
            if idx_start
            < idx_start.replace(hour=12, minute=0, second=0, microsecond=0)
            else idx_start.replace(hour=12, minute=0, second=0, microsecond=0)
        )
        idx = pd.date_range(start=idx_start, periods=1440, freq="60s")
        raw = raw.reindex(idx, fill_value=np.nan)
```

CONTINUES IN FIG. 11H

# *FIG. 11G*

```
# ACTIVITY INDEX
act = self.activity_index_days[day]
act = act.resample("60s").max()
act = act.reindex(idx, fill_value=np.nan)

# ARM ANGLE
ang = self.arm_angle_days[day]
ang = ang.resample("60s").max()
ang = ang.reindex(idx, fill_value=np.nan)

# SLEEP WAKE PREDICTIONS
swp = self.sleep_wake_prediction_days[day]
swp[swp == 0] = np.nan
swp = swp.resample("60s").max()
swp = swp.reindex(idx, fill_value=np.nan)

# REST PERIODS
rps = self.rest_period_days[day]
rps[rps == 1] = np.nan
rps[rps == 0] = 1
rps = rps.resample("60s").max()
rps = rps.reindex(idx, fill_value=np.nan)

# DATAFRAME FOR STRAIGHT LINES
df = swp.copy()
df.columns = ["wake predictions"]
df[["wake predictions"]] -= 0.25
df["rest periods"] = rps.values - 0.75

# SET FIRST AND LAST VALUES TO PREVENT NAN PLOT ISSUES
df.loc[:1, "wake predictions"] = 0.75
df.loc[-1:, "wake predictions"] = 0.75
df.loc[:1, "rest periods"] = 0.25
df.loc[-1:, "rest periods"] = 0.25

# ENDPOINTS FOR TABLE
    t_labels = (
        "Total Sleep Time(min)",
        "Percent Time Asleep",
        "Wake After Sleep Onset(min)",
        "Sleep Onset Latency(min)",
        "Number of Wake Bouts",
        "Total Sleep Opportunity(min)",
    )
    t_vals = [self.endpoints[day + 1]]

    # PLOTTING
    fig, (axt, ax0, ax1, ax2, ax3, ax4, ax5) = plt.subplots(
        7, 1, figsize=(30, 15), sharex=True
    )
    plt.suptitle(
        "Visual Report for Source: {}\nDay: {}\nDate: {}".format(
            self.src_name, day + 1, idx[0].date()
        ),
        fontsize=25,
    )
    hours = mdates.HourLocator(interval=1)
    h_fmt = mdates.DateFormatter("%H:%M")
    all_axes = (ax0, ax1, ax2, ax3, ax4, ax5)
```

*FIG. 11H*

CONTINUES FROM FIG. 11H

```
# PLOT TABLE
tbl = axt.table(
  cellText=t_vals,
  colLabels=t_labels,
  cellLoc="center",
  rowLoc="center",
  loc="center",
  fontsize=20,
)
tbl.auto_set_font_size(False)
tbl.set_fontsize(24)
tbl.scale(1.15, 2.4)
axt.axis("off")

# RAW DATA
raw.rename(columns={"T": "Temperature", "LUX": "Light"}, inplace=True)
raw[["X", "Y", "Z"]].plot(ax=ax0, lw=1, x_compat=True).legend(
  bbox_to_anchor=(0, 1), fontsize=20
)

# TEMPERATURE
raw[["Temperature"]].plot(
  ax=ax1, lw=1, x_compat=True, color=sns.xkcd_rgb["pale red"]
).legend(bbox_to_anchor=(0, 1), fontsize=20)
idx_first = raw.Temperature.first_valid_index()
idx_last = raw.Temperature.last_valid_index()
ax1.hlines(
  y=self.min_t,
  xmin=idx_first,
  xmax=idx_last,
  color="r",
  linestyle="--",
  lw=2,
)
props = dict(boxstyle="round", facecolor="lavender", alpha=0.35)
textstr = "max: {}\xb0C\nmin: {}\xb0C\nthresh: {}\xb0C".format(
  np.round(raw[["Temperature"]].max().values[0], decimals=2),
  np.round(raw[["Temperature"]].min().values[0], decimals=2),
  self.min_t,
)
ax1.text(
  1.01,
  0.95,
  textstr,
  transform=ax1.transAxes,
  fontsize=14,
  verticalalignment="top",
  bbox=props,
)

# LIGHT
raw[["Light"]].plot(
  ax=ax2, lw=1, x_compat=True, color=sns.xkcd_rgb["pale orange"]
).legend(bbox_to_anchor=(0, 1), fontsize=20)
```

CONTINUES IN FIG. 11J

*FIG. 11I*

CONTINUES FROM FIG. 11I ⋮

```python
# ACTIVITY INDEX
act.plot(ax=ax3, lw=1, x_compat=True, color="#6fc276").legend(
    labels=["activity"], bbox_to_anchor=(0, 0.75), fontsize=20
)

# ARM ANGLE
ang.plot(ax=ax4, lw=1, x_compat=True, color="#b36ff6").legend(
    labels=["arm angle"], bbox_to_anchor=(0, 0.75), fontsize=20
)
textstr = "thresh: {}".format(
    np.round(self.arm_angle_thresholds[day], decimals=2)
)
ax4.text(
    1.01,
    0.95,
    textstr,
    transform=ax4.transAxes,
    fontsize=14,
    verticalalignment="top",
    bbox=props,
)

# SLEEP WAKE/REST PERIODS
df.plot(ax=ax5, lw=10, x_compat=True, ylim=(0, 1)).legend(
    bbox_to_anchor=(0, 0.85), fontsize=20
)

# FORMAT
plt.draw()
count = 0
for ax in all_axes:
    count += 1
    ax.set_xlabel("")
    ax.set_ylabel("")
    ax.spines["top"].set_visible(False)
    ax.spines["right"].set_visible(False)
    ax.spines["bottom"].set_visible(False)
    ax.spines["left"].set_visible(False)
    ax.grid(False)
    if count < 6:
        ax.get_xaxis().set_ticks([])
    ax.get_yaxis().set_ticks([])
    ax5.xaxis.set_major_locator(hours)
    ax5.xaxis.set_major_formatter(h_fmt)
    plt.subplots_adjust(wspace=0, hspace=0)
    fig.autofmt_xdate()
    for tick in ax5.xaxis.get_major_ticks():
        tick.label1.set_fontsize(16)
    pdf.savefig(fig.number)
pdf.close()
plt.close("all")

# SUMMARY REPORT
fig, (ax0, ax1, ax2, ax3, ax4, ax5) = plt.subplots(6, 1, figsize=(12, 12))
plt.suptitle("Summary Report for Source: {}".format(self.src_name), fontsize=16)
all_axes = (ax0, ax1, ax2, ax3, ax4, ax5)
```

*FIG. 11J*

⋮ CONTINUES IN FIG. 11K

CONTINUES FROM FIG. 11J

```python
# ENDPOINTS DATAFRAME
endpoints = pd.DataFrame(self.endpoints[1:])
endpoints.columns = self.endpoints[0]
if endpoints.isna().values.all():
    self.logger.error(
        "No valid endpoints detected across all days, aborting summary report generation..."
    )
    return
ylabels = [
    "Total Sleep\nTime(min)\nMean: {}".format(
        int(np.round(endpoints.total_sleep_time.mean()))
    ),
    "Percent Time\nAsleep\nMean: {}".format(
        int(np.round(endpoints.percent_time_asleep.mean()))
    ),
    "Wake After\nSleep Onset(min)\nMean: {}".format(
        int(np.round(endpoints.waso.mean()))
    ),
    "Sleep Onset\nLatency(min)\nMean: {}".format(
        int(np.round(endpoints.sleep_onset_latency.mean()))
    ),
    "Number of\nWake Bouts\nMean: {}".format(
        int(np.round(endpoints.number_wake_bouts.mean()))
    ),
    "Total Sleep\nOpportunity(min)\nMean: {}".format(
        int(np.round(endpoints.total_sleep_opportunity.mean()))
    ),
]


# TOTAL SLEEP TIME
endpoints.total_sleep_time.plot.bar(
    ax=ax0, title="", color=["C" + str(i) for i in range(len(endpoints))]
)
# PERCENT TIME ASLEEP
endpoints.percent_time_asleep.plot.bar(
    ax=ax1, title="", color=["C" + str(i) for i in range(len(endpoints))]
)
# WAKE AFTER SLEEP ONSET
endpoints.waso.plot.bar(
    ax=ax2, title="", color=["C" + str(i) for i in range(len(endpoints))]
)
# SLEEP ONSET LATENCY
endpoints.sleep_onset_latency.plot.bar(
    ax=ax3, title="", color=["C" + str(i) for i in range(len(endpoints))]
)
# NUMBER OF WAKE BOUTS
endpoints.number_wake_bouts.plot.bar(
    ax=ax4, title="", color=["C" + str(i) for i in range(len(endpoints))]
)
# TOTAL SLEEP OPPORTUNITY
endpoints.total_sleep_opportunity.plot.bar(
    ax=ax5, title="", color=["C" + str(i) for i in range(len(endpoints))]
)
```

*FIG. 11K*

CONTINUES FROM FIG. 11K :

```
# FORMAT
count = 0
for ax in all_axes:
    count += 1
    ax.spines["top"].set_visible(False)
    ax.spines["right"].set_visible(False)
    ax.spines["bottom"].set_visible(False)
    ax.spines["left"].set_visible(False)
    ax.grid(False)
    ax.set_ylabel(ylabels[count - 1], rotation=0, fontsize=12, labelpad=50)
    if count < 5:
        ax.set_xlabel("")
        ax.get_xaxis().set_ticks([])
        ax.get_yaxis().set_ticks([])
    else:
        ax.set_xlabel("Day", fontsize=20)
        ax.get_yaxis().set_ticks([])
    plt.setp(ax.xaxis.get_majorticklabels(), rotation=0)
    for p in ax.patches:
        ax.annotate(
            np.round(p.get_height(), decimals=2),
            (p.get_x() + p.get_width() / 2.0, 0),
            ha="center",
            va="center",
            xytext=(0, 10),
            textcoords="offset points",
            fontweight="bold",
        )
plt.subplots_adjust(wspace=0, hspace=0.01)
plt.xticks(fontsize=20)
plt.draw()
plt.savefig(self.dst + "/{}_summary_report.pdf".format(self.src_name))
plt.close("all")
return

def export(self):
    """
    Exports a results table to csv.
    """
    header = ["date"] + self.endpoints[0] + ["sleep_start", "sleep_end"]
    days = list(range(0, len(self.raw_days_to_plot)))
    export_df = []
    for day in days:
        idx_start = self.raw_days_to_plot[day].index[0]
        idx_start = (
            idx_start.replace(hour=12, minute=0, second=0, microsecond=0)
            - pd.to_timedelta("24h")
            if idx_start
            < idx_start.replace(hour=12, minute=0, second=0, microsecond=0)
            else idx_start.replace(hour=12, minute=0, second=0, microsecond=0)
        )
        if self.major_rest_periods[day]:
            export_df.append(
                [str(idx_start.date())]
                + self.endpoints[day + 1]
                + [str(i) for i in self.major_rest_periods[day]]
            )
```

*FIG. 11L*

: CONTINUES IN FIG. 11M

CONTINUES FROM FIG. 11L  .
.

```
        else:
            export_df.append(
                [str(idx_start.date())]
                + [np.nan for i in self.endpoints[day + 1]]
                + [np.nan, np.nan]
            )

    export_df = pd.DataFrame(export_df)
    export_df.columns = header
    export_df.to_csv(
        self.dst + "/{}_output_table.csv".format(self.src_name), index=False
    )
    return


if __name__ == "__main__":
    infile = ""
    outdir = ""
    SleepPy(
        input_file=infile,
        results_directory=outdir,
        sampling_frequency=None,
        aws_object=None,
        start_buffer="0s",
        stop_buffer="0s",
        start_time="",
        stop_time="",
        temperature_threshold=25.0,
        minimum_rest_block=30,
        allowed_rest_break=150,
        minimum_rest_threshold=0.1,
        maximum_rest_threshold=1.0,
        minimum_rest_period=None,
        movement_based_nonwear=0.001,
        minimum_hours=12,
        downsample=True,
        plot=True,
        fast_load=True,
        logger=None,
    ).run()
```

# FIG. 11M

1200

MEMORY

1212

PROCESSOR(S)

1214

PRESENTATION
COMPONENT(S)

1216

RADIO(S)

1224

1210

I/O PORT(S)

1218

I/O COMPONENTS

1220

POWER SUPPLY

1222

*FIG. 12*

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **ARNAUD MOREAU et al.** Detection of Nocturnal Scratching Movements in Patients with Atopic Dermatitis Using Accelerometers and Recurrent Neural Networks. *IEEE JOURNAL OF BIOMEDICAL AND HEALTH INFORMATICS*, 01 July 2018, vol. 22 (4)

- **ROGER J. COLE ; DANIEL F. KRIPKE ; WILLIAM GRUEN ; DANIEL J. MULLANEY ; J. CHRISTIAN GILLIN**. Automatic SleeplWake Identification From Wrist Activity. *Sleep*, September 1992, vol. 15 (5), 461-469, https://doi.org/10.1093/sleep/15.5.461